(19) Europäisches Patentamt
European Patent Office
Office européen des brevets

(11) Veröffentlichungsnummer : **0 278 907 B1**

(12) **EUROPÄISCHE PATENTSCHRIFT**

(45) Veröffentlichungstag der Patentschrift : **29.05.91 Patentblatt 91/22**

(21) Anmeldenummer : **88810062.5**

(22) Anmeldetag : **03.02.88**

(51) Int. Cl.$^5$ : **C07C 275/26,** C07C 233/32, C07C 271/24, C07C 271/36, C07C 271/56, C07C 251/18, A01N 37/18, A01N 47/10, A01N 47/32

# (54) Neue Cyclohexandione.

(30) Priorität : **09.02.87 CH 469/87**

(43) Veröffentlichungstag der Anmeldung : **17.08.88 Patentblatt 88/33**

(45) Bekanntmachung des Hinweises auf die Patenterteilung : **29.05.91 Patentblatt 91/22**

(84) Benannte Vertragsstaaten : **AT BE CH DE ES FR GB GR IT LI LU NL SE**

(56) Entgegenhaltungen :
**EP-A- 0 072 528**
**EP-A- 0 126 713**
**DE-A- 2 516 556**

(73) Patentinhaber : **CIBA-GEIGY AG**
**Klybeckstrasse 141**
**CH-4002 Basel (CH)**

(72) Erfinder : **Brunner, Hans-Georg, Dr.**
**Wannenstrasse 14**
**CH-4415 Lausen (CH)**

EP 0 278 907 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Cyclohexandione mit herbizider und pflanzenwuchsregulatorischer Wirkung, agrochemische Mittel, enthaltend diese Cyclohexandione, deren Verwendung zur Bekämpfung unerwünschten Pflanzenwachstums und zur Regulierung des Pflanzenwuchses sowie Verfahren zur Herstellung der erfindungsgemässen Verbindungen. Ferner betrifft die Erfindung auch neue Zwischenprodukte und Verfahren zu deren Herstellung.

Es sind bereits zahlreiche 2,5-disubstituierte Cyclohexan-1,3-dione mit herbizider Wirkung bekannt geworden. Diese Verbindungen befriedigen nicht immer im Hinblick auf Wirkungsstärke, Wirkdauer, Selektivität und Anwendbarkeit. Demgegenüber wurde überraschenderweise gefunden, dass die neuen 2,5-disubstituierten Cyclohexan-1,3-dione der allgemeinen Formel I gut herbizid und pflanzenwuchsregulatorisch wirksam sind.

Die Erfindung betrifft die neuen Cyclohexan-1,3-dione der Formel I

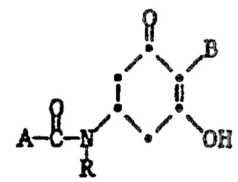

(I)

worin

R für Wasserstoff oder $C_1$-$C_6$-Alkyl,

A für $R_2$, $OR_3$ oder $NR_3R_4$,

$R_2$ für $C_3$-$C_6$-Cycloalkyl oder gegebenenfalls einfach durch $C_1$-$C_4$-Alkoxy oder ein- oder mehrfach durch Halogen substituiertes $C_1$-$C_6$-Alkyl,

$R_3$ für $C_1$-$C_6$-Alkyl, $C_3$-$C_6$-Cycloalkyl oder gegebenenfalls bis zu dreifach durch $R_5$ substituiertes Phenyl oder Benzyl,

$R_4$ für Wasserstoff, $C_1$-$C_6$-Alkyl, $C_1$-$C_4$-Alkoxy oder $C_3$-$C_6$-Cycloalkyl,

$R_3$ und $R_4$ ausserdem gemeinsam mit dem Stickstoffatom, an welches sie gebunden sind für einen Pyrrolidin-, Morpholin- oder Piperidinrest,

B für einen der Reste

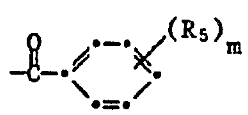 , 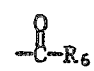 oder 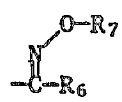 ,

m für 0, 1, 2 oder 3

$R_5$ für Halogen, Nitro, Cyano, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Alkylsulfinyl oder $C_1$-$C_4$-Alkylsulfonyl,

$R_6$ für $C_1$-$C_6$-Alkyl oder $C_3$-$C_6$-Cycloalkyl,

$R_7$ für Wasserstoff, $C_1$-$C_6$-Alkyl, $C_1$-$C_6$-Halogenalkyl, $C_3$-$C_6$-Alkenyl, $C_3$-$C_6$-Halogenalkenyl oder $C_3$-$C_6$-Alkinyl steht

sowie Salze des Verbindungen der Formel I mit Säuren, Basen oder Komplexbildnern.

Im Rahmen der hier offenbarten Erfindung umfassen die generischen Begriffe beispielsweise die nachstehend genannten Einzelsubstituenten, wobei diese Aufzählung keine Beschränkung der Erfindung darstellt.

Alkyl umfasst die geradkettigen oder verzweigten $C_1$-$C_6$-Alkyle, wie Methyl, Ethyl, n-Propyl, iso-Propyl, n-Butyl, iso-Butyl, tert-Butyl, sowie die isomeren Pentyl- und Hexylreste.

Halogen ist Fluor, Chlor, Brom und Iod ; in erster Linie Fluor und Chlor.

$C_1$-$C_4$-Alkoxy ist Methoxy, Ethoxy, n-Propyloxy, iso-Propyloxy, sowie die isomeren Butyloxyreste. Bevorzugt ist Methoxy und Ethoxy.

$C_1$-$C_4$-Alkoxy-$C_1$-$C_4$-alkyl umfasst die isomeren Alkoxyalkyle, die im Rahmen der vorstehenden Definition aus den Alkoxy- und Alkylresten herleitbar sind. Bevorzugt ist $C_1$-$C_2$-Alkoxy-$C_1$-$C_2$-alkyl, wie Methoxymethyl, Ethoxymethyl, 2-Methoxyethyl, 1-Methoxyethyl oder 2-Ethoxyethyl.

Mit Halogenalkyl sind die ganz oder teilweise gleich oder unterschiedlich halogensubstituierten Alkylradikale gemäss der jeweiligen Definitionsbreite gemeint ; wie etwa Trifluormethyl, Trichlormethyl, Chlormethyl,

Dichlormethyl, 2,2,2-Triflorethyl etc.

Die $C_3$-$C_6$-Alkenyl-, $C_3$-$C_6$-Halogenalkenyl- bzw. $C_3$-$C_6$-Alkinylreste sind durch ein gesättigtes Kohlenstoffatom an das Sauerstoffatom der Oximgruppe gebunden. Bevorzugt sind der Allyl- und Propargylrest sowie der 3-Chlorprop-2-en-1-yl-Rest.

$C_3$-$C_6$-Cycloalkyl sind Cyclopropyl, Cyclobutyl, Cyclopentyl und Cyclohexyl. Hevorzuheben sind Cyclopropyl und Cyclohexyl.

In Fällen, in welchen ein Substituent, wie etwa $R_5$, mehrfach vorkommen kann, sind die jeweiligen Bedeutungen aus der diesem Substituenten zugeordneten Gruppe von Radikalen frei wählbar.

Unter Beibehaltung der Definitionsbreite der jeweiligen anderen Substituenten können die Verbindungen der Formel I hinsichtlich ihrer Struktur als

a) Cyclohexyl-Phenyl-Ketone

(mit B = 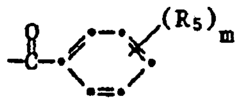 ) ,

b) Cyclohexyl-Alkyl-Ketone

(mit B = 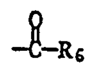

und $R_6$ = $C_1$-$C_6$-Alkyl),

c) Cyclohexyl-Cycloalkyl-Ketone

(mit B = 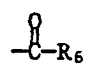

und $R_6$ = $C_3$-$C_6$-Cycloalkyl),

d) Oxime

(mit B = 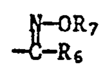 ),

e) Harnstoffe (mit A = $NR_3R_4$),

f) Urethane (mit A = $OR_3$) oder

g) Carbamide (mit A = $R_2$) bezeichnet werden.

Hervorzuheben sind die Verbindungen der Formel I,

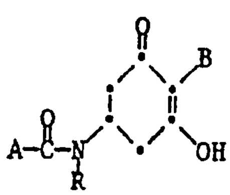

(I) ,

worin

R für Wasserstoff oder $C_1$-$C_4$-Alkyl,

A für $R_2$, $OR_3$ oder $NR_3R_4$,

$R_2$ für Cyclopropyl, Cyclopentyl, Cyclohexyl oder gegebenenfalls einfach durch $C_1$-$C_4$-Alkoxy oder ein- oder mehrfach durch Halogen substituiertes $C_1$-$C_4$-Alkyl,

$R_3$ für $C_1$-$C_4$-Alkyl, Cyclopropyl, Cyclopentyl, Cyclohexyl oder für gegebenenfalls bis zu zweifach durch $R_5$ substituiertes Phenyl oder Benzyl,
$R_4$ für Wasserstoff, $C_1$-$C_4$-Alkyl, Methoxy, Cyclopropyl, Cyclopentyl oder Cyclohexyl,
$R_3$ und $R_4$ ausserdem gemeinsam mit dem Stickstoffatom, an welches sie gebunden sind für einen Pyrrolidin oder Piperidinrest,
B für einen der Reste

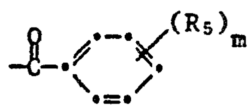 , 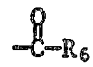 oder 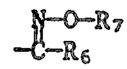 ,

m für 0, 1, 2 oder 3
$R_5$ für Fluor, Chlor, Brom, Nitro, Cyano, $C_1$-$C_4$-Alkyl, $C_1$-$C_4$-Alkoxy, $C_1$-$C_4$-Alkylthio, Triflormethyl, $C_1$-$C_4$-Alkylsulfinyl oder $C_1$-$C_4$-Alkylsulfonyl,
$R_6$ für $C_1$-$C_4$-Alkyl, Cyclopropyl, Cyclopentyl oder Cyclohexyl,
$R_7$ für Wasserstoff, $C_1$-$C_4$-Alkyl, $C_1$-$C_4$-Halogenalkyl, $C_3$-$C_4$-Alkenyl, $C_3$-$C_4$-Halogenalkenyl oder $C_3$-$C_4$-Alkinyl steht.

Bevorzugt sind die Verbindungen der Formel I

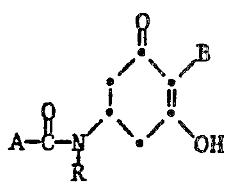

(I)

worin
R für Wasserstoff oder $C_1$-$C_3$-Alkyl,
A für $R_2$, $OR_3$ der $NR_3R_4$,
$R_2$ für $C_1$-$C_4$-Alkyl, Methoxymethyl oder Trifluormethyl,
$R_3$ für $C_1$-$C_4$-Alkyl, Cyclopropyl, Cyclohexyl, Benzyl oder gegebenenfalls durch Chlor, Methoxy oder Methyl substituiertes Phenyl
$R_4$ für Wasserstoff, Methyl, Methoxy oder Ethyl,
$R_3$ und $R_4$ ausserdem gemeinsam mit dem Stickstoffatom, an welches sie gebunden sind für einen Pyrrolidin oder Piperidinrest,
B für einen der Reste

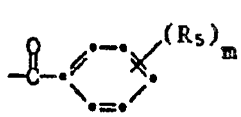 , 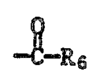 oder 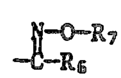 ,

m für 0, 1, 2 oder 3,
$R_5$ für Fluor, Chlor, Brom, Nitro, Methyl, Methoxy, Trifluormethyl, $C_1$-$C_4$-Alkylthio, $C_1$-$C_4$-Alkylsulfinyl oder $C_1$-$C_4$-Alkylsulfonyl,
$R_6$ für $C_1$-$C_6$-Alkyl, Cyclopropyl oder Cyclohexyl,
$R_7$ für Methyl, Ethyl, Allyl, Propargyl oder 3-Chlor-prop-2-en-1-yl steht

Besonders bevorzugt sind die Verbindungen der Formel I

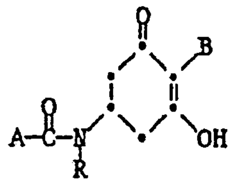

(I)

worin
R für Wasserstoff oder $C_1$-$C_3$-Alkyl,
A für $R_2$, $OR_3$ oder $NR_3R_4$,
$R_2$ für Methoxymethyl oder $C_1$-$C_4$-Alkyl,
$R_3$ für $C_1$-$C_4$-Alkyl oder gegebenenfalls durch Chlor oder Methyl substituiertes Phenyl
$R_4$ für Wasserstoff oder Methyl,
$R_3$ und $R_4$ ausserdem gemeinsam mit dem Stickstoffatom, an welches sie gebunden sind für einen Pyrrolidinrest,
B für einen der Reste

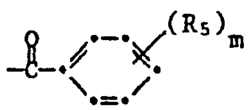 , 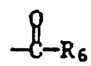 oder 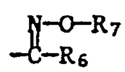 ,

m für 0, 1 oder 2,
$R_5$ für Chlor, Nitro, Trifluoromethyl, Methylthio, Methylsulfinyl oder Methylsulfonyl,
$R_6$ für $C_1$-$C_3$-Alkyl oder Cyclopropyl
$R_7$ für Methyl, Allyl, Ethyl oder 3-Chlor-prop-2-en-1-yl steht.

Aufgrund ihrer herbiziden, beziehungsweise pflanzenwuchsregulatorischen Wirkung sind die folgenden Einzelverbindungen zu nennen :
2-Butanoyl-5-ethoxycarbonylaminocyclohexan-1,3-dion,
2-Propanoyl-5-(N',N'-dimethylureido)-cyclohexan-1,3-dion,
2-Butanoyl-5-(N',N'-dimethylureido)-cyclohexan-1,3-dion,
5-Acetamido-2-propanoyl-cyclohexan-1,3-dion,
2-(2,4-Dichlorbenzoyl)-5-ethoxycarbonylamino-cyclohexan-1,3-dion,
2-(2,4-Dichlorbenzoyl)-5-(N'-isopropylureido)-cyclohexan-1,3-dion,
2-(2,4-Dichlorbenzoyl)-5-(N',N'-dimethylureido)-cyclohexan-1,3-dion,
5-(N'-tert-Butylureido)-2-(1-allyloxyiminobutyl)-cyclohexan-1,3-dion,
5-(N',N'-Dimethylureido)-2-(1-ethoxyiminopropyl)-cyclohexan-1,3-dion,
5-(N'-Phenylureido)-2-(1-ethoxyiminobutyl)-cyclohexan-1,3-dion,
5-(N'-Phenylureido)-2-[1-(3-chlorallyl)oxyiminobutyl]-cyclohexan-1,3-dion,
5-(N'-Phenylureido)-2-(1-ethoxyiminobutyl)-cyclohexan-1,3-dion,
5-(N'-Phenylureido)-2-(1-methoxyiminobutyl)-cyclohexan-1,3-dion,
5-[N'-(2-Methylphenyl)-ureido)-2-(1-ethoxyiminobutyl)-cyclohexan-1,3-dion und
5-[N'-(Chlorphenyl)-ureido]-2-(1-ethoxyiminobutyl)-cyclohexan-1,3-dion.

Die Cyclohexandione der Formel I liegen in einem tautomeren Gleichgewicht gemäss nachstehender Gleichung vor :

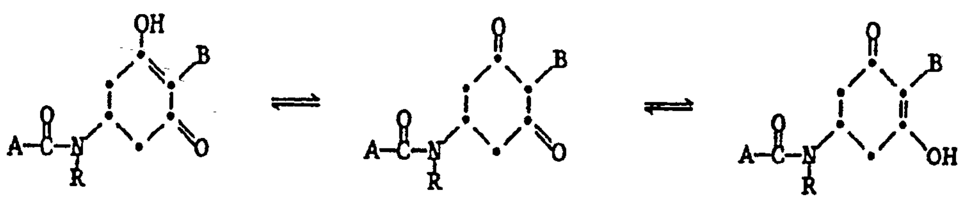

(I) (I') (I)

Die über das Gleichgewichtsstystem I ⇄I' ⇄I leicht enolisierbaren Diketone der Formel I bilden bereits mit relativ schwachen Basen Salze. In den daraus resultierenden Salzen bilden die Verbindungen der Formel I den anionischen Teil. Als geeignete Kationen sind zu nennen : Erdalkali-, Alkali- und Ammoniumionen. Unter Ammoniumionen sind neben dem unsubstituierten Ammoniumion auch die verschiedenen Mono-, Di-, Tri- und Tetraalkylammoniumionen zu verstehen. Durch Bildung geeigneter Salze kann unter anderem die Wasserlöslichkeit sowie insgesamt die Bioverfügbarkeit und das Wirkungsspektrum der Verbindungen der Formel I beeinflusst werden.

Die Verbindungen der Formel I können hergestellt werden, indem man

a) eine Cyclohexandion der Formel II mit einer Verbindung der

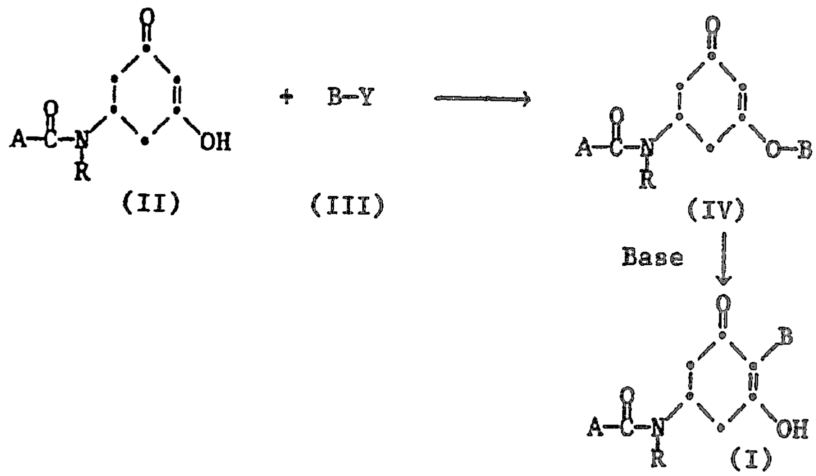


(II) (III) (IV) (I)

Formel III zu einer Verbindung der Formel IV umsetzt und diese anschliessend in Gegenward einer Base thermisch zu einer Verbindung der Formel I umlagert, wobei A, B und R wie zuvor definiert sind, aber mit der Einschränkung, dass B nicht für die Oximethergruppe

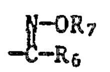

steht, und in der Y für eine unter den Reaktionsbedingungen abspaltbare nucleofuge Gruppe, wie Chlor, Brom oder $C_1$-$C_4$-Alkoxy steht,

oder indem man

b) ein Cyclohexandion der Formel II mit einer Verbindung

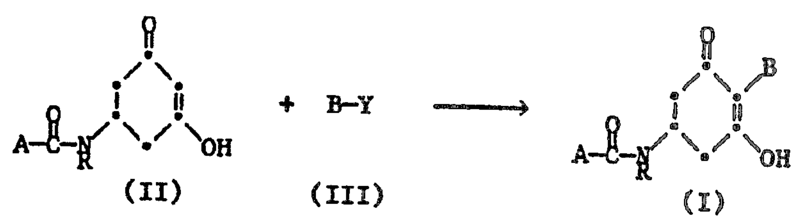

(II) (III) (I)

der Formel III, in der Y für eine Cyangruppe steht, in Gegenwart einer Base und $ZnCl_2$ zu einer Verbindung der Formel I umsetzt, wobei die Reste A, R und B in den Formeln I, II und III wie zuvor definiert sind, mit der Massgabe, dass B nicht für die Oximethergruppe

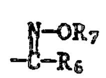

steht,

oder indem man

c) ein Cyclohexylketon der Formel Ia mit einem

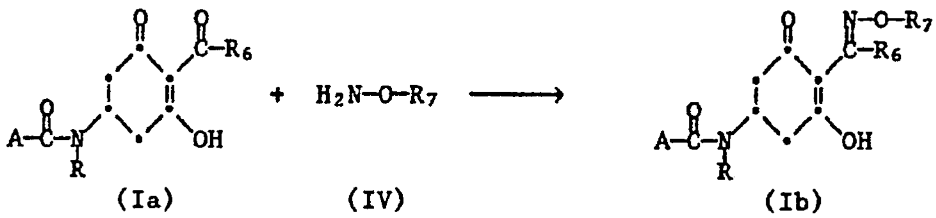

(Ia) (IV) (Ib)

Hydroxylamin der Formel IV zu einem Oxim der Formel Ib umsetzt.

Die als Zwischenprodukte verwendeten Cyclohexane der Formel II und IV sind ebenfalls neu. Sie sind wertvolle Edukte zur Herstellung von Verbindungen der Formel I.

Die Erfindung betrifft somit auch die neuen Cyclohexanone der Formel II

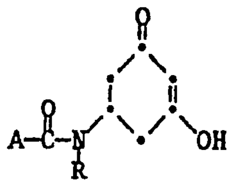

(II)

worin A und R im gleichen Umfang wie unter Formel I definiert sind und die neuen Cyclohexanone der Formel IV

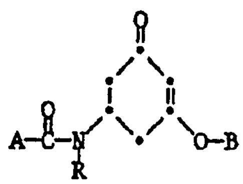

(IV)

worin A und R und B im gleichen Umfang wie unter Formel I definiert sind, mit der Massgabe, dass B nicht für die Oximethergruppe

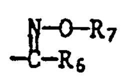

steht.

Die Verbindungen der Formel II können hergestellt werden, indem man

a) ein Resorcin der Formel V nach an sich bekannten Verfahren hydriert

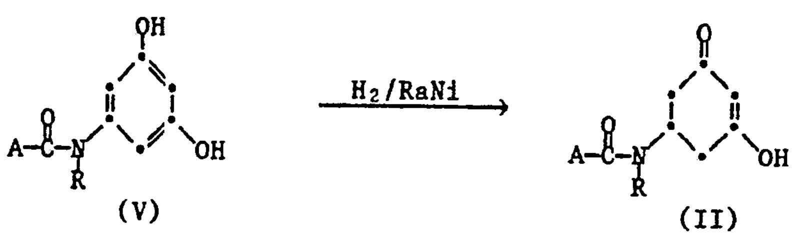


(V) (II)

wobei die Substituenten A und R wie zuvor definiert sind,
oder indem man
b) ein Isocyanat der Formel VI mit einem Alkohol oder einem Amin der Formel VII bzw. VIII

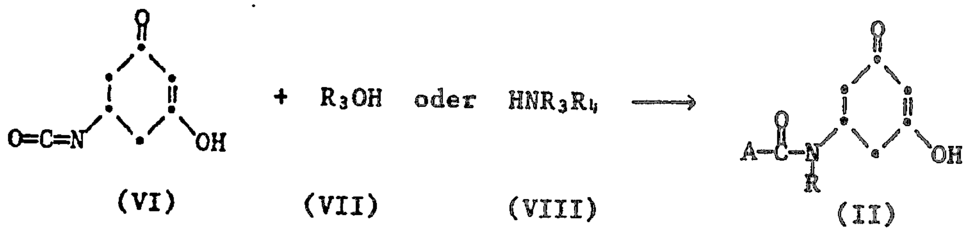


(VI) (VII) (VIII) (II)

zu einem Urethan oder Harnstoff der Formel II, in der R für Wasserstoff und A für $R_3O$ oder $NR_3R_4$ steht und $R_3$ und $R_4$ wie zuvor definiert sind, umsetzt.

Die als Ausgangsverbindung benötigten Resorcine der Formel V können analog zu bekannten Verfahren [J. Org. Chem. 40 (1975), 1556 ; Helv. Chim. Acta 56 (1973) 510] durch die Umsetzung von Phloroglucin IX mit Aminen der Formel X zu den Aminoresorcinen der Formel XI umgesetzt werden. Ausgehend von den Aminoresorcinen XI sind die N-acylierten

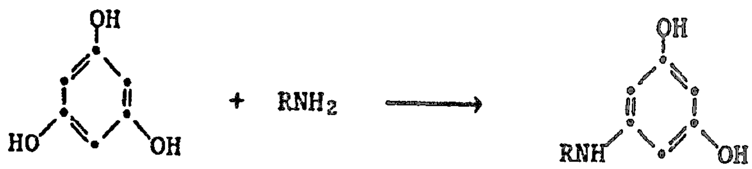


(IX) (X) (XI)

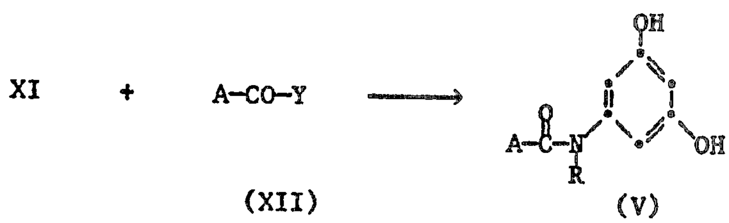


(XII) (V)

Verbindungen der Formel V durch Umsetzung mit XII leicht zugänglich, Die Reste R und A im vorstehenden Reaktionsschema entsprechen der Definition der Formel I, Y steht für eine unter den Reaktionsbedingungen abspaltbare nucleofuge Gruppe, wie Halogen, $C_1$-$C_4$-Alkoxy oder Phenoxy.

Die Urethane und Harnstoffe der Formel II, in denen A für $OR_3$ oder $NR_3R_4$ steht können auf einem alternativen Syntheseweg ausgehend von der Cyclohexancarbonsäure XIII hergestellt werden.

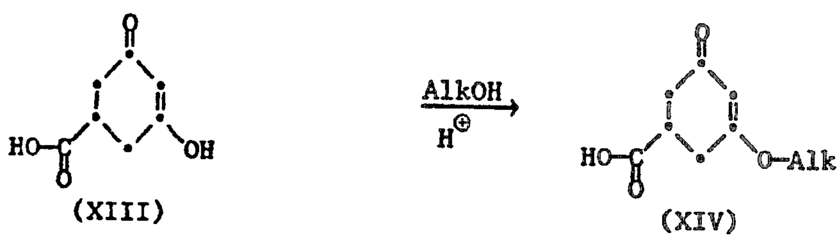


(XIII) (XIV)

Dazu wird aus XIII zunächst der Monoether XIV mit Alk = $C_1$-$C_4$-Alkyl hergestellt und dieser dann nach Curtius via Azid in das Isocyanat XV überführt.

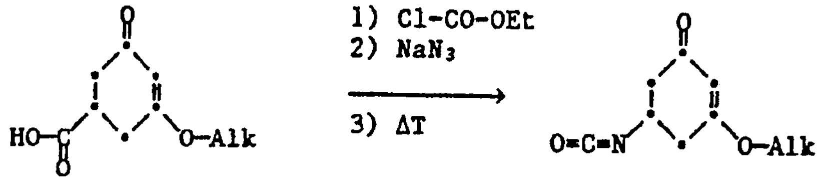


(XIV) (XV)

XV + ROH oder $HNR_3R_4$ 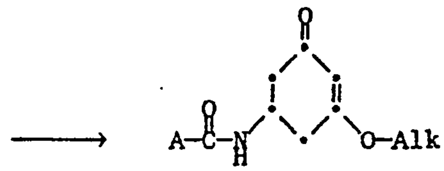

(VII) (VIII) (XVI)

XVI 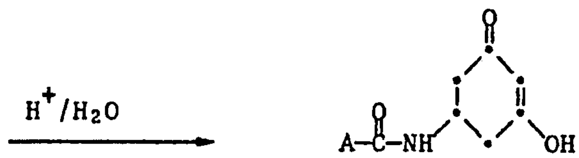


II

Aus XV ist dann durch Addition der Alkohole VII bzw. der Amine VIII Verbindung XVI leicht zugänglich. Aus XVI kann durch Etherspaltung das gewünschte Endprodukt II generiert werden.

Die neuen Verbindungen der Formel II liegen im tautomeren Gleichgewicht II ⇄II' ⇄II mit der Diketoform II' vor

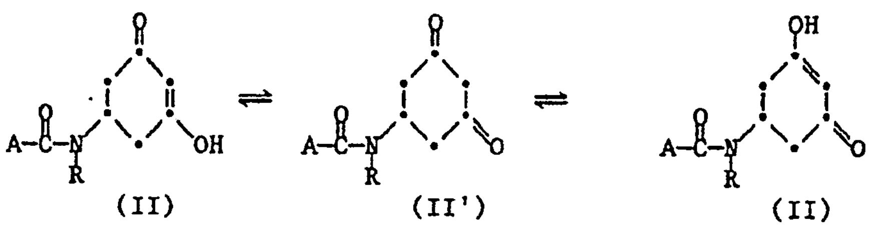

(II) (II') (II)

Die Verbindungen der Formel I sind hochaktive Pflanzenwirkstoffe, welche sich bei geeigneten Aufwandmengen hervorragend als Selektivherbizide zur Unkrautbekämpfung in Nutzpflanzenkulturen eignen. Das heisst, bei diesen Aufwandmengen zeichnen sich die Wirkstoffe der Formel I durch gute selektiv-herbizide Eigenschaft gegen Umkräuter aus. Kulturpflanzen wie Roggen, Gerste, Hafer, Weizen, Mais, Hirse, Reis, Baumwolle und Soja bleiben bei niedrigen Aufwandmengen praktisch ungeschädigt. Bei gesteigerten Aufwandmengen werden die Kuturpflanzen nur geringfügig in ihrem Wachstum beeinflusst. Werden sehr hohe Aufwandmengen appliziert, entfalten die Substanzen der Formel I totalherbizide Eigenschaften.

Die selektiv-herbizide Wirkung der erfindungsgemässen Verbindungen wird sowohl bei der preemergenten als auch der postemergenten Anwendung festgestellt. Diese Wirkstoffe können daher im vorauflaufverfahren und im Nachauflaufverfahren zur selektiven Unkrautbekämpfung mit gutem Erfolg verwendet werden.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzte Leguminosen durch die Verbindungen der Formel I in ihrem Wachstum selektiv gehemmt werden, so dass zwar die Bodenerosion zwischen den Kulturpflanzen verhindert wird, die "cover crops" hedoch nicht zur Konkurrenz für die Kultur werden können.

Eine Hemmung des vegetativen Wachstums erlaubt bei vielen Kulturpflanzen eine dichtere Anpflanzung

der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten-und Fruchtbildung zugute kommen, während das vegetative Wachstum eingeschränkt wird.

Bei grösseren Aufwandmengen an Wirkstoffen der Formel I werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na– oder K-Salze der Oel– oder Stearinsäure, oder von natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na– oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na–, Ca– oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18

Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben:

1985 International Mc Cutcheon's Emulsifiers a Detergents Glen Rock, NJ. USA ;

H. Stache, "Tensid-Taschenbuch", 2. Auflage., C. Hanser Verlag, München, Wien, 1981 ;

M. and J. Ash. "Encyclopedia of Surfactants", Vol. I-III, Chemical Publishing Co., New York, 1980-1981.

Die Wirkstoffzubereitungen enthalten in der Regel 0,1 bis 95%, insbesondere 0,1 bis 80% Wirkstoff der Formel I, 1 bis 99,9% eines oder mehrerer fester oder flüssiger Zusatzstoffe und 0 bis 25% eines Tensides.

Insbesondere setzen sich bevorzugte Formulierungen folgendermassen zusammen : (% = Gewichtsprozent).

Emulgierbare Konzentrate :

| | |
|---|---|
| Aktiver Wirkstoff : | 1 bis 20%, bevorzugt 5 bis 10% |
| oberflächenaktive Mittel : | 5 bis 30%, vorzugsweise 10 bis 20% |
| flüssige Trägermittel : | 50 bis 94%, vorzugsweise 70 bis 85% |

Stäube :

| | |
|---|---|
| Aktiver Wirkstoff : | 0,1 bis 10%, vorzugsweise 0,1 bis 1% |
| festes Trägermittel : | 99,9 bis 90%, vorzugsweise 99,9 bis 99% |

Suspensions-Konzentrate :

| | |
|---|---|
| Aktiver Wirkstoff : | 5 bis 75%, vorzugsweise 10 bis 50% |
| Wasser : | 94 bis 25%, vorzugsweise 88 bis 30% |
| oberflächenaktives Mittel : | 1 bis 40%, vorzugsweise 2 bis 30% |

Benetzbares Pulver :

| | |
|---|---|
| Aktiver Wirkstoff : | 0,5 bis 90%, vorzugsweise 1 bis 80% |
| oberflächenaktives Mittel : | 0,5 bis 20%, vorzugsweise 1 bis 15% |
| festes Trägermittel : | 5 bis 95%, vorzugsweise 15 bis 90% |

Granulate :

| | |
|---|---|
| Aktiver Wirkstoff : | 0,5 bis 30%, vorzugsweise 3 bis 15% |
| festes Trägermittel : | 99,5 bis 70%, vorzugsweise 97 bis 85% |

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,001 bis 4 kg AS/ha, vorzugsweise 0,005 bis 1 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellung der Ausgangsverbindungen

H.0.1. 5-Aminoresorcin

500 g (4 Mol) Phloroglucin werden portionsweise in 4 l einer 30%igen wässrigen Ammoniaklösung eingetragen und sodann noch während 40 min. $NH_3$-Gas eingeleitet. Nach vierundzwanzigstündigem Rühren bei Raumtemperatur wird die so erhaltene klare braune Lösung bei 45°C am Wassertrahlvakuum eingeengt. Der zurückbleibende Feststoff wird mit Toluol und wenig Aceton verrieben, abgenutscht und bei 45°C im Vakuum getrocknet.

Man isoliert 464 g (92,8%) der Titelverbindung der Formel

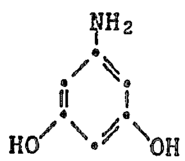

als Kristalle vom Smp. 150°C.

H.0.2. 5-(Methylamino)-resorcin

Zu einer Lösung von 189 g (1.5 Mol) Phloroglucin in 1500 ml Dimethylformamid und 1000 ml Wasser werden bei 25°C 140 ml einer 40%-igen wässrigen Methylaminlösung getropft. Es wird noch 20 Stunden bei Raumtemperatur nachgerührt und dann bei 70°C im Vakuum das Lösungsmittel abdestilliert und im Hochvakuum getrocknet.

Der Rückstand wird dann nochmals mit Diethylether verrieben und getrocknet.

Man isoliert 173 g (83%) der Titelverbindung der Formel

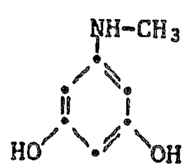

als Kristalle vom Smp. 130°C.

H.0.3. 3-Methoxy-cyclohex-3-en-5-on-carbonsäure

Zu einer Lösung von 1560 g (10 Mol) Cyclohexan-3,5-dion-carbonsäure in 3 l Methanol werden 10 ml konzentrierter Schwefelsäure gegeben und für 3 Stunden unter Rühren bei 20-25°C belassen. Danach wird mit 3 l Diethylether versetzt und von dem ausgefallenen Produkt abgesaugt.

Man isoliert 1508 g (88,7%) der Titelverbindung der Formel

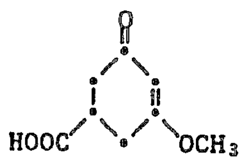

H.1. Herstellung von Verbindungen der Formel V

H.1.1. N-Isopropyl-N'-(3,5-dihydroxyphenyl)-harnstoff

Zu einer Lösung von 62,5 g (0.5 Mol) 5-Aminoresorcin in 1000 ml Dioxan werden innerhalb von 20 min 52 ml (0.53 Mol) Isopropylisocyanat zugetropft, wobei die Innentemperatur auf 35°C ansteigt. Es wird noch 5 Stunden bei Raumtemperatur nachgerührt und dann mit 1000 ml Petrolether versetzt, wobei die Titelverbindung feinkristallin ausfällt.

Man isoliert 162 g (84%) der Titelverbindung der Formel

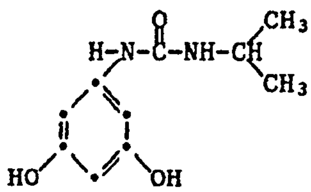

• 2 $C_4H_8O_2$

als Kristalle vom Smp. > 210°C (Zers.) ; die Kristalle der Titelverbindung enthalten 2 Moläquivalente Dioxan. (Verb. No. 1.04).

H.1.2. N-Isopropyl-N'-methyl-N'-(3,5-dihydroxyphenyl)-harnstoff

Analog zu Beispiel H.1.1. erhält man aus 69,5 g (0,5 Mol) 5-Methylaminoresorcin und 52 ml (0.53 Mol) Isopropylisocyanat 113 g (84%) der Titelverbindung der Formel

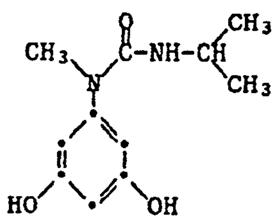

• 1/2 $C_4H_8O_2$

als Kristalle vom Smp. > 230°C (Zers.) ; die Kristalle der Titelverbindung enthalten 0,5 Moläquivalente Dioxan (Verb. No. 1.022).

H.1.3. 5-Acetamidoresorcin

Zu einer Lösung von 12,5 g (0,1 Mol) 5-Aminoresorcin in 100 ml Dioxan werden 10,4 ml (0,11 Mol) Acetanhydrid so zugetropft, dass die Innentemperatur 50°C nicht übersteigt. Es wird noch 2 Stunden nachgerührt und dann mit 200 ml Petrolether versetzt, wobei das Produkt ausfällt.

Man isoliert 15,3 g (91,7%) der Titelverbindung der Formel

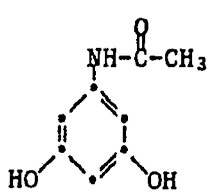

als Kristalle vom Smp. > 200°C (Zers.) (Verb. No. 1.011).

H. 1.4. N-(3,5-Dihydroxyphenyl)-carbaminsäure-phenylester

Zu einem Gemisch von 375 g 5-Aminoresorcin, 270 g Natriumacetat und 30 ml Dimethylformamid in 3 l Dioxan werden unter Kühlen bei 15-20°C 410 ml Chlorameisensäurephenylester getropft. Es wird noch 1

Stunde bei 20-25°C nachgerührt und dann mit 3 l Essigsäureethylester verdünnt und von dem ausfallenden Natriumchlorid abfiltriert. Nach Abdestillieren des Lösungsmittels wird aus Petrolether umkristallisiert.

Man isoliert 868 g (87%) der Titelverbindung der Formel

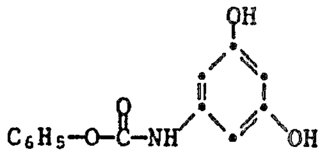

1 Dioxan

als Kristalle mit Smp. 176°C (Zers.) (Verb. No. 1.031).

H.1.5. N,N-Dimethyl-N'-(3,5-dihydroxyphenyl)-harnstoff

Zu einer Lösung von 840 g N-(3,5-Dihydroxyphenyl)-carbaminsäure-phenylester in 2,5 l Ethanol werden 900 ml einer 33%-igen ethanolischen Dimethylaminlösung gegeben. Es wird für weitere 10 Stunden bei 20 bis 25°C gerührt und danach das Lösungsmittel im Vakuum abdestilliert.

Der so erhältliche Brei wird mehrfach mit Diethylether verrieben und abgenutscht.

Man isoliert 469 g (95%) der Titelverbindung der Formel

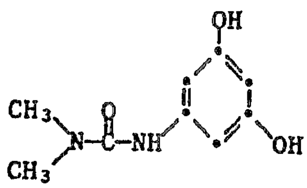

als Kristalle vom Smp. 230°C (Verb. No.1.014).

Analog sind die Verbindungen der Tabelle 1 herstellbar.

Tabelle 1

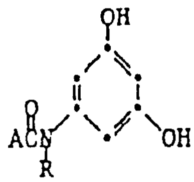

| Nr. | R | A | phys. Konstante |
|---|---|---|---|
| 1.001 | H | $NHCH_3$ | Smp. 215°C (• 1 Dioxan) |
| 1.002 | H | $NHC_2H_5$ | Smp. 98 - 100°C (• 1 1/4 Dioxan) |
| 1.003 | H | $NHC_3H_7$ | |
| 1.004 | H | $NHC_3H_7$ (i) | Smp. > 210°C (Zers.) (• 2 Dioxan) |
| 1.005 | H | $NHC_4H_9$ (n) | |
| 1.006 | H | $NHC_4H_9$ (t) | Smp. 115 - 118° (• 1 Dioxan) |
| 1.007 | H | $OCH_3$ | |
| 1.008 | H | $OC_2H_5$ | Smp. 152 - 153° |
| 1.009 | H | $OC_3H_7$ | |
| 1.010 | H | $OC_3H_7$ (i) | |
| 1.011 | H | $CH_3$ | Smp. > 200° Zers. |
| 1.012 | H | $C_2H_5$ | Smp. > 190° Zers. |
| 1.013 | H | $C_4H_9$ | |
| 1.014 | H | $N(CH_3)_2$ | Smp. > 230° C. |
| 1.015 | H | $N(C_2H_5)_2$ | |
| 1.016 | H | 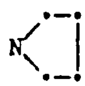 | Smp. > 230° C. |
| 1.017 | H | -NH- 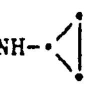 | |
| 1.018 | H | -O- 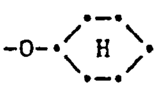 | |
| 1.019 | H | 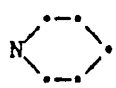 | |

Tabelle 1 (Fortsetzung)

| Nr. | R | A | phys. Konstante |
|---|---|---|---|
| 1.020 | $-CH_3$ | $NHCH_3$ | Smp. > 170° C. |
| 1.021 | $-CH_3$ | 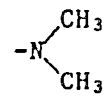 | |
| 1.022 | $-CH_3$ | $-NH-C_3H_7$ (i) | Smp. > 230° C. (• 1/2 Dioxan) |
| 1.023 | $-CH_3$ | $-OCH_3$ | |
| 1.024 | $-CH_3$ | $-OC_3H_7$ (i) | |
| 1.025 | $-CH_3$ | $-CH_3$ | Smp. > 185°C (Zers.) |
| 1.026 | $-CH_3$ | $-C_2H_5$ | Smp. > 100°C (Zers.) (• 1/4 Dioxan) |
| 1.027 | $-C_2H_5$ | $-NHCH_3$ | |
| 1.028 | $-C_2H_5$ | $-N(CH_3)_2$ | |
| 1.029 | $-C_3H_7$ (i) | $-NHCH_3$ | |
| 1.030 | $-CH_3$ | 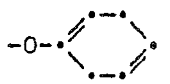 | Smp. 140 - 143° (• 0,5 Dioxan) |
| 1.031 | -H | 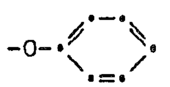 | Smp. 175 - 176° (• 1/2 Dioxan) |
| 1.032 | $-CH_3$ | 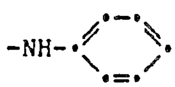 | Smp. > 150° C. |
| 1.033 | H | 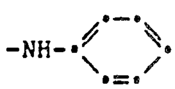 | Smp. 189 - 191° |
| 1.034 | H | $C_3H_7$ (i) | Smp. > 220° C. (Zers.) (• 1/2 Dioxan) |
| 1.035 | $CH_3$ | $C_3H_7$ (i) | Smp. > 170° C. (Zers.) |
| 1.036 | $CH_3$ | $CH_2OCH_3$ | Smp. 170° C. (Zers.) |
| 1.037 | H | 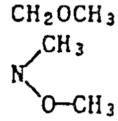 | |
| 1.038 | $CH_3$ | 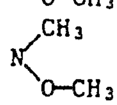 | |
| 1.039 | H | $CF_3$ | |
| 1.040 | H | $CH_2OCH_3$ | |

H.2 Herstellung von Verbindungen der Formel II

H 2.1. N-Isopropyl-N'-(cyclohexan-3,5-dion-1-yl)-harnstoff

Eine Lösung von 0,1 Mol (154 g der nach Beispiel H 1.1 erhältlichen Verbindung) N-Isopropyl-N'-(3,5-dihydroxyphenyl)-harnstoff in 440 ml 1 N-NaOH werden 18 Stunden bei 60-65°C/10 bar in Gegenwart von 30 g Ra-Ni-$H_2O$ hydriert. Nach Abfiltrieren des Katalysators wird das Filtrat mit HCl auf pH 1 angesäuert und auf 5°C gekühlt, wobei das Produkt ausfällt.

Nach dem Abnutschen wird mit Wasser gespült und bei 60°C/0,1 bar getrocknet.

Man isoliert 76 g (90%) der Titelverbindung der Formel

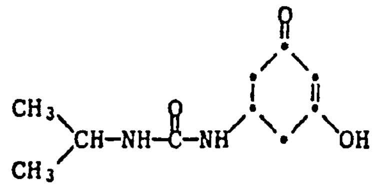

als Kristalle vom Smp. > 100°C (Zers.) (Verb. No. 2.004).

H.2.2. N-Isopropyl-N'-methyl-N'-(cyclohexan-3,5-dion-1-yl)-harnstoff

Analog zu Beispiel H.2.1 erhält man aus 0.37 Mol (100 g der nach Beispiel H 1.2 erhältlichen Verbindung 1.022) N-Isopropyl-N'-methyl-N'-(3,5-dihydroxyphenyl)-harnstoff beim Hydrieren 58,2 g der Titelverbindung der Formel

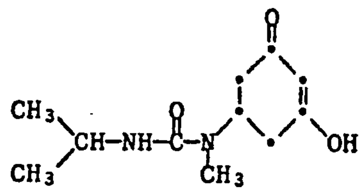

als Kristalle vom Smp. 93-95°C (langsame Zers.) (Verb. No. 2.022)

H.2.3. 1-Acetamido-cyclohexan-3,5-dion

Eine Lösung von 16.7 g (0.1 Mol) 5-Acetamidoresorcin in 110 ml 0,1 N NaOH wird in Gegenwart von 10 g Ra-Ni-$H_2O$ bei 60-65°C/10 bar 50 Stunden hydriert. Nach Abfiltrieren des Katalysators und Ansäuern der wässrigen Lösung wird bei 50°C zur Trockene eingeengt, der Rückstand mit 200 ml Ethanol und 0,1 ml konz. Schwefelsäure versetzt und vom ausfallenden Natriumchlorid abgetrennt und das Filtrat erneut bis zur Trockene eingeengt. Der Rückstand wird in 200 ml Tetrahydrofuran und 10 ml 1N-Salzsäure aufgenommen, 15 Stunden bei Raumtemperatur belassen und die so erhaltene Suspension über einem Molekularsieb getrocknet und nach Abtrennen des Molekularsiebes erneut zur Trockene eingeengt.

Man isoliert 11,5 g (68%) der Titelverbindung der Formel

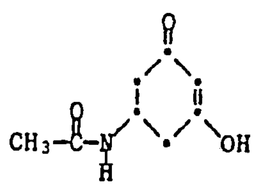

als Kristalle vom Smp. 181-183°C (Verb. No. 2.011).

Analog sind die Verbindungen der Tabelle 2 erhältlich.

Tabelle 2

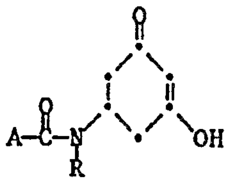

| Nr. | R | A | phys. Daten |
|---|---|---|---|
| 2.001 | H | $NHCH_3$ | fest |
| 2.002 | H | $NHC_2H_5$ | |
| 2.003 | H | $NHC_3H_7$ | |
| 2.004 | H | $NHC_3H_7$ (i) | > 100° Z. |
| 2.005 | H | $NHC_4H_9$ (n) | |

Tabelle 2 (Fortsetzung)

| Nr. | R | A | phys. Daten |
|---|---|---|---|
| 2.006 | H | $NHC_4H_9$ (t) | > 180° Z. |
| 2.007 | H | $OCH_3$ | Smp. 146 - 150° |
| 2.008 | H | $OC_2H_5$ | Smp. 172 - 174° |
| 2.009 | H | $OC_3H_7$ | Smp. 156 - 160° |
| 2.010 | H | $OC_3H_7$ (i) | |
| 2.011 | H | $CH_3$ | Smp. 181 - 183 |
| 2.012 | H | $C_2H_5$ | |
| 2.013 | H | $C_4H_9$ (n) | |
| 2.014 | H | $N(CH_3)_2$ | > 180° Z. |
| 2.015 | H | $N(C_2H_5)_2$ | |
| 2.016 | H | 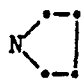 | > 190° Z. |
| 2.017 | H | 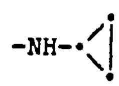 | |
| 2.018 | H | 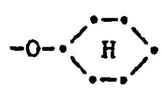 | |
| 2.019 | H | 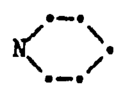 | |
| 2.020 | $CH_3$ | $NHCH_3$ | fest |
| 2.021 | $CH_3$ | $N(CH_3)_2$ | |
| 2.022 | $CH_3$ | $NHC_3H_7$ (i) | Smp. 93 - 95° |
| 2.023 | $CH_3$ | $-OCH_3$ | |
| 2.024 | $CH_3$ | $-OC_3H_7$ (i) | |
| 2.025 | $CH_3$ | $CH_3$ | fest |
| 2.026 | $CH_3$ | $C_2H_5$ | |
| 2.027 | H | 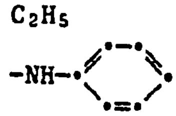 | Smp. > 188° Z. |
| 2.028 | H | 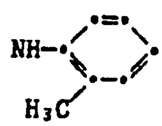 | fest |
| 2.029 | H | 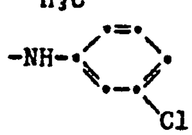 | |
| 2.030 | H | 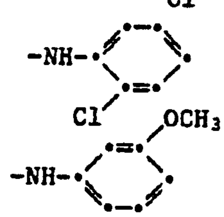 | Smp. 170° Z. |
| 2.031 | H | | fest |

Tabelle 2 (Fortsetzung)

| Nr. | R | A | phys. Daten |
|---|---|---|---|
| 2.032 | H | 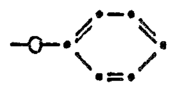 | |
| 2.033 | H | 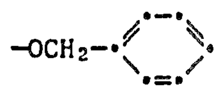 | Smp. 180° Z. |
| 2.034 | H | 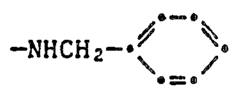 | |
| 2.035 | H | 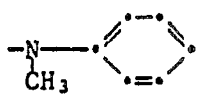 | |
| 2.036 | $C_2H_5$ | $-NHCH_3$ | |
| 2.037 | $C_2H_5$ | $-N(CH_3)_2$ | |
| 2.038 | $C_3H_7$ (i) | $-NHCH_3$ | |
| 2.039 | H | $C_3H_7$ (i) | Smp. 204°C |
| 2.040 | $CH_3$ | $C_3H_7$ (i) | |
| 2.041 | $CH_3$ | $CH_2-O-CH_3$ | |
| 2.042 | H | 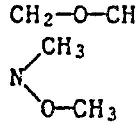 | |
| 2.043 | $CH_3$ | 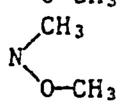 | |
| 2.044 | H | $CF_3$ | |
| 2.045 | H | $CH_2-O-CH_3$ | |

H.3.1. N,N-Dimethyl-N'-(3-propionyloxy-cyclohex-3-en-5-on-1-yl)-harnstoff

Eine Lösung von 13,5 g (68 mMol) N,N-Dimethyl-N'-(cyclohexan-3,5-dion-1-yl)-harnstoff in 10,5 ml Triethylamin und 70 ml Essigsäuerethylester werden unter Kühlen auf 20-25°C mit 6,4 ml (71 mMol) Propionsäurechlorid versetzt und 15 Stunden bei Raumtemperatur nachgerührt. Danach wird von dem ausgefallenen Triethylamin-Hydrochlorid abfiltriert und das Filtrat zur Trockene eingeengt.

Man isoliert 16.8 g (97%) der Titelverbindung der Formel

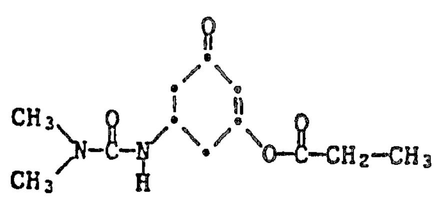

als wachsartigen Festkörper vom Smp. 69-74°C.

H.3.2. N-Phenyl-N'-(3-methoxycyclohex-3-en-5-on-1-yl)-harnstoff

Zu einer Suspension von 34 g (0.2 Mol) 3-Methoxy-cyclohex-3-en-5-on-carbonsäure in 500 ml Toluol werden 30,7 ml (0,22 Mol) Triethylamin gegeben, wobei eine klare Lösung entsteht. Zu dieser Lösung werden bei 0-5°C 21,5 ml (0,22 Mol) Chlorameisensäure-ethylester getropft. Es wird noch 1 Stunde bei 0-5°C nachgerührt

und zu der dabei entstehenden Suspension dann eine Lösung von 16,25 g (0.25 Mol) Natriumazid in 100 ml Wasser getropft. Es wird erneut eine Stunde bei 0-5°C nachgerührt und dann mit 200 ml Toluol versetzt.

Die organische Phase wird mit 200 ml Natriumbicarbonatlösung (50%) gewaschen und über Molekularsieb (4 Å) getrocknet. Nach Entfernen des Molekularsiebs wird mit 20 ml (0,22 Mol) Anilin versetzt. Die Lösung wird dann langsam auf 90°C erwärmt. Ab 80°C beginnt sich Stickstoff zu entwickeln. Es wird für 2 Stunden bei 90°C belassen und das Produkt anschliessend durch Zusatz von Hexan ausgefällt.

Man isoliert 31 g (60%) der Titelverbindung der Formel

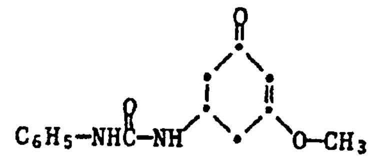

als Kristalle vom Smp. 183-185°C.

H.3.3. N-Phenyl-N'-(cyclohexan-3,5-dion-1-yl)-harnstoff

30 g N-Phenyl-N'-(3-methoxycyclohex-3-en-5-on-1-yl)-harnstoff werden in 230 ml Tetrahydrofuran, 11.5 ml Wasser und 1 ml 37%-iger Salzsäure 12 Stunden bei Raumtemperatur gerührt. Anschliessend wird im Wasserstrahlvakuum bis zur Trockne eingeengt und der Rückstand mit Diethylether verrieben, abgesaugt und getrocknet.

Man isoliert 28 g (98,9%) der Titelverbindung der Formel

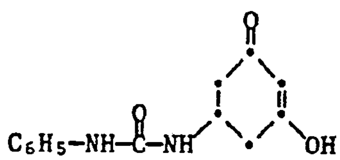

als Kristalle vom Smp. 188°C (Zers.).

H.4 Herstellung von Verbindungen der Formel I

H.4.1. 5-(N,N-Dimethylureido)-2-propionyl-cyclohexan-1,3-dion

Eine Lösung von 12,7 g (50 mMol) N,N-Dimethyl-N'-(3-propionyloxy-cyclohex-3-en-5-on-1-yl)-harnstoff in 100 ml Dichlorethan wird zusammen mit 6,1 g (50 mMol) 4-Dimethylaminopyridin 15 Stunden bei Raumtemperatur gerührt. Anschliessend wird mit 0,5 N Salzsäure (100 ml) neutralisiert, die organische Phase über $MgSO_4$ getrocknet und zur Trockene eingeengt.

Man isoliert 8.2 g (64.6%) der Titelverbindung der Formel

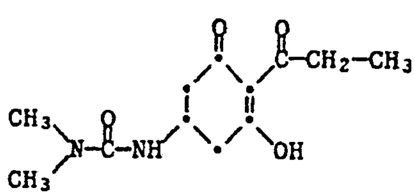

als Kristalle vom Smp. 136-138°C (Verb. 4.029).

H.4.2. N-Isopropyl-N'-[4-(4-chlor-2-nitrobenzoyl)-cyclohexan-3,5-dion-1-yl]-harnstoff

Zu einer Lösung von 140 mMol N-Isopropyl-N'-(3-hydroxycyclohex-3-en-5-on-1-yl)-harnstoff in 80 ml Dichlormethan und 23 ml (0,16 Mol) Triethylamin werden unter Kühlen 8,8 g (40 mMol) 4-Chlor-2-nitrobenzo-

ylchlorid getropft und noch 1 Stunde bei Raumtemperatur nachgerührt. Danach wird mit 1 ml Acetoncyanhydrin versetzt und weitere 15 Stunden bei Raumtemperatur gerührt. Das Reaktionsgemisch wird eingedampft und mit 400 ml 0,5 N Salzsäure verrührt. Das ausgefallene Produkt wird abgetrennt, mit Wasser gewaschen und getrocknet. Die Rohausbeute beträgt 174 g. Zur Reinigung wird das Produkt in das Natriumsalz überführt und erneut durch Säurezusatz gefällt.

Man isoliert 7.7 g (53.5%) der Titelverbindung der Formel

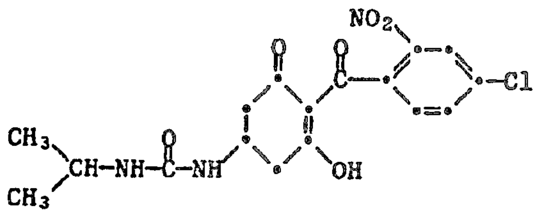

als Kristalle vom Smp. > 160°C (Zers.) (Verb. No. 4.010).

Analog sind die Verbindungen der Tabelle 3 und 4 erhältlich.

Tabelle 3

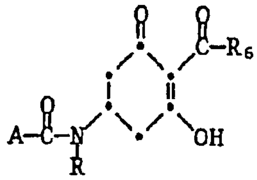

| Nr. | R | A | $R_6$ | Physikal. Daten |
|---|---|---|---|---|
| 3.001 | H | $NHCH_3$ | $-C_3H_7$ | |
| 3.002 | H | $NHCH_3$ | 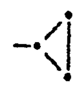 | |
| 3.003 | H | $NHC_2H_5$ | $-C_2H_5$ | |
| 3.004 | H | $NHC_2H_5$ | $-C_3H_7$ | |
| 3.005 | H | $NHC_3H_7$ (i) | $-C_2H_5$ | Smp. 188 - 190°C |
| 3.006 | H | $NHC_3H_7$ (i) | $-C_3H_7$ | |
| 3.007 | H | $NHC_3H_7$ (i) | $-C_4H_9$ | |
| 3.008 | H | $NHC_3H_7$ (n) | $-C_3H_7$(i) | |
| 3.009 | H | $NHC_4H_9$ (n) | $-CH_3$ | |
| 3.010 | H | $NHC_4H_9$ (n) | $-C_2H_5$ | |
| 3.011 | H | $NHC_4H_9$ (t) | $-C_2H_5$ | Smp. 174 - 175°C |
| 3.012 | H | $NHC_4H_9$ (t) | $-C_3H_7$ | Smp. 141 - 143°C |
| 3.013 | H | $NHC_4H_9$ (t) | 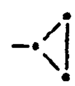 | Smp. 195 - 196°C |
| 3.014 | H | $NHC_3H_7$ (i) | 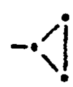 | Smp. 208 - 209°C |
| 3.015 | H | $OCH_3$ | $-C_2H_5$ | |
| 3.016 | H | $OCH_3$ | $-C_4H_9$ | |
| 3.017 | H | $OC_2H_5$ | $-C_2H_5$ | |
| 3.018 | H | $OC_2H_5$ | $-C_3H_7$ | Smp. 102 - 104°C |
| 3.019 | H | $OC_3H_7$ | $-CH_3$ | |
| 3.020 | H | $OC_3H_7$ (i) | $-C_2H_5$ | |
| 3.021 | H | $C_3H_7$ (i) | 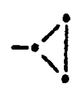 | |
| 3.022 | H | $CH_3$ | $C_2H_5$ | Smp. 159 - 161°C |
| 3.023 | H | $CH_3$ | $C_3H_7$ | Smp. 124 - 126°C |
| 3.024 | H | $CH_3$ | 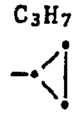 | Smp. 148 - 150°C |
| 3.025 | H | $C_2H_5$ | 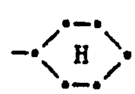 | |

Tabelle 3 (Fortsetzung)

| Nr. | R | A | $R_6$ | Physikal. Daten |
|---|---|---|---|---|
| 3.026 | H | $C_2H_5$ | $CH_3$ | |
| 3.027 | H | $C_4H_9$ | $CH_3$ | |
| 3.028 | H | $C_4H_9$ | $C_2H_5$ | |
| 3.029 | H | $N(CH_3)_2$ | $C_2H_5$ | Smp. 136 - 138°C |
| 3.030 | H | $N(CH_3)_2$ | $C_3H_7$ | Smp. 114 - 117°C |
| 3.031 | H | $N(CH_3)_2$ | $C_6H_{13}$ | |
| 3.032 | H | $N(C_2H_5)_2$ | $C_2H_5$ | |
| 3.033 | H | $N(C_2H_5)_2$ | $C_3H_7$ | |
| 3.034 | H | $N(C_2H_5)_2$ | 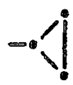 | |
| 3.035 | H | 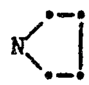 | $C_2H_5$ | |
| 3.036 | H | 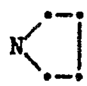 | $C_3H_7$ | |
| 3.037 | H | 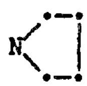 | 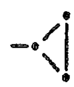 | Smp. 152 - 154°C |
| 3.038 | H | 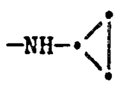 | $C_3H_7$ (i) | |
| 3.039 | H | 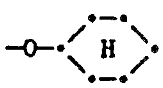 | $C_2H_5$ | |
| 3.040 | H | 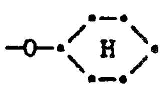 | 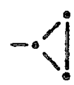 | |
| 3.041 | H | 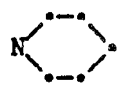 | $C_2H_5$ | |
| 3.042 | $CH_3$ | $NHCH_3$ | $C_2H_5$ | |
| 3.043 | $CH_3$ | $N(CH_3)_2$ | $C_2H_5$ | |
| 3.044 | $CH_3$ | $N(CH_3)_2$ | $C_3H_7$ | |
| 3.045 | $CH_3$ | $N(CH_3)_2$ | 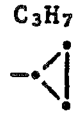 | |
| 3.046 | $CH_3$ | $NHC_3H_7$ (i) | $C_2H_5$ | |
| 3.047 | $CH_3$ | $NHC_3H_7$ (i) | 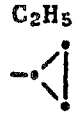 | Smp. 145 - 147°C |
| 3.048 | $CH_3$ | $NHC_3H_7$ (i) | $CH_3$ | |
| 3.049 | $CH_3$ | $OCH_3$ | $C_2H_5$ | |
| 3.050 | $CH_3$ | $OCH_3$ | $C_3H_7$ | |
| 3.051 | $CH_3$ | $-OC_3H_7$ (i) | $C_2H_5$ | |

Tabelle 3 (Fortsetzung)

| Nr. | R | A | $R_6$ | Physikal. Daten |
|---|---|---|---|---|
| 3.052 | $CH_3$ | $-OC_3H_7$ (i) | 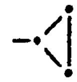 | |
| 3.053 | $CH_3$ | $CH_3$ | $C_2H_5$ | Oel |
| 3.054 | $CH_3$ | $CH_3$ | 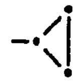 | |
| 3.055 | $CH_3$ | $CH_3$ | $C_3H_7$ | |
| 3.056 | $CH_3$ | $C_2H_5$ | $C_2H_5$ | |
| 3.057 | $CH_3$ | $C_2H_5$ | $C_3H_7$ | |
| 3.058 | H | 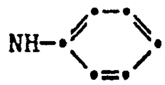 | $C_2H_5$ | |
| 3.059 | H | 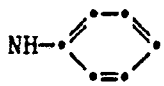 | 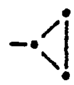 | Smp. 188 - 191°C |
| 3.060 | H | 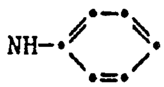 | $C_3H_7$ | Smp. 182 - 183°C |
| 3.061 | H | 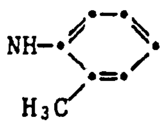 | $C_3H_7$ | Smp. > 150°C Z. |
| 3.062 | H | 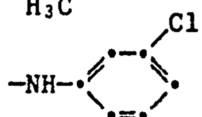 | $C_3H_7$ | Smp. > 200°C Z. |
| 3.063 | H | 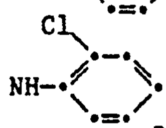 | $C_3H_7$ | |
| 3.064 | H | 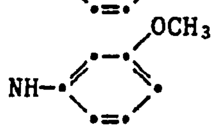 | $C_2H_5$ | |
| 3.065 | H | 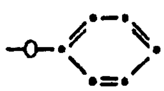 | $C_2H_5$ | |
| 3.066 | H | 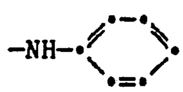 | $CH_3$ | Smp. 205°C Z. |
| 3.067 | H | 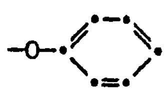 | 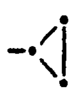 | |
| 3.068 | H | 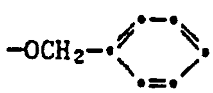 | $C_2H_5$ | |
| 3.069 | H | 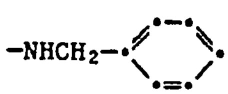 | $C_2H_5$ | |

<u>Tabelle 3</u> (Fortsetzung)

| Nr. | R | A | $R_6$ | Physikal. Daten |
|---|---|---|---|---|
| 3.070 | H | 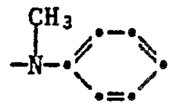 | $C_2H_5$ | |
| 3.071 | $C_2H_5$ | $NHCH_3$ | $C_2H_5$ | |
| 3.072 | $C_2H_5$ | $NHCH_3$ | $C_3H_7$ | |
| 3.073 | $C_2H_5$ | $NHCH_3$ | 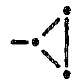 | |
| 3.074 | $C_2H_5$ | $N(CH_3)_2$ | $C_2H_5$ | |
| 3.075 | $C_3H_7$ (i) | $NHCH_3$ | $C_2H_5$ | |
| 3.076 | $C_3H_7$ (i) | $NHCH_3$ | $C_3H_7$ | |
| 3.077 | H | $C_3H_7$ (i) | $C_3H_7$ (n) | Smp. 169 - 170°C |
| 3.078 | H | $C_3H_7$ (i) | $C_2H_5$ | Smp. 210 - 212°C |
| 3.079 | H | $C_3H_7$ (i) | 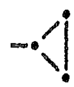 | Smp. 202 - 204°C |
| 3.080 | H | $CH_2OCH_3$ | $C_3H_7$ (n) | |
| 3.081 | H | $CF_3$ | 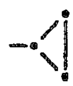 | |
| 3.082 | H | 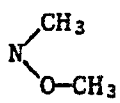 | $C_2H_5$ | |

Tabelle 4:

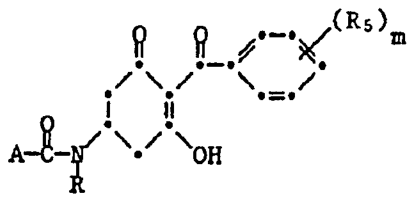

| Nr. | R | A | $(R_5)_m$ | Physikal. Daten |
|---|---|---|---|---|
| 4.001 | H | $NHCH_3$ | 2-Cl | |
| 4.002 | H | $NHCH_3$ | 2,4-$Cl_2$ | |
| 4.003 | H | $NHCH_3$ | 2-$NO_2$ | |
| 4.004 | H | $NHC_2H_5$ | 2-$CF_3$ | |
| 4.005 | H | $NHC_2H_5$ | 2,4-$Cl_2$ | |
| 4.006 | H | $NHC_3H_7$ | 2-Cl | |
| 4.007 | H | $NHC_3H_7$ (i) | H | |
| 4.008 | H | $NHC_3H_7$ (i) | 2-Cl | |
| 4.009 | H | $NHC_3H_7$ (i) | 2,4-$Cl_2$ | Smp. 113 - 116°C |
| 4.010 | H | $NHC_3H_7$ (i) | 2-$NO_2$,4-Cl | Smp. > 160°C (Zers.) |
| 4.011 | H | $NHC_3H_7$ (i) | 2-$CH_3$ | |
| 4.012 | H | $NHC_3H_7$ (i) | 2-$NO_2$ | |
| 4.013 | H | $NHC_3H_7$ (i) | 2-$OCH_3$ | |
| 4.014 | H | $NHC_4H_9$ (n) | 3-$OCH_3$ | |
| 4.015 | H | $NHC_4H_9$ (n) | 4-$CH_3$ | |
| 4.016 | H | $NHC_4H_9$ (n) | 2,4-$Cl_2$ | |
| 4.017 | H | $NHC_4H_9$ (t) | 2,4-$Cl_2$ | Smp. 154 - 156°C |
| 4.018 | H | $NHC_4H_9$ (t) | 2-$NO_2$ | Smp. 170°C Z. |
| 4.019 | H | $NHC_4H_9$ (t) | H | Smp. 148 - 150°C |
| 4.020 | H | $NHC_4H_9$ (t) | 3-$NO_2$ | |
| 4.021 | H | $NHC_4H_9$ (t) | 2-$NO_2$,4-Cl | Smp. > 172°C (Zers.) |
| 4.022 | H | $OCH_3$ | 2,4 $Cl_2$ | |
| 4.023 | H | $OCH_3$ | H | |
| 4.024 | H | $OC_2H_5$ | 2-$NO_2$,4-Cl | |
| 4.025 | H | $OC_2H_5$ | 2,4-$Cl_2$ | Smp. 100 - 103°C |
| 4.026 | H | $OC_3H_7$ (i) | 2,4-$Cl_2$ | |
| 4.027 | H | $OC_3H_7$ (i) | 2-$NO_2$ | |
| 4.028 | H | $OC_3H_7$ (i) | H | |
| 4.029 | H | $OC_3H_7$ | 2,4-$Cl_2$ | |
| 4.030 | H | $OC_3H_7$ | 2-$NO_2$ | |
| 4.031 | H | $CH_3$ | 2,4-$Cl_2$ | Smp. 152 - 153°C |
| 4.032 | H | $CH_3$ | 2-$NO_2$ | |
| 4.033 | H | $CH_3$ | H | |
| 4.034 | H | $CH_2CH_3$ | 2-$NO_2$,4-Cl | |
| 4.035 | H | $CH_2CH_3$ | 4-$OCH_3$ | |
| 4.036 | H | $CH_2CH_3$ | 2-F, 6-Cl | |
| 4.037 | H | $C_4H_9$ (n) | 2-Cl | |

Tabelle 4 (Fortsetzung)

| Nr. | R | A | $(R_5)_m$ | Physikal. Daten |
|---|---|---|---|---|
| 4.038 | H | $N(CH_3)_2$ | 2,4-$Cl_2$ | Smp. 90 - 95°C |
| 4.039 | H | $N(CH_3)_2$ | 2-$NO_2$ | |
| 4.040 | H | $N(CH_3)_2$ | H | |
| 4.041 | H | $N(C_2H_5)_2$ | 2-$NO_2$,4Cl | |
| 4.042 | H | $N(C_2H_5)_2$ | 2Cl | |
| 4.043 | H | $N(C_2H_5)_2$ | 4-Br | |
| 4.044 | H | 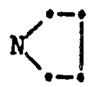 | 2,4 $Cl_2$ | Smp. 93 - 96°C |
| 4.045 | H | 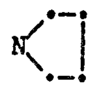 | 2-$NO_2$ | |
| 4.046 | H | 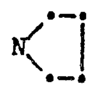 | 3-$CF_3$ | |
| 4.047 | H | -NH- 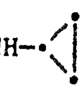 | 2-$NO_2$ | |
| 4.048 | H | O- 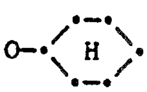 | H | |
| 4.049 | H | 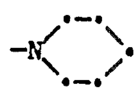 | 2-Cl | |
| 4.050 | H | 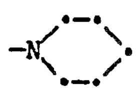 | 2-$NO_2$ | |
| 4.051 | $CH_3$ | $NHCH_3$ | 2-$NO_2$ | |
| 4.052 | $CH_3$ | $NHCH_3$ | 2,4-$Cl_2$ | |
| 4.053 | $CH_3$ | $NHC_3H_7$ (i) | 2-$NO_2$ | |
| 4.054 | $CH_3$ | $NHC_3H_7$ (i) | H | |
| 4.055 | $CH_3$ | $NHC_3H_7$ (i) | 2-$NO_2$,4-Cl | |
| 4.056 | $CH_3$ | $NHC_3H_7$ (i) | 2,4 $Cl_2$ | |
| 4.057 | $CH_3$ | $NHC_3H_7$ (i) | 3-$OCH_3$ | |
| 4.058 | $CH_3$ | $-OCH_3$ | 2-$NO_2$ | |
| 4.059 | $CH_3$ | $-OCH_3$ | 2-Cl | |
| 4.060 | $CH_3$ | $-OC_3H_7$ (i) | 2,4-$Cl_2$ | |
| 4.061 | $CH_3$ | $CH_3$ | 2-$NO_2$ | |
| 4.062 | $CH_3$ | $CH_3$ | H | |
| 4.063 | $CH_3$ | $CH_3$ | 2-$CF_3$ | |
| 4.064 | $CH_3$ | $C_2H_5$ | 2,4-$Cl_2$ | |
| 4.065 | H | -NH- 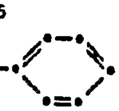 | 2-$NO_2$ | |
| 4.066 | H | -NH- 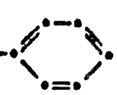 | 2,4-$Cl_2$ | Smp. 174 - 175°C |

Tabelle 4 (Fortsetzung)

| Nr. | R | A | $(R_5)_m$ | Physikal. Daten |
|---|---|---|---|---|
| 4.067 | H | 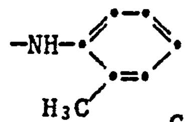 | 2-Cl | |
| 4.068 | H | 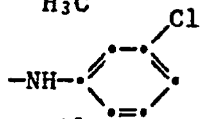 | H | |
| 4.069 | H | 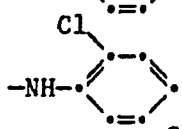 | $4-OCH_3$ | |
| 4.070 | H | 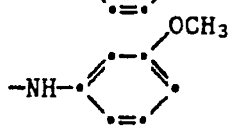 | $2-NO_2,4-Cl$ | |
| 4.071 | H | 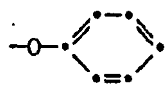 | H | |
| 4.072 | H | 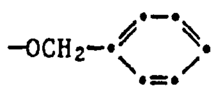 | $2,4-Cl_2$ | |
| 4.073 | H | 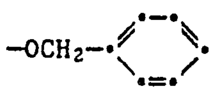 | H | |
| 4.074 | H | 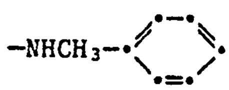 | $2,4-Cl_2$ | |
| 4.075 | $CH_3$ | 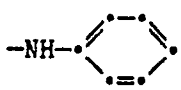 | $2-NO_2$ | |
| 4.076 | $C_2H_5$ | $-NHCH_3$ | $2,4-Cl_2$ | |
| 4.077 | $C_2H_5$ | $-NHCH_3$ | H | |
| 4.078 | $C_2H_5$ | $-NHCH_3$ | $2-NO_2$ | |
| 4.079 | $C_2H_5$ | $N(CH_3)_2$ | $2,4-Cl_2$ | |
| 4.080 | $C_2H_5$ | $N(CH_3)_2$ | $2-NO_2$ | |
| 4.081 | $C_2H_5$ | $N(CH_3)_2$ | H | |
| 4.082 | $C_3H_7$ (i) | $NHCH_3$ | $2,4-Cl_2$ | Smp. 127 - 130°C |
| 4.083 | $C_3H_7$ (i) | $NHCH_3$ | $2-NO_2$ | |
| 4.084 | $C_3H_7$ (i) | $NHCH_3$ | $2-NO_2,4-Cl$ | |
| 4.085 | $C_3H_7$ (i) | $NHCH_3$ | H | |
| 4.086 | H | $N(CH_3)_2$ | 2-Cl, $4-SCH_3$ | |
| 4.087 | H | $N(CH_3)_2$ | 2-Cl, $4-SO-CH_3$ | |
| 4.088 | H | $N(CH_3)_2$ | 2-Cl, $4-SO_2-CH_3$ | |
| 4.089 | H | $N(CH_3)_2$ | $2-SCH_3$, 4-Cl | |

Tabelle 4 (Fortsetzung)

| Nr. | R | A | $(R_5)_m$ | Physikal. Daten |
|---|---|---|---|---|
| 4.090 | H | $N(CH_3)_2$ | $2-SO-CH_3$, $4-Cl$ | |
| 4.091 | H | $N(CH_3)_2$ | $2-SO_2-CH_3$, $4-Cl$ | |
| 4.092 | $CH_3$ | $N(CH_3)_2$ | $4-SO_2-CH_3$, $2-Cl$ | |
| 4.093 | $CH_3$ | $N(CH_3)_2$ | $2-SO_2-CH_3$, $4-Cl$ | |
| 4.094 | H | $CH_3$ | $2-SO_2-CH_3$, $4-Cl$ | |
| 4.095 | H | $CH_3$ | $4-SO_2-CH_3$, $2-Cl$ | |
| 4.096 | H | 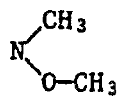 | $2,4-Cl_2$ | |
| 4.097 | H | 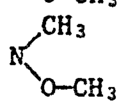 | $2-Cl$, $4-SO_2-CH_3$ | |
| 4.098 | H | $CH_2-O-CH_3$ | $2-SO_2-CH_3$, $4-Cl$ | |
| 4.099 | H | $CF_3$ | $2,4-Cl_2$ | |
| 4.100 | $CH_3$ | $CF_3$ | $2-Cl$, $4-SO_2-CH_3$ | |
| 4.101 | H | $C_3H_7$ (i) | $2,4-Cl_2$ | Smp. 133 - 134°C |
| 4.102 | H | $NH(C_3H_7)$ (i) | $2,5-Cl_2$ | Smp. 162 - 164°C |
| 4.103 | H | $CH_3$ | $2,5-Cl_2$ | Smp. 136 - 138°C |

H.5 Herstellung der Oximether der Formel I

H.5.1. 5-(N',N'-Dimethylureido)-2-(1-ethoxyiminobutyl)-cyclohexan-1,3-dion

3,05 g (12 mMol) 5-(N,N-Dimethylureido)-2-propionyl-cyclohexan-1,3-dion, 1,3 g (13 mMol) O-Ethylhydroxylaminhydrochlorid und 2 ml (13 mMol) 1,8-Diazabicyclo(5, 4, 0)undec-7-en in 24 ml Ethanol werden 15 Stunden bei Raumtemperatur gerührt. Das Reaktionsgemisch wird danach mit 200 ml Essigsäureethylester verdünnt und unter Kühlen auf 0-5°C mit 1N-Salzsäure auf pH 1-2 gestellt, mehrmals mit Wasser gewaschen und nach dem Trocknen über $MgSO_4$ zur Trockne eingedampft. Das so erhaltene Rohprodukt wird an Kieselgel mit Essigsäureethylester/Ethanol (10 : 1) gereinigt.

Man isoliert 2,4 g (67,3%) der Titelverbindung der Formel

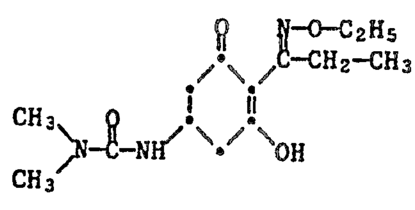

als wachsartige Kristalle vom Smp. 94-97°C (Verb. No. 5.028).

Analog sind die Verbindungen der Tabelle 5 erhältlich

Tabelle 5

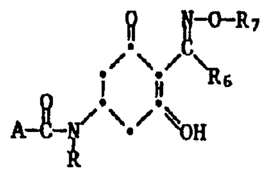

| Nr. | R | A | $R_6$ | $R_7$ | Physikal. Daten |
|---|---|---|---|---|---|
| 5.001 | H | $NHCH_3$ | $C_3H_7$ | $C_2H_5$ | |
| 5.002 | H | $NHC_2H_5$ | $C_2H_5$ | $C_2H_5$ | |
| 5.003 | H | $NHC_2H_5$ | $C_3H_7$ | $CH_2CH{=}CHCl$ | |
| 5.004 | H | $NHC_3H_7$ (i) | $C_2H_5$ | $C_2H_5$ | Smp. 153 - 154°C |
| 5.005 | H | $NHC_3H_7$ (i) | $C_2H_5$ | $CH_2CH{=}CHCl$ | |
| 5.006 | H | $NHC_3H_7$ (i) | $C_3H_7$ | $C_2H_5$ | |
| 5.007 | H | $NHC_3H_7$ (i) | $C_4H_9$ | $CH_3$ | |
| 5.008 | H | $NHC_3H_7$ (n) | $C_3H_7$ (i) | $C_2H_5$ | |
| 5.009 | H | $NHC_4H_9$ (n) | $CH_3$ | $C_4H_9$ (n) | |
| 5.010 | H | $NHC_4H_9$ (n) | $C_2H_5$ | $C_2H_5$ | |
| 5.011 | H | $NHC_4H_9$ (t) | $C_2H_5$ | $C_2H_5$ | Smp. 121 - 124°C |
| 5.012 | H | $NHC_4H_9$ (t) | $C_2H_5$ | $CH_2CH{=}CHCl$ | |
| 5.013 | H | $NHC_4H_9$ (t) | $C_3H_7$ | $C_2H_5$ | Smp. 123 - 126°C |
| 5.014 | H | $NHC_4H_9$ (t) | $C_3H_7$ | $CH_2CH{=}CH_2$ | |
| 5.015 | H | $OCH_3$ | $C_2H_5$ | $C_2H_5$ | |
| 5.016 | H | $OCH_3$ | $C_4H_9$ | $C_2H_5$ | |
| 5.017 | H | $OC_2H_5$ | $C_2H_5$ | $CH_2CH{=}CHCl$ | |

**Tabelle 5 (Fortsetzung)**

| Nr. | R | A | $R_6$ | $R_7$ | Physikal. Daten |
|---|---|---|---|---|---|
| 5.018 | H | $OC_2H_5$ | $C_3H_7$ | $C_2H_5$ | Wachs |
| 5.019 | H | $OC_2H_5$ | $C_3H_7$ | $CH_2C{\equiv}CH$ | |
| 5.020 | H | $OC_3H_7$ | $CH_3$ | $C_4H_9$ (n) | |
| 5.021 | H | $OC_3H_7$ (i) | $C_2H_5$ | $C_2H_5$ | |
| 5.022 | H | $CH_3$ | $C_2H_5$ | $C_2H_5$ | Wachs |
| 5.023 | H | $CH_3$ | $C_2H_5$ | $CH_2CH{=}CHCl$ | |
| 5.024 | H | $CH_3$ | $C_3H_7$ | $C_2H_5$ | Smp. 111 - 113°C |
| 5.025 | H | $C_2H_5$ | $CH_3$ | $C_2H_5$ | |
| 5.026 | H | $C_4H_9$ | $CH_3$ | $CH_2CH{=}CH_2$ | |
| 5.027 | H | $C_4H_9$ | $C_2H_5$ | $C_2H_5$ | |
| 5.028 | H | $N(CH_3)_2$ | $C_2H_5$ | $C_2H_5$ | Smp. 94 - 97°C |
| 5.029 | H | $N(CH_3)_2$ | $C_3H_7$ | $CH_2CH{=}CHCl$ | cis/trans Harz |
| 5.030 | H | $N(CH_3)_2$ | $C_3H_7$ | $CH_2CH{=}CHCl$ | trans |
| 5.031 | H | $N(CH_3)_2$ | $C_3H_7$ | $C_2H_5$ | Smp. 105 - 107°C |
| 5.032 | H | $N(C_2H_5)_2$ | $C_2H_5$ | $C_2H_5$ | |
| 5.033 | H | $N(C_2H_5)_2$ | $C_3H_7$ | $C_2H_5$ | |
| 5.034 | H | 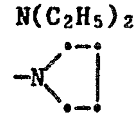 | $C_2H_5$ | $CH_2CH{=}CHCl$ | |
| 5.035 | H | 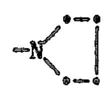 | $C_3H_7$ | $C_2H_5$ | |
| 5.036 | H | 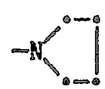 | $C_2H_5$ | $C_2H_5$ | |
| 5.037 | H | 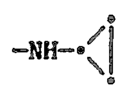 | $C_3H_7$ (i) | $C_2H_5$ | |

Tabelle 5 (Fortsetzung)

| Nr. | R | A | $R_6$ | $R_7$ | Physikal. Daten |
|---|---|---|---|---|---|
| 5.038 | H | 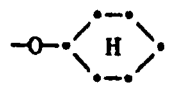 | $C_2H_5$ | $C_2H_5$ | |
| 5.039 | H | 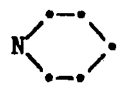 | $C_2H_5$ | $C_2H_5$ | |
| 5.040 | $CH_3$ | $NHCH_3$ | $C_2H_5$ | $CH_2CH=CHCl$ | |
| 5.041 | $CH_3$ | $N(CH_3)_2$ | $C_2H_5$ | $C_2H_5$ | |
| 5.042 | $CH_3$ | $N(CH_3)_2$ | $C_3H_7$ | $C_2H_5$ | |
| 5.043 | $CH_3$ | $NHC_3H_7$ (i) | $C_2H_5$ | $C_2H_5$ | |
| 5.044 | $CH_3$ | $NHC_3H_7$ (i) | $C_2H_5$ | $CH_2CH=CHCl$ | |
| 5.045 | $CH_3$ | $NHC_3H_7$ (i) | $CH_3$ | $C_4H_9$ (n) | |
| 5.046 | $CH_3$ | $OCH_3$ | $C_2H_5$ | $C_2H_5$ | |
| 5.047 | $CH_3$ | $OCH_3$ | $C_3H_7$ | $C_2H_5$ | |
| 5.048 | $CH_3$ | $OC_3H_7$ (i) | $C_2H_5$ | $C_2H_5$ | |
| 5.049 | $CH_3$ | $CH_3$ | $C_2H_5$ | $C_2H_5$ | Oel |
| 5.050 | $CH_3$ | $CH_3$ | $C_2H_5$ | $CH_2CH=CHCl$ | |
| 5.051 | $CH_3$ | $CH_3$ | $C_3H_7$ | $C_2H_5$ | |
| 5.052 | $CH_3$ | $C_2H_5$ | $C_2H_5$ | $C_2H_5$ | |
| 5.053 | $CH_3$ | $C_2H_5$ | $C_3H_7$ | $C_2H_5$ | |
| 5.054 | H | 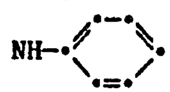 | $C_2H_5$ | $C_2H_5$ | |
| 5.055 | H | 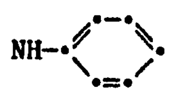 | $C_3H_7$ | $C_2H_5$ | Smp. 133 - 135°C |

Tabelle 5 (Fortsetzung)

| Nr. | R | A | $R_6$ | $R_7$ | Physikal. Daten |
|---|---|---|---|---|---|
| 5.056 | H | 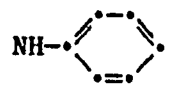 | $C_3H_7$ | $CH_2CH{=}CHCl$ | cis/trans Smp. 101 - 103°C |
| 5.057 | H | 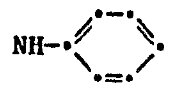 | $C_3H_7$ | $CH_2CH{=}CHCl$ | trans |
| 5.058 | H | 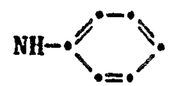 | $C_3H_7$ | $CH_3$ | Smp. 159° C. |
| 5.059 | H | 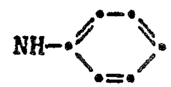 | $CH_3$ | $C_2H_5$ | Smp. 156° C. |
| 5.060 | H | 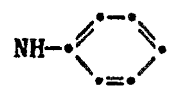 | $CH_3$ | $CH_2CH{=}CHCl$ | cis/trans Smp. 135° C. |
| 5.061 | H | 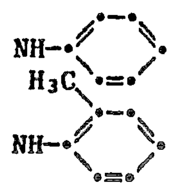 | $CH_3$ | $CH_2CH{=}CHCl$ | trans |
| 5.062 | H | | $C_3H_7$ | $C_2H_5$ | Smp. 133 - 135°C |
| 5.063 | H | 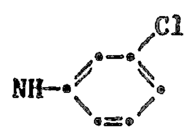 | $C_3H_7$ | $C_2H_5$ | Smp. 117 - 120°C |

Tabelle 5 (Fortsetzung)

| Nr. | R | A | $R_6$ | $R_7$ | Physikal. Daten |
|---|---|---|---|---|---|
| 5.064 | H | 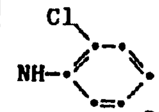 | $C_3H_7$ | $C_2H_5$ | |
| 5.065 | H | 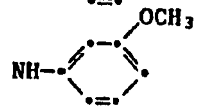 | $C_2H_5$ | $C_2H_5$ | |
| 5.066 | H | 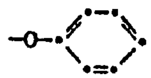 | $C_2H_5$ | $C_2H_5$ | |
| 5.067 | H | 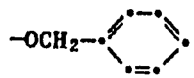 | $C_2H_5$ | $C_2H_5$ | |
| 5.068 | H | 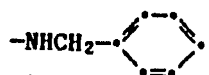 | $C_2H_5$ | $C_2H_5$ | |
| 5.069 | H | 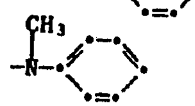 | $C_2H_5$ | $C_2H_5$ | |
| 5.070 | $C_2H_5$ | $NHCH_3$ | $C_2H_5$ | $C_2H_5$ | |
| 5.071 | $C_2H_5$ | $NHCH_3$ | $C_3H_7$ | $C_2H_5$ | |
| 5.072 | $C_2H_5$ | $N(CH_3)_2$ | $C_2H_5$ | $CH_2CH{=}CHCl$ | |
| 5.073 | $C_2H_5$ | $N(CH_3)_2$ | $C_2H_5$ | $C_2H_5$ | |
| 5.074 | $C_3H_7$ (i) | $NHCH_3$ | $C_2H_5$ | $C_2H_5$ | |
| 5.075 | $C_3H_7$ (i) | $NHCH_3$ | $C_2H_5$ | $CH_2CH{=}CHCl$ | |
| 5.076 | $C_3H_7$ (i) | $NHCH_3$ | $C_3H_7$ | $C_2H_5$ | |
| 5.077 | H | $C_3H_7$ (i) | $C_3H_7$ | $C_2H_5$ | Smp. 80 - 90°C (Wachs) |
| 5.078 | H | $C_3H_7$ (i) | $C_2H_5$ | $C_2H_5$ | Smp. 105 - 107°C |

Beispiel F.6 :

Formulierungsbeispiele

Beispiel F.6.1 : Formulierungsbeispiel für Wirkstoffe der Formel I

(% = Gewichtsprozent)

| a) Emulsionskonzentrate | a) | b) | c) |
|---|---|---|---|
| Wirkstoff gemäss Herstellungsbeispiel 4 oder 5 | 20 % | 40 % | 50 % |
| Ca-Dodecylbenzolsulfonat | 5 % | 8 % | 5,8 % |
| Ricinusöl-polyethylenglykolether (36 Mol EO) | 5 % | - | - |
| Tributylphenoyl-polyethylenglykolether (30 mol EO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| b) Lösungen | a) | b) | c) |
|---|---|---|---|
| Wirkstoff gemäss Herstellungsbeispiel 4 oder 5 | 80 % | 10 % | 5 % |
| Ethylenglykol-monomethylether | 20 % | - | - |
| Polyethylenglykol MG 400 | - | 70 % | - |
| N-Methyl-2-pyrrolidon | - | 20 % | 5 % |
| Epoxidiertes Kokosnussöl | - | - | 90 % |

Die Lösungen sind zur Anwendung in Form kleinster Tropfen geeignet.

| c) Granulate | a) | b) |
|---|---|---|
| Wirkstoff gemäss Herstellungsbeispiel 4 oder 5 | 5 % | 10 % |
| Kaolin | 94 % | - |
| Hochdisperse Kieselsäure | 1 % | |
| Attapulgit | - | 90 % |

Der Wirkstoff wird gelöst, auf den Träger aufgesprüht und das Lösungsmittel anschliessend im Vakuum abgedampft.

| d) Stäubemittel | a) | b) | c) |
|---|---|---|---|
| Wirkstoff gemäss Herstellungsbeispiel 4 oder 5 | 2 % | 5 % | 8 % |
| Hochdisperse Kieselsäure | 1 % | 5 % | 5 % |
| Talkum | 97 % | - | 10 % |
| Kaolin | - | 90 % | 77 % |

Durch inniges Vermischen der Trägerstoffe mit dem Wirkstoff erhält man gebrauchsfertiges Stäubemittel.

| e) Spritzpulver | a) | b) |
|---|---|---|
| Wirkstoff gemäss Herstellungsbeispiel 4 oder 5 | 20 % | 60 % |
| Na-Ligninsulfonat | 5 % | 5 % |
| Na-Laurylsulfat | 3 % | 6 % |
| Octylphenolpolyethylenglykolether (7-8 Mol EO) | - | 2 % |
| Hochdisperse Kieselsäure | 5 % | 27 % |
| Kaolin | 67 % | - |

Der Wirkstoff wird mit den Zusatzstofen gut vermischt und in einer geeigneten Mühle gut vermahlen. Man erhält Spritzpulver, die sich mit Wasser zur Suspension jeder gewünschten Konzentration verdünnen lassen.

| f) Extruder Granulat | |
|---|---|
| Wirkstoff gemäss Herstellungsbeispiel 4 oder 5 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| g) Umhüllungs-Granulat | |
|---|---|
| Wirkstoff gemäss Herstellungsbeispiel 4 oder 5 | 3 % |
| Polyethylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

h) Suspensions-Konzentrat

| | | |
|---|---|---|
| Wirkstoff gemäss Herstellungsbeispiel 4 oder 5 | 40 | % |
| Ethylenglykol | 10 | % |
| Nonylphenolpolyethylenglykolether (15 Mol EO) | 6 | % |
| Na-Ligninsulfonat | 10 | % |
| Carboxymethylcellulose | 1 | % |
| 37%ige wässrige Formaldehyd-Lösung | 0,2 | % |
| Silikonöl in Form einer 75%igen wässrigen Emulsion | 0,8 | % |
| Wasser | 32 | % |

Der fein gemahlene Wirktoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel B.7.

Biologische Beispiele

Beispiel 7.1 : Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25%igen Emulsions-Konzentrat behandelt. Es werden Konzentrationen von 4 kg Wirksubstanz/Hektar angewendet. Die Saatschalen werden im Gewächshaus bei 22-25°C und 50-70% relativer Luftfeuchtigkeit gehalten und der Versuch nach 3 Wochen ausgewertet.

Die herbizide Wirkung wird anhand eines linearen neunstufigen Bonitierungsschemas bewertet, in welchem 1 für totale Schädigung (100% Herbizidwirkung) und 9 für keine Wirkung (Versuchspflanze wächst wie die unbehandelte Referenz) steht.

Einzelergebnisse sind in Tabelle 6 zusammengestellt.

Tabelle 6: pre emergente Herbizidwirkung bei 4 kg/ha-Aufwandmenge.

| Verb. No. | Avena | Sinapis | Setaria | Stellaria |
|---|---|---|---|---|
| 5.056 | 5 | 9 | 1 | 9 |
| 5.055 | 1 | 9 | 1 | 9 |
| 5.059 | 4 | 9 | 1 | 9 |
| 4.038 | 3 | 1 | 1 | 1 |
| 5.062 | 4 | 9 | 1 | 9 |
| 3.029 | 3 | 8 | 1 | 1 |
| 3.030 | 2 | 9 | 1 | 4 |
| 5.028 | 1 | 9 | 1 | 9 |
| 5.063 | 2 | 9 | 1 | 9 |
| 4.009 | 2 | 4 | 1 | 1 |

Beispiel 7.2 : Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24°-26°C und 45-60% relativer Luftfeuchtigkeit gehalten. 15 Tage nach der Behandlung wird der Versuch ausgewertet.

In diesem Versuch zeigen die Verbindungen nach Herstellungsbeispiel 4 und 5 starke bis sehr starke Herbizidwirkung.

Beispiel 7.3 : Herbizidwirkung für Wasserreis (paddy)

Die Wasserunkräuter Echinochloa crus galli und Monocharia vag. werden in Plastikbechern (60 cm$^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die Gefässe mit einer Aufwandmenge von 0,5 bis 4 kg AS pro Hektar. Die Pflanzenbecher werden dann im Gewächshaus unter optimalen Wachstumsbedingungen für die Reisunkräuter aufgestellt, d.h. bei 25°-30°C und hoher Luftfeuchtigkeit.

Die Auswertung der Versuche findet 3 Wochen nach Applikation statt.

Einzelergebnisse sind in Tabelle 7 zusammengestellt.

Tabelle 7

| Verb. Nr. | Echinochloa | Monocharia |
|---|---|---|
| 3.018 | 1 | 2 |
| 5.058 | 1 | 9 |
| 4.038 | 1 | 1 |
| 3.029 | 2 | 3 |
| 3.030 | 1 | 4 |
| 4.025 | 2 | 1 |
| 5.013 | 1 | 2 |
| 4.009 | 1 | 1 |

Beispiel 7.4 : Wuchshemmung bei tropischen Bodenbedecker-Leguminosen (cover crops)

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70% relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit Wirkstoffen gemäss Herstellungsbeispiel 4 und 5 in Aufwandmengen bis zu 3000 g/ha behandelten Pflanzen eine deutliche Reduktion des Neuzuwachses, ohne dass dabei die Versuchspflanzen geschädigt wurden.

Beispiel 7.5 : Wuchsregulierung an Sojabohnen

In Kunststoffbehältern mit einem Erde-Torf-Sandgemisch im Verhältnis 6 :3 : 1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken erfindungsgemässe Wirkstoffe gemäss Herstellungsbeispiel 4 und 5 eine merkliche Erhöhung der Anzahl und des Gewichts der Schoten im Haupttrieb.

Beispiel 7.6 : Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes gemäss Herstellungsbeispiel 5 oder 5 besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90% der Kontrolle), sowie teilweise eine Zunahme der Stengeldurchmesser auf.

Beispiel 7.7 : Wuchshemmung bei Gräsern

In Kunststoffschalen mit Erde-Torf-Sand-Gemisch (6 : 3 : 1) werden die Gräser Lolium perenne, Poa pratensis, Festuca ovina, Dactylis glomerata und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes gemäss Herstellungsbeispiel 4 oder 5 besprüht. Die Wirkstoffmenge beträgt umgerechnet bis zu 500 g Aktivsubstanz pro Hektar. 21 Tage nach Applikation wird Wachstum der Gräser beurteilt.

Verbindungen gemäss Herstellungsbeispiel 4 und 5 bewirken eine Reduzierung des Neuzuwachses im Vergleich zur unbehandelten Kontrolle.

## Ansprüche

**Patentansprüche für die Vertragsstaaten AT-BE-CH/LI-DE-FR-GB-GR-IT-LU-NL-SE**

1. Verbindungen der Formel I

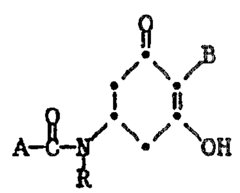

(I)

worin
R für Wasserstoff oder $C_1$-$C_6$-Alkyl,
A für $R_2$, $OR_3$ oder $NR_3R_4$,
$R_2$ für $C_3$-$C_6$-Cycloalkyl oder gegebenenfalls einfach durch $C_1$-$C_4$-Alkoxy oder ein- oder mehrfach durch Halogen substituiertes $C_1$-$C_6$-Alkyl,
$R_3$ für $C_1$-$C_6$-Alkyl, $C_3$-$C_6$-Cycloalkyl oder gegebenenfalls bis zu dreifach durch $R_5$ substituiertes Phenyl oder Benzyl,
$R_4$ für Wasserstoff, $C_1$-$C_6$-Alkyl, $C_1$-$C_4$-Alkoxy oder $C_3$-$C_6$-Cycloalkyl,
$R_3$ und $R_4$ ausserdem gemeinsam mit dem Stickstoffatom, an welches sie gebunden sind für einen Pyrrolidin-, Morpholin- oder Piperidinrest,
B für einen der Reste

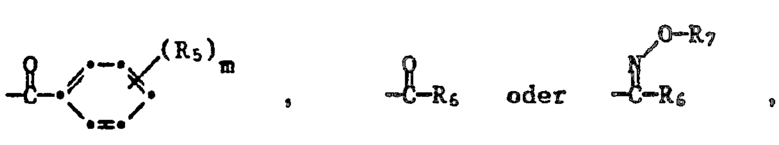

m für 0, 1, 2 oder 3
$R_5$ für Halogen, Nitro, Cyano, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Alkylsulfinyl oder $C_1$-$C_4$-Alkylsulfonyl,

$R_6$ für $C_1$-$C_6$-Alkyl oder $C_3$-$C_6$-Cycloalkyl,
$R_7$ für Wasserstoff, $C_1$-$C_6$-Alkyl, $C_1$-$C_6$-Halogenalkyl, $C_3$-$C_6$-Alkenyl, $C_3$-$C_6$-Halogenalkenyl oder $C_3$-$C_6$-Alkinyl steht
sowie Salze der Verbindungen der Formel I mit Säuren, Basen oder Komplexbildnern.

2. Verbindungen der Formel I gemäss Anspruch 1
worin
R für Wasserstoff oder $C_1$-$C_3$-Alkyl,
A für $R_2$, $OR_3$ oder $NR_3R_4$,
$R_2$ für $C_1$-$C_4$-Alkyl, Methoxymethyl oder Trifluormethyl,
$R_3$ für $C_1$-$C_4$-Alkyl, Cyclopropyl, Cyclohexyl, Benzyl oder gegebenenfalls durch Chlor, Methoxy oder Methyl substituiertes Phenyl
$R_4$ für Wasserstoff, Methyl, Methoxy oder Ethyl,
$R_3$ und $R_4$ ausserdem gemeinsam mit dem Stickstoffatom, an welches sie gebunden sind für einen Pyrrolidin oder Piperidinrest,
B für einen der Reste

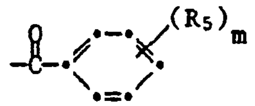 , 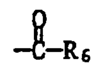 oder 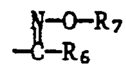 ,

m für 0, 1, 2 oder 3,
$R_5$ für Fluor, Chlor, Brom, Nitro, Methyl, Methoxy, Trifluormethyl, $C_1$-$C_4$-Alkylthio, $C_1$-$C_4$-Alkylsulfinyl oder $C_1$-$C_4$-Alkylsulfonyl,
$R_6$ für $C_1$-$C_6$-Alkyl, Cyclopropyl oder Cyclohexyl,
$R_7$ für Methyl, Ethyl, Allyl, Propargyl oder 3-Chlor-prop-2-en-1-yl steht.

3. 2-Butanoyl-5-ethoxycarbonylaminocyclohexan-1,3-dion,
2-Propanoyl-5-(N′,N′-dimethylureido)-cyclohexan-1,3-dion,
2-Butanoyl-5-(N′,N′-dimethylureido)-cyclohexan-1,3-dion,
5-Acetamido-2-propanoyl-cyclohexan-1,3-dion,
2-(2,4-Dichlorbenzoyl)-5-ethoxycarbonylamino-cyclohexan-1,3-dion,
2-(2,4-Dichlorbenzoyl)-5-(N′-isopropylureido)-cyclohexan-1,3-dion,
2-(2,4-Dichlorbenzoyl)-5-(N′,N′-dimethylureido)-cyclohexan-1,3-dion,
5-(N′-tert-Butylureido)-2-(1-allyloxyiminobutyl)-cyclohexan-1,3-dion,
5-(N′,N′-Dimethylureido)-2-(1-ethoxyiminopropyl)-cyclohexan-1,3-dion,
5-(N′-Phenylureido)-2-(1-ethoxyiminobutyl)-cyclohexan-1,3-dion,
5-(N′-Phenylureido)-2-[1-(3-chlorallyl)oxyiminobutyl]-cyclohexan-1,3-dion,
5-(N′-Phenylureido)-2-(1-ethoxyiminoethyl)-cyclohexna-1,3-dion,
5-(N′-Phenylureido)-2-(1-methoxyiminobutyl)-cyclohexan-1,3-dion,
5-[N′-(2-Methylphenyl)-ureido]-2-(1-ethoxyiminobutyl)-cyclohexan-1,3-dion oder
5-[N′-(3-Chlorphenyl)-ureido]-2-(1-ethoxyiminobutyl)cyclohexan-1,3-dion als Verbindung der Formel I gemäss Anspruch 1 oder 2.

4. Verfahren zur Herstellung von Verbindungen der Formel I gemäss einem der Ansprüche 1 bis 3, dadurch gekennzeichnet, dass man

a) eine Cyclohexandion der Formel II mit einer Verbindung der

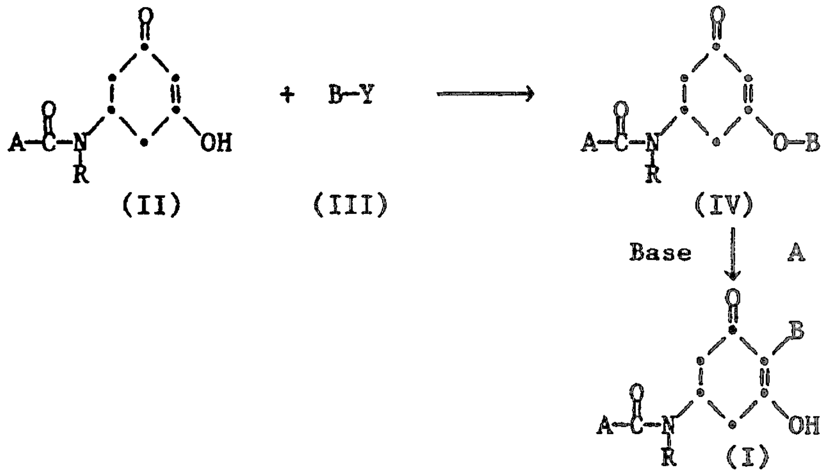

(II) (III) (IV) (I)

Formel III zu einer Verbindung der Formel IV umsetzt und diese anschliessend in Gegenwart einer Base thermisch zu einer Verbindung der Formel I umlagert, wobei A, B und R wie zuvor definiert sind, aber mit der Einschränkung, dass B nicht für die Oximethergruppe

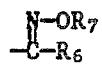

steht, und in der Y für eiine unter den Reaktionsbedingungen abspaltbare nucleofuge Gruppe, wie Chlor, Brom oder $C_1$-$C_4$-Alkoxy steht,
oder
b) ein Cyclohexandion der Formel I mit einer Verbindung

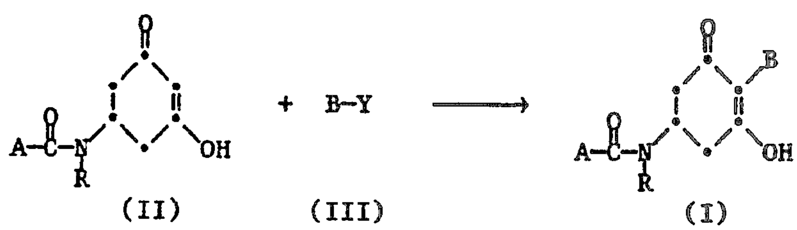
(II) (III) (I)

der Formel III, in der Y für eine Cyangruppe steht, in Gegenwart einer Base und $ZnCl_2$ zu einer Verbindung der Formel I umsetzt, wobei die Reste A, R und B in den Formeln I, II und III wie zuvor definiert sind, mit der Massgabe, dass B nicht für die Oximethergruppe

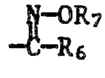

steht,
oder
c) ein Cyclohexylketon der Formel Ia mit einem

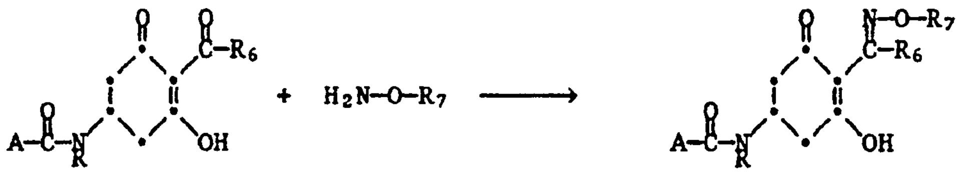

(Ia) (IV) (Ib)

Hydroxylamin der Formel IV zu einem Oxim der Formel Ib umsetzt, wobei die Reste A, R, $R_6$ und $R_7$ wie zuvor definiert sind.

5. Verbindungen der Formel II

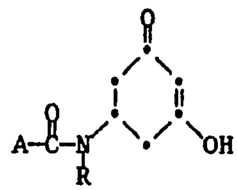

II

worin
R für Wasserstoff oder $C_1$-$C_6$-Alkyl,
A für $R_2$, $OR_3$ oder $NR_3R_4$,
$R_2$ für $C_1$-$C_6$-Alkyl oder $C_3$-$C_6$-Cycloalkyl,
$R_3$ für $C_1$-$C_6$-Alkyl, $C_3$-$C_6$-Cycloalkyl oder gegebenenfalls bis zu dreifach durch $R_5$ substituiertes Phenyl oder Benzyl,
$R_4$ für Wasserstoff, $C_1$-$C_6$-Alkyl oder $C_3$-$C_6$-Cycloalkyl,
$R_3$ und $R_4$ ausserdem gemeinsam mit dem Stickstoffatom, an welches sie gebunden sind für einen Pyrrolidin oder Piperidinrest,
steht.

6. Verfahren zur Herstellung von Verbindungen der Formel II gemäss Anspruch 5, dadurch gekennzeichnet, dass man

a) ein Resorcin der Formel V nach an sich bekannten Verfahren hydriert

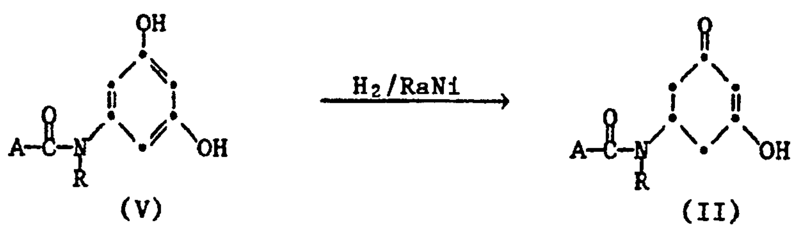


(V) (II)

wobei die Substituenten A und R wie zuvor definiert sind,
oder

b) ein Isocyanat der Formel VI mit einem Alkohol oder einem Amin der Formel VII bzw. VIII

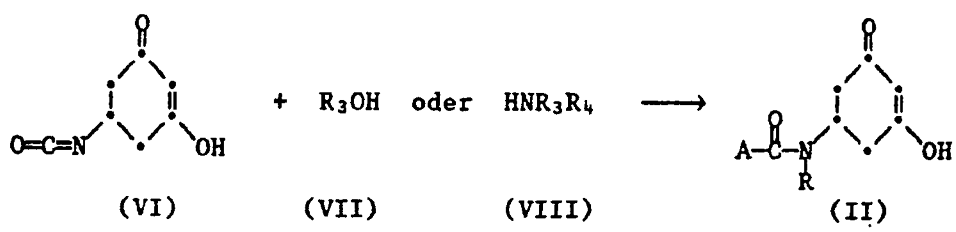

\+ $R_3OH$ oder $HNR_3R_4$

(VI) (VII) (VIII) (II)

zu einem Urethan oder Harnstoff der Formel II, in der R für Wasserstoff und A für $R_3O$ oder $NR_3R_4$ steht und $R_3$ und $R_4$ wie zuvor definiert sind, umsetzt.

7. Verbindungen der Formel IV

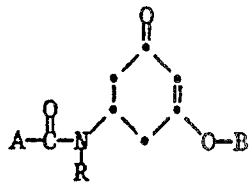

(IV)

IV

worin
R für Wasserstoff oder $C_1$-$C_6$-Alkyl,
A für $R_2$, $OR_3$ oder $NR_3R_4$,
$R_2$ für $C_3$-$C_6$-Cycloalkyl oder gegebenenfalls einfach durch $C_1$-$C_4$-Alkoxy oder ein- oder mehrfach durch Halogen substituiertes $C_1$-$C_6$-Alkyl,
$R_3$ für $C_1$-$C_6$-Alkyl, $C_3$-$C_6$-Cycloalkyl oder gegebenenfalls bis zu dreifach durch $R_5$ substituiertes Phenyl oder Benzyl,
$R_4$ für Wasserstoff, $C_1$-$C_6$-Alkyl, $C_1$-$C_4$-Alkoxy oder $C_3$-$C_6$-Cycloalkyl,
$R_3$ und $R_4$ ausserdem gemeinsam mit dem Stickstoffatom, an welches sie gebunden sind für einen Pyrrolidin-, Morpholin- oder Piperidinrest,
B für einen der Reste

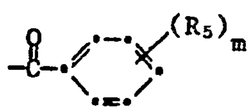 , 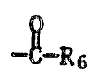 oder 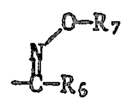 ,

m für 0, 1, 2 oder 3
$R_5$ für Halogen, Nitro, Cyano, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Alkylsulfinyl oder $C_1$-$C_4$-Alkylsulfonyl,
$R_6$ für $C_1$-$C_6$-Alkyl oder $C_3$-$C_6$-Cycloalkyl,
$R_7$ für Wasserstoff, $C_1$-$C_6$-Alkyl, $C_1$-$C_6$-Halogenalkyl, $C_3$-$C_6$-Alkenyl, $C_3$-$C_6$-Halogenalkenyl oder $C_3$-$C_6$-Alkinyl steht.

8. Verfahren zur Herstellung von Verbindungen der Formel IV gemäss Anspruch 7, dadurch gekennzeichnet, dass man
a) eine Cyclohexandion der Formel II mit einer Verbindung der

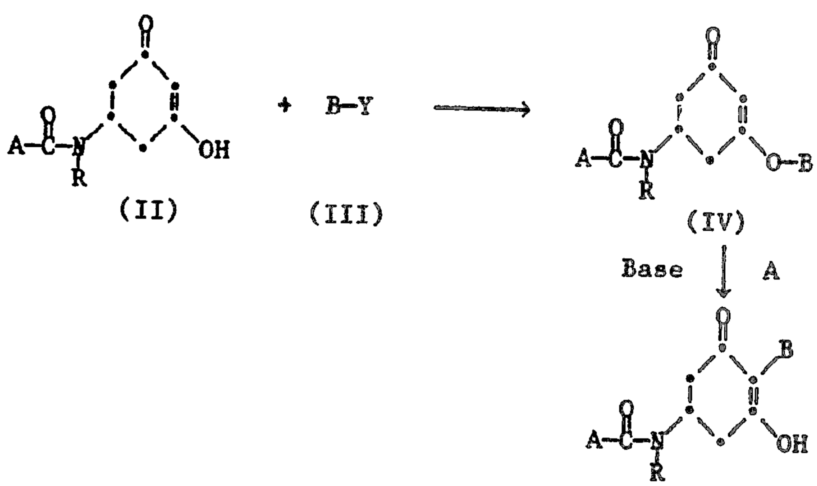


(II) (III) (IV)

Formel III zu einer Verbindung der Formel IV umsetzt, wobei A, B und R wie zuvor definiert sind, aber mit der

Einschränkung, dass B nicht für die Oximestergruppe

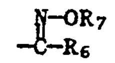

steht, und in der Y für eine unter den Reaktionsbedingungen abspaltbaren nucleofugen Gruppe, wie Chlor, Brom oder $C_1$-$C_4$-Alkoxy steht.

9. Ein herbizides oder pflanzenwuchsregulierendes Mittel, dadurch gekennzeichnet, dass es neben Träger- und/oder anderen Hilfsstoffen als Wirkstoff mindestens eine Verbindung der Formel I gemäss einem der Ansprüche 1 bis 3 enthält.

10. Die Verwendung einer Verbindung der Formel I gemäss einem der Ansprüche 1 bis 3 oder eines Mittels gemäss Anspruch 9 zur Bekämpfung unerwüschten Pflanzenwuchses.

11. Die Verwendung einer Verbindung der Formel I gemäss einem der Ansprüche 1 bis 3 oder eines Mittels gemäss Anspruch 9 zur Wuchsregulierung bei Pflanzen.

12. Ein Verfahren zur Bekämpfung unerwünschten Pflanzenwuchses oder zur Wuchsregulierung bei Pflanzen, dadurch gekennzeichnet, dass man die Pflanzen oder deren Lebensraum mit einer herbizid wirksamen Menge eines Wirkstoffes gemäss einem der Ansprüche 1 bis 3 oder eines Mittels gemäss Anspruch 9 oder mit einer Wuchsregulatorisch wirksamen Menge einer Verbindung gemäss einem der Ansprüche 1 bis 3 oder eines Mittels gemäss Anspruch 9 behandelt.

**Patentansprüche für den folgenden Vertragsstaat : ES**

1. Herbizides oder pflanzenwuchsregulierendes Mittel enthaltend eine Verbindung der Formel I

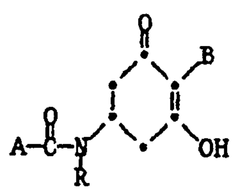

(I)

worin
R für Wasserstoff oder $C_1$-$C_6$-Alkyl,
A für $R_2$, $OR_3$ oder $NR_3R_4$,
$R_2$ für $C_3$-$C_6$-Cycloalkyl oder gegebenenfalls einfach durch $C_1$-$C_4$-Alkoxy oder ein- oder mehrfach durch Halogen substituiertes $C_1$-$C_6$-Alkyl,
$R_3$ für $C_1$-$C_6$-Alkyl, $C_3$-$C_6$-Cycloalkyl oder gegebenenfalls bis zu dreifach durch $R_5$ substitiertes Phenyl oder Benzyl,
$R_4$ für Wasserstoff, $C_1$-$C_6$-Alkyl, $C_1$-$C_4$-Alkoxy oder $C_3$-$C_6$-Cycloalkyl,
$R_3$ und $R_4$ ausserdem gemeinsam mit dem Stickstoffatom, an welches sie gebunden sind für einen Pyrrolidin-, Morpholin- oder Piperidinrest,
B für einen der Reste

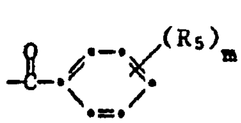 , 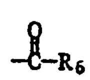 oder 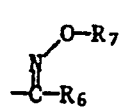 ,

m für 0, 1, 2 oder 3
$R_5$ für Halogen, Nitro, Cyano, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Alkylsulfinyl oder $C_1$-$C_4$-Alkylsulfonyl,
$R_6$ für $C_1$-$C_6$-Alkyl oder $C_3$-$C_6$-Cycloalkyl,
$R_7$ für Wasserstoff, $C_1$-$C_6$-Alkyl, $C_1$-$C_6$-Halogenalkyl, $C_3$-$C_6$-Alkenyl, $C_3$-$C_6$-Halogenalkenyl oder $C_3$-$C_6$-Alkinyl steht

sowie Salze der Verbindungen der Formel I mit Säuren, Basen oder Komplexbildnern.

2. Mittel gemäss Anspruch 1 enthaltend eine Verbindung der Formel I worin

R für Wasserstoff oder $C_1$-$C_3$-Alkyl,

A für $R_2$, $OR_3$ oder $NR_3R_4$,

$R_2$ für $C_1$-$C_4$-Alkyl, Methoxymethyl oder Trifluormethyl,

$R_3$ für $C_1$-$C_4$-Alkyl, Cyclopropyl, Cyclohexyl, Benzyl oder gegebenenfalls durch Chlor, Methoxy oder Methyl substituiertes Phenyl

$R_4$ für Wasserstoff, Methyl, Methoxy oder Ethyl,

$R_3$ und $R_4$ ausserdem gemeinsam mit dem Stickstoffatom, an welches sie gebunden sind für einen Pyrrolidin oder Piperidinrest,

B für einen der Reste

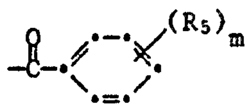 , 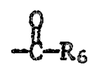 oder 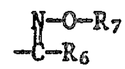 ,

m für 0, 1, 2 oder 3,

$R_5$ für Fluor, Chlor, Brom, Nitro, Methyl, Methoxy, Trifluormethyl, $C_1$-$C_4$-Alkylthio, $C_1$-$C_4$-Alkylsulfinyl oder $C_1$-$C_4$-Alkylsulfonyl,

$R_6$ für $C_1$-$C_6$-Alkyl, Cyclopropyl oder Cyclohexyl,

$R_7$ für Methyl, Ethyl, Allyl, Propargyl oder 3-Chlor-prop-2-en-1-yl steht.

3. Mittel gemäss Anspruch 1 oder 2 enthaltend

2-Butanoyl-5-ethoxycarbonylaminocyclohexan-1,3-dion,
2-Propanoyl-5-(N',N'-dimethylureido)-cyclohexan-1,3-dion,
2-Butanoyl-5-(N',N'-dimethylureido)-cyclohexan-1,3-dion,
5-Acetamido-2-propanoyl-cyclohexan-1,3-dion,
2-(2,4-Dichlorbenzoyl)-5-ethoxycarbonylamino-cyclohexan-1,3-dion,
2-(2,4-Dichlorbenzoyl)-5-(N'-isopropylureido)-cyclohexan-1,3-dion,
2-(2,4-Dichlorbenzoyl-5-(N',N'-dimethylureido)-cyclohexan-1,3-dion,
5-(N'-tert-Butylureido)-2-(1-allyloxyiminobutyl)-cyclohexan-1,3-dion,
5-(N',N'-Dimethylureido)-2-(1-ethoxyiminoproppyl)-cyclohexan-1,3-dion,
5-(N'-Phenylureido)-2-(1-ethoxyiminobutyl)-cyclohexan-1,3-dion,
5-(N'-Phenylureido)-2-[1-(3-chlorallyl)oxyiminobutyl]-cyclohexan-1,3-dion,
5-(N'-Phenylureido)-2-(1-ethoxyiminoethyl)-cyclohexan-1,3-dion,
5-(N'-Phenylureido)-2-(1-methoxyiminobutyl)-cyclohexan-1,3-dion,
5-[N'-(2-Methylphenyl)-ureido]-2-(1-ethoxyiminobutyl)-cyclohexan-1,3-dion oder
5-[N'-(3-Chlorphenyl)-ureido]-2-(1-ethoxyiminobutyl)-cyclohexan-1,3-dion.

4. Verfahren zur Herstellung von Verbindungen der Formel I

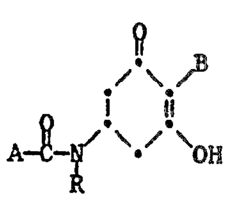

(I)

worin

R für Wasserstoff oder $C_1$-$C_6$-Alkyl,

A für $R_2$, $OR_3$ oder $NR_3R_4$,

$R_2$ für $C_1$-$C_4$-Cycloalkyl oder gegebenenfalls einfach durch $C_1$-$C_4$-Alkoxy oder ein- oder mehrfach durch Halogen substituiertes $C_1$-$C_6$-Alkyl,

$R_3$ für $C_1$-$C_6$-Alkyl, $C_3$-$C_6$-Cycloalkyl oder gegebenenfalls bis zu dreifach durch $R_5$ substituiertes Phenyl oder Benzyl,

$R_4$ für Wasserstoff, $C_1$-$C_6$-Alkyl, $C_1$-$C_4$-Alkoxy oder $C_3$-$C_6$-Cycloalkyl,

$R_3$ und $R_4$ ausserdem gemeinsam mit dem Stickstoffatom, an welches sie gebunden sind für einen Pyrrolidin-,

Morpholin- oder Piperidinrest,
B für einen der Reste

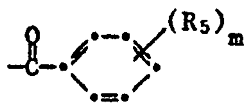 , 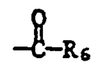 oder 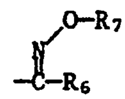 ,

m für 0, 1, 2 oder 3
$R_5$ für Halogen, Nitro, Cyano, $C_1$-$C_4$-Alykl, $C_1$-$C_4$-Alkoxy, $C_1$-$C_4$-Alkylthio, $C_1$-$C_4$-Halogenalkyl, $C_1$-$C_4$-Alkylsulfinyl oder $C_1$-$C_4$-Alkylsulfonyl,
$R_6$ für $C_1$-$C_6$-Alkyl oder $C_3$-$C_6$-Cycloalkyl,
$R_7$ für Wasserstoff, $C_1$-$C_6$-Alkyl, $C_1$-$C_6$-Halogenalkyl, $C_3$-$C_6$-Alkenyl, $C_3$-$C_6$-Halogenalkenyl oder $C_3$-$C_6$-Alkinyl steht
sowie Salze der Verbindungen der Formel I mit Säuren, Basen oder Komplexbildnern, dadurch gekennzeichnet, dass man

a) eine Cyclohexandion der Formel II mit einer Verbindung der

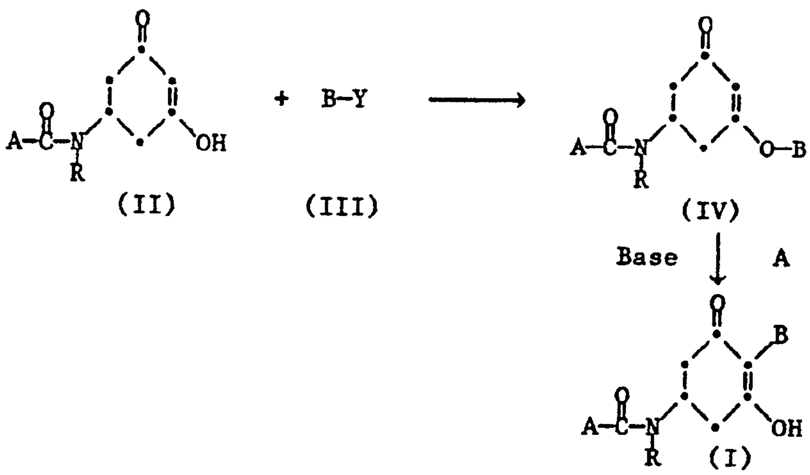

(II) (III) (IV) (I)

Formel III zu einer Verbindung der Formel IV umsetzt und diese anschliessend in Gegenwart einer Base thermisch zu einer Verbindung der Formel I umlagert, wobei A, B und R wie zuvor definiert sind, aber mit der Einschränkung, dass B nicht für die Oximethergruppe

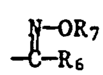

steht, und in der Y für eine unter den Reaktionsbedingungen abspaltbare nucleofuge Gruppe, wie Chlor, Brom oder $C_1$-$C_4$-Alkoxy steht,
oder

b) ein Cyclohexandion der Formel I mit einer Verbindung

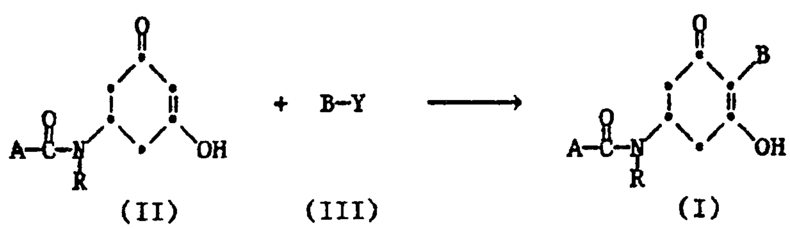
(II) (III) (I)

der Formel III, in der Y für eine Cyangruppe steht, in Gegenwart einer Base und $ZnCl_2$ zu einer Verbindung der Formel I umsetzt, wobei die Reste A, R und B in den Formeln I, II und III wie zuvor definiert sind, mit der Massgabe, dass B nicht für die Oximethergruppe

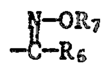

steht,

oder

c) ein Cyclohexylketon der Formel Ia mit einem

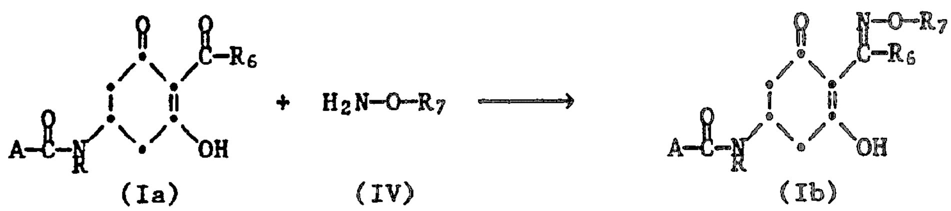

(Ia) (IV) (Ib)

Hydroxylamin der Formel IV zu einem Oxim der Formel Ib umsetzt, wobei die Reste A, R, $R_6$ und $R_7$ wie zuvor definiert sind.

5. Verfahren zur Herstellung von Verbindungen der Formel II

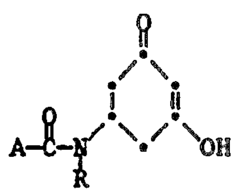

II

worin

R für Wasserstoff oder $C_1$-$C_6$-Alkyl,

A für $R_2$, $OR_3$ oder $NR_3R_4$,

$R_2$ für $C_1$-$C_6$-Alkyl oder $C_3$-$C_6$-Cycloalkyl,

$R_3$ für $C_1$-$C_6$-Alkyl, $C_3$-$C_6$-Cycloalkyl oder gegebenenfalls bis zu dreifach durch $R_5$ substituiertes Phenyl oder Benzyl,

$R_4$ für Wasserstoff, $C_1$-$C_6$-Alkyl oder $C_3$-$C_6$-Cycloalkyl,

$R_3$ und $R_4$ ausserdem gemeinsam mit dem Stickstoffatom, an welches sie gebunden sind für einen Pyrrolidin oder Piperidinrest,

steht, dadurch gekennzeichnet, dass man

a) ein Resorcin der Formel V nach an sich bekannten Verfahren hydriert

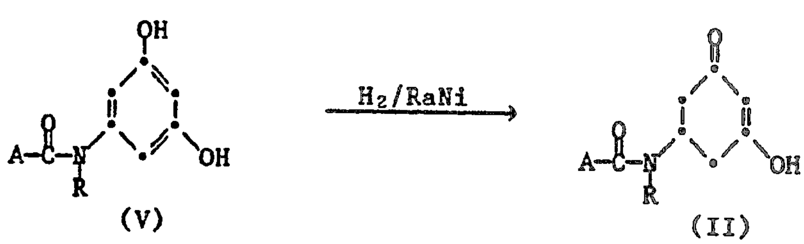


(V) (II)

wobei die Substituenten A und R wie zuvor definiert sind,

oder

b) ein Isocyanat der Formel VI mit einem Alkohol oder einem Amin der Formel VII bzw. VIII

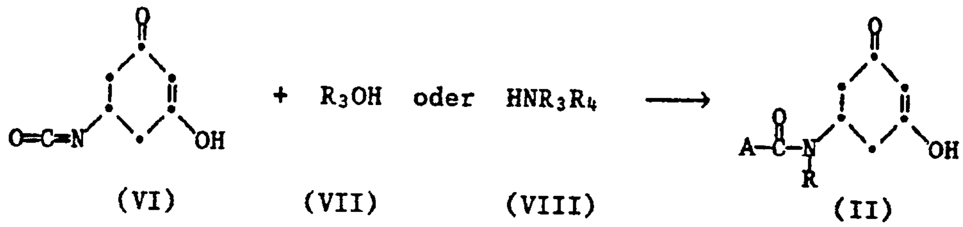


(VI) (VII) (VIII) (II)

zu einem Urethan oder Harnstoff der Formel II, in der R für Wasserstoff und A für $R_3O$ oder $NR_3R_4$ steht und $R_3$ und $R_4$ wie zuvor definiert sind, umsetzt.

6. Verfahren zur Herstellung von Verbindungen der Formel IV

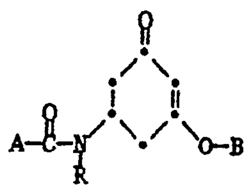

(IV)

IV

worin

R für Wasserstoff oder $C_1$-$C_6$-Alkyl,

A für $R_2$, $OR_3$ oder $NR_3R_4$,

$R_2$ für $C_3$-$C_6$-Cycloalkyl oder gegebenenfalls einfach durch $C_1$-$C_4$-Alkoxy oder ein- oder mehrfach durch Halogen substituiertes $C_1$-$C_6$-Alkyl,

$R_3$ für $C_1$-$C_6$-Alkyl, $C_3$-$C_6$-Cycloalkyl oder gegebenenfalls bis zu dreifach durch $R_5$ substituiertes Phenyl oder Benzyl,

$R_4$ für Wasserstoff, $C_1$-$C_6$-Alkyl, $C_1$-$C_4$-Alkoxy oder $C_3$-$C_6$-Cycloalkyl,

$R_3$ und $R_4$ ausserdem gemeinsam mit dem Stickstoffatom, an welches sie gebunden sind für einen Pyrrolidin-, Morpholin- oder Piperidinrest,

B für einen der Reste

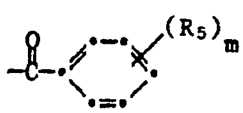 , 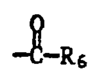 oder 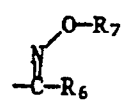 ,

m für 0, 1, 2 oder 3

$R_5$ für Halogen, Nitro, Cyano, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Alkylsulfinyl oder $C_1$-$C_4$-Alkylsulfonyl,

$R_6$ für $C_1$-$C_6$-Alkyl oder $C_3$-$C_6$-Cycloalkyl,

$R_7$ für Wasserstoff, $C_1$-$C_6$-Alkyl, $C_1$-$C_6$-Halogenalkyl, $C_3$-$C_6$-Alkenyl, $C_3$-$C_6$-Halogenalkenyl oder $C_3$-$C_6$-Alkinyl steht, dadurch gekennzeichnet, dass man

a) eine Cyclohexandion der Formel II mit einer Verbindung der

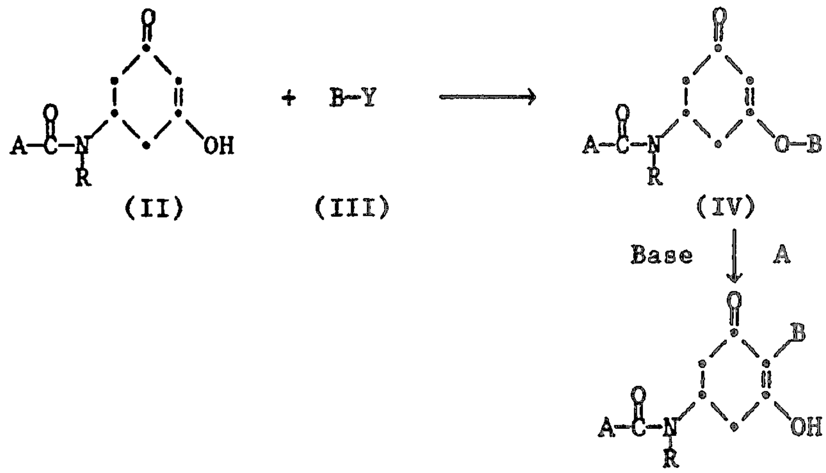


(II) (III) (IV)

Formel III zu einer Verbindung der Formel IV umsetzt, wobei A, B und R wie zuvor definiert sind, aber mit der Einschränkung, dass B nicht für die Oximestergruppe

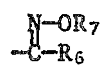

steht, und in der Y für eine unter den Reaktionsbedingungen abspaltbaren nucleofugen Gruppe, wie Chlor, Brom oder $C_1$-$C_4$-Alkoxy steht.

7. Verfahren zur Herstellung eines Mittels gemäss einem der Ansprüche 1 bis 3, dadurch gekennzeichnet, dass man eine Verbindung der Formel I mit Hilfs- und/oder Trägerstoffen innig vermischt.

8. Die Verwendung eines Mittels gemäss einem der Ansprüche 1 bis 3 zur Bekämpfung unerwünschten Pflanzenwuchses.

9. Die Verwendung eines Mittels gemäss einem der Ansprüche 1 bis 3 zur Wuchsregulierung bei Pflanzen.

10. Ein Verfahren zur Bekämpfung unerwünschten Pflanzenwuchses oder zur Wuchsregulierung bei Pflanzen, dadurch gekennzeichnet, dass man die Pflanze oder deren Lebensraum mit einer herbizid wirksamen Menge eines Mittels gemäss einem der Ansprüche 1 bis 3 oder mit einer wuchsregulatorisch wirksamen Menge eines Mittels gemäss einem der Ansprüche 1 bis 3 behandelt.

11. Verfahren gemäss Anspruch 4 zur Herstellung von Verbindungen der Formel I
worin
R für Wasserstoff oder $C_1$-$C_3$-Alkyl,
A für $R_2$, $OR_3$ oder $NR_3R_4$,
$R_2$ für $C_1$-$C_4$-Alkyl, Methoxymethyl oder Trifluormethyl,
$R_3$ für $C_1$-$C_4$-Alkyl, Cyclopropyl, Cyclohexyl, Benzyl oder gegebenenfalls durch Chlor, Methoxy oder Methyl substituiertes Phenyl
$R_4$ für Wasserstoff, Methyl, Methoxy oder Ethyl,
$R_3$ und $R_4$ ausserdem gemeinsam mit dem Stickstoffatom, an welches sie gebunden sind für einen Pyrrolidin oder Piperidinrest,
B für einen der Reste

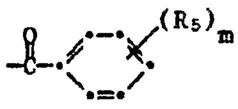 , 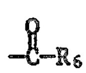 oder 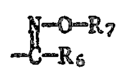 ,

m für 0, 1, 2 oder 3,
$R_5$ für Fluor, Chlor, Brom, Nitro, Methyl, Methoxy, Trifluormethyl, $C_1$-$C_4$-Akylthio, $C_1$-$C_4$-Alkylsulfinyl oder $C_1$-$C_4$-Alkylsulfonyl,
$R_6$ für $C_1$-$C_6$-Alkyl, Cyclopropyl oder Cyclohexyl,
$R_7$ für Methyl, Ethyl, Allyl, Propargyl oder 3-Chlor-prop-2-en-1-yl steht.

12. Verfahren gemäss Anspruch 4 oder 11 zur Herstellung von
2-Butanoyl-5-ethoxycarbonylaminocyclohexan-1,3-dion,
2-Propanoyl-5-(N',N'-dimethylureido)-cyclohexan-1,3-dion,
2-Butanoyl-5-(N',N'-dimethylureido)-cyclohexan-1,3-dion,
5-Acetamido-2-propanoyl-cyclohexan-1,3-dion,
2-(2,4-Dichlorbenzoyl)-5-ethoxycarbonylamino-cyclohexan-1,3-dion,
2-(2,4-Dichlorbenzoyl)-5-(N'-isopropylureido)-cyclohexan-1,3-dion,
2-(2,4-Dichlorbenzoyl)-5-(N',N'-dimethylureido)-cyclohexan-1,3-dion,
5-(N'-tert-Butylureido)-2-(1-allyloxyiminobutyl)-cyclohexan-1,3-dion,
5-(N',N'-Dimethylureido)-2-(1-ethoxyiminopropyl)-cyclohexan-1,3-dion,
5-(N'-Phenylureido)-2-(1-ethoxyiminobutyl)-cyclohexan-1,3-dion,
5-(N'-Phenylureido)-2-[1-(3-chlorallyl)oxyiminobutyl]-cyclohexan-1,3-dion,
5-(N'-Phenylureido)-2-(1-ethoxyiminoethyl)-cyclohexan-1,3-dion,
5-(N'-Phenylureido)-2-(1-methoxyiminobutyl)-cyclohexan-1,3-dion,
5-[N'-(2-Methylphenyl)-ureido]-2-(1-ethoxyiminobutyl)-cyclohexan-1,3-dion oder
5-[N'-(3-Chlorphenyl)-ureido]-2-(1-ethoxyiminobutyl)-cyclohexan-1,3-dion.

## Claims

### Claims for the Contracting States : AT BE CH/LI DE FR GB GR IT LU NL SE

1. Compounds of formula I

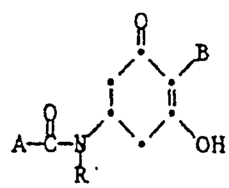

(I)

in which
R is hydrogen or $C_1$-$C_6$-alkyl,
A is $R_2$, $OR_3$ or $NR_3R_4$,
$R_2$ is $C_3$-$C_6$-cycloalkyl, or is $C_1$-$C_6$-alkyl that is unsubstituted or is mono-substituted by $C_1$-$C_4$-alkoxy or mono- or poly-substituted by halogen,
$R_3$ is $C_1$-$C_6$-alkyl or $C_3$-$C_6$-cycloalkyl, or is phenyl or benzyl each of which is unsubstituted or is mono-, di- or tri-substituted by $R_5$,
$R_4$ is hydrogen, $C_1$-$C_6$-alkyl, $C_1$-$C_4$-alkoxy or $C_3$-$C_6$-cycloalkyl,
$R_3$ and $R_4$ are, in addition, together with the nitrogen atom to which they are bonded, a-pyrrolidine, morpholine or piperidine radical,
B is one of the radicals

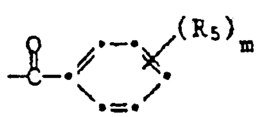 , 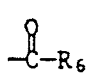 or 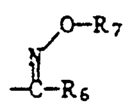 ,

m is 0, 1, 2 or 3,
$R_5$ is halogen, nitro, cyano, $C_1$-$C_4$-alkyl, $C_1$-$C_4$-alkoxy, $C_1$-$C_4$-alkylthio, $C_1$-$C_4$-haloalkyl, $C_1$-$C_4$-alkylsulphinyl or $C_1$-$C_4$-alkylsulphonyl,
$R_6$ is $C_1$-$C_6$-alkyl or $C_3$-$C_6$-cycloalkyl, and
$R_7$ is hydrogen, $C_1$-$C_6$-alkyl, $C_1$-$C_6$-haloalkyl, $C_3$-$C_6$-alkenyl, $C_3$-$C_6$-haloalkenyl or $C_3$-$C_6$-alkynyl,
and salts of the compounds of formula I with acids, bases or complexing agents.

2. Compounds of formula I according to claim 1,
in which
R is hydrogen or $C_1$-$C_3$-alkyl,
A is $R_2$, $OR_3$ or $NR_3R_4$,

$R_2$ is $C_1$-$C_4$-alkyl, methoxymethyl or trifluoromethyl,
$R_3$ is $C_1$-$C_4$-alkyl, cyclopropyl, cyclohexyl or benzyl, or is phenyl that is unsubstituted or is substituted by chlorine, methoxy or by methyl,
$R_4$ is hydrogen, methyl, methoxy or ethyl, $R_3$ and $R_4$ are, in addition, together with the nitrogen atom to which they are bonded, a pyrrolidine or piperidine radical,
B is one of the radicals

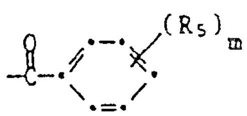 , 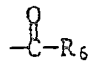 or 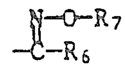 ,

m is 0, 1, 2 or 3,
$R_5$ is fluorine, chlorine, bromine, nitro, methyl, methoxy, trifluoromethyl, $C_1$-$C_4$-alkylthio, $C_1$-$C_4$-alkylsulphinyl or $C_1$-$C_4$-alkylsulphonyl,
$R_6$ is $C_1$-$C_6$-alkyl, cyclopropyl or cyclohexyl, and
$R_7$ is methyl, ethyl, allyl, propargyl or 3-chloro-prop-2-en-1-yl.

3. 2-butanoyl-5-ethoxycarbonylaminocyclohexane-1,3-dione,
2-propanoyl-5-(N',N'-dimethylureido)-cyclohexane-1,3-dione,
2-butanoyl-5-(N',N'-dimethylureido)-cyclohexane-1,3-dione,
5-acetamido-2-propanoyl-cyclohexane-1,3-dione,
2-(2,4-dichlorobenzoyl)-5-ethoxycarbonylamino-cyclohexane-1,3-dione,
2-(2,4-dichlorobenzoyl)-5-(N'-isopropylureido)-cyclohexane-1,3-dione,
2-(2,4-dichlorobenzoyl)-5-(N',N'-dimethylureido)-cyclohexane-1,3-dione,
5-(N'-tert-butylureido)-2-(1-allyloxyiminobutyl)-cyclohexane-1,3-dione,
5-(N',N'-dimethylureido)-2-(1-ethoxyiminopropyl)-cyclohexane-1,3-dione,
5-(N'-phenylureido)-2-(1-ethoxyiminobutyl)-cyclohexane-1,3-dione,
5-(N'-phenylureido)-2-[1-(3-chloroallyl)oxyiminobutyl]-cyclohexane-1,3-dione,
5-(N'-phenylureido)-2-(1-ethoxyiminoethyl)-cyclohexane-1,3-dione,
5-(N'-phenylureido)-2-(1-methoxyiminobutyl)-cyclohexane-1,3-dione,
5-[N'-(2-methylphenyl)-ureido]-2-(1-ethoxyiminobutyl)-cyclohexane-1,3-dione or
5-[N'-(3-chlorophenyl)-ureido]-2-(1-ethoxyiminobutyl)-cyclohexane-1,3-dione as compound of formula I according to claim 1 or 2.

4. A process for the preparation of compounds of formula I according to any one of claims 1 to 3, which comprises

a) reacting a cyclohexanedione of formula II with a compound of

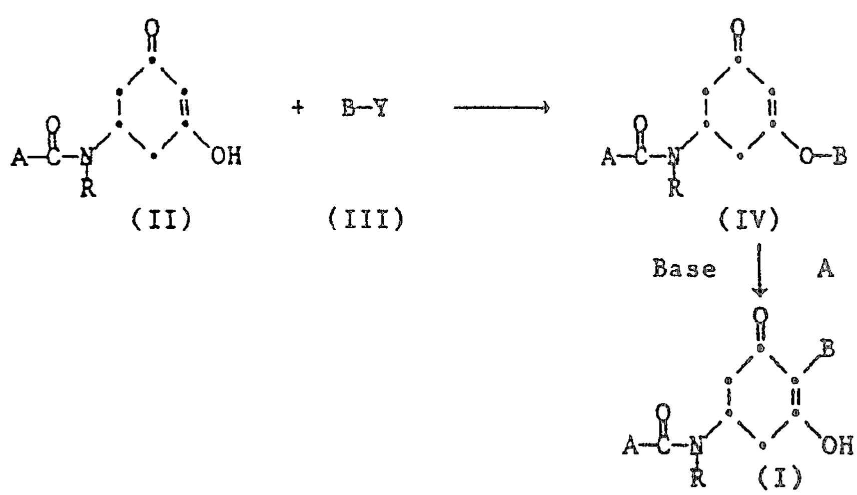


(II) (III) (IV) (I)

formula III to form a compound of formula IV which is then thermally rearranged in the presence of a base to form a compound of formula I, with A, B and R being as defined hereinbefore but with the limitation that B is not the oxime ether group

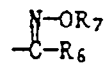

and Y being a nucleofugal group, such as chlorine, bromine or $C_1$-$C_4$-alkoxy, that can be removed under the reaction conditions,

or

b) reacting a cyclohexanedione of formula I with a compound

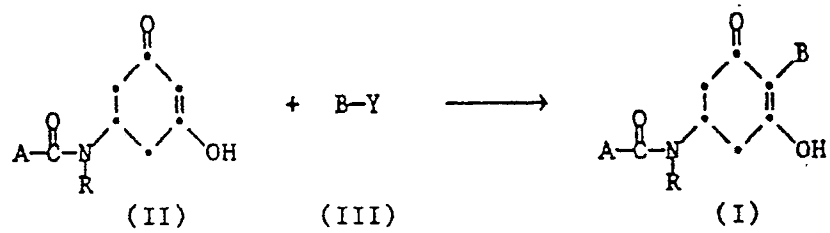

(II) (III) (I)

of formula III in which Y is a cyano group, in the presence of a base and $ZnCl_2$, to form a compound of formula I, the radicals A, R and B in formulae I, II and III being as defined hereinbefore, with the proviso that B is not the oxime ether group

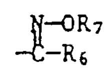

or

c) reacting a cyclohexyl ketone of formula Ia with a

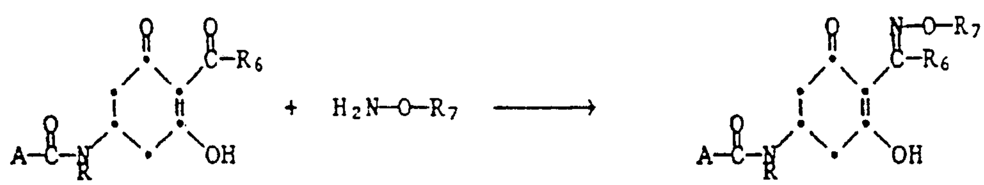

(Ia) (IV) (Ib)

hydroxylamine of formula IV to form an oxime of formula Ib, the radicals A, R, $R_6$ and $R_7$ being as defined hereinbefore.

5. Compounds of formula II

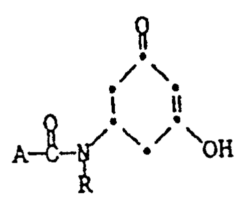

II

in which

R is hydrogen or $C_1$-$C_6$-alkyl,

A is $R_2$, $OR_3$ or $NR_3R_4$,

$R_2$ is $C_1$-$C_6$-alkyl or $C_3$-$C_6$-cycloalkyl,

$R_3$ is $C_1$-$C_6$-alkyl or $C_3$-$C_6$-cycloalkyl, or is phenyl or benzyl each of which is unsubstituted or is mono-, di- or tri-substituted by $R_5$,

$R_4$ is hydrogen, $C_1$-$C_6$-alkyl or $C_3$-$C_6$-cycloalkyl, $R_3$ and $R_4$ are, in addition, together with the nitrogen atom to which they are bonded, a pyrrolidine or piperidine radical.

6. A process for the preparation of compounds of formula II, according to claim 5, which comprises

a) hydrogenating a resorcinol of formula V in accordance with methods known per se

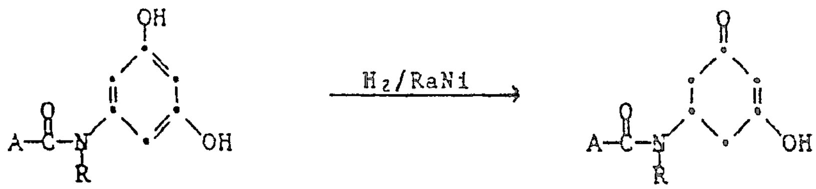


(V) (II)

the substituents A and R being as defined hereinbefore,

or

b) reacting an isocyanate of formula VI with an alcohol or an amine of formula VII or VIII, respectively,

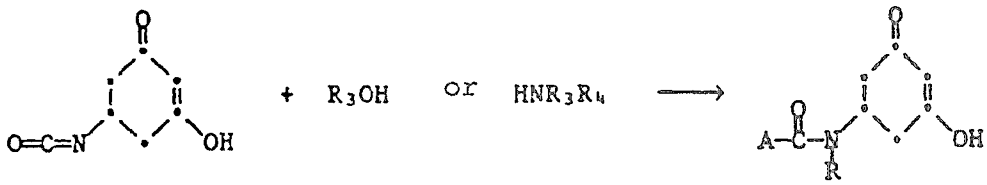


(VI) (VII) (VIII) (II)

to form a urethane or urea of formula II in which R is hydrogen and A is $R_3O$ or $NR_3R_4$ and $R_3$ and $R_4$ are as defined hereinbefore.

7. Compounds of formula IV

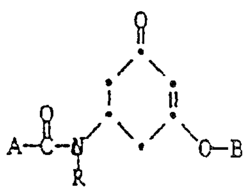

IV

(IV)

in which

R is hydrogen or $C_1$-$C_6$-alkyl,

A is $R_2$, $OR_3$ or $NR_3R_4$,

$R_2$ is $C_3$-$C_6$-cycloalkyl, or is $C_1$-$C_6$-alkyl that is unsubstituted or is mono-substituted by $C_1$-$C_4$-alkoxy or mono- or poly-substituted by halogen,

$R_3$ is $C_1$-$C_6$-alkyl or $C_3$-$C_6$-cycloalkyl, or is phenyl or benzyl each of which is unsubstituted or is mono-, di- or tri-substituted by $R_5$,

$R_4$ is hydrogen, $C_1$-$C_6$-alkyl, $C_1$-$C_4$-alkoxy or $C_3$-$C_6$-cycloalkyl,

$R_3$ and $R_4$ are, in addition, together with the nitrogen atom to which they are bonded, a pyrrolidine, morpholine or piperidine radical,

B is one of the radicals

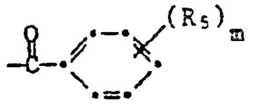 , 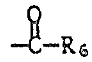 or 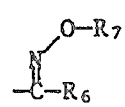 ,

m is 0, 1, 2 or 3,

$R_5$ is halogen, nitro, cyano, $C_1$-$C_4$-alkyl, $C_1$-$C_4$-alkoxy, $C_1$-$C_4$-alkylthio, $C_1$-$C_4$-haloalkyl, $C_1$-$C_4$-alkylsulphinyl or $C_1$-$C_4$-alkylsulphonyl,

$R_6$ is $C_1$-$C_6$-alkyl or $C_3$-$C_6$-cycloalkyl, and

$R_7$ is hydrogen, $C_1$-$C_6$-alkyl, $C_1$-$C_6$-haloalkyl, $C_3$-$C_6$-alkenyl, $C_3$-$C_6$-haloalkenyl or $C_3$-$C_6$-alkynyl.

8. A process for the preparation of compounds of formula IV according to claim 7, which comprises a) reacting a cyclohexanedione of formula II with a compound of

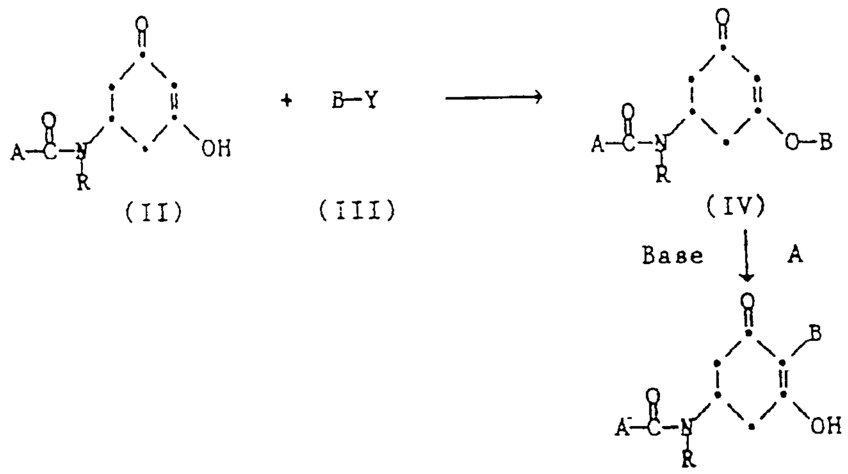


(II) (III) (IV)

formula III to form a compound of formula IV, with A, B and R being as defined hereinbefore, but with the limitation that B is not the oxime ester group

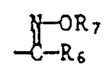

and Y being a nucleofugal group, such as chlorine, bromine or $C_1$-$C_4$-alkoxy, that can be removed under the reaction conditions.

9. A herbicidal or plant-growth-regulating composition which contains as active ingredient at least one compound of formula I according to any one of claims 1 to 3, together with carriers and/or other adjuvants.

10. The use of a compound of formula I according to any one of claims 1 to 3 or of a composition according to claim 9 for controlling undesired plant growth.

11. The use of a compound of formula I according to any one of claims 1 to 3 or of a composition according to claim 9 for regulating plant growth.

12. A method of controlling undesired plant growth or of regulating plant growth, which comprises treating the plants or the locus thereof with a herbicidally effective amount of a compound according to any one of claims 1 to 3 or of a composition according to claim 9, or with a compound according to any one of claims 1 to 3 or a composition according to claim 9, in an amount capable of regulating growth.

**Claims for the Contracting State : ES**

1. A herbicidal or plant-growth-regulating composition containing a compound of formula I

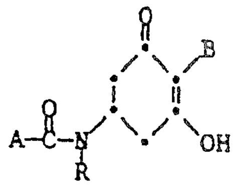

(I)

in which
R is hydrogen or $C_1$-$C_6$-alkyl,
A is $R_2$, $OR_3$ or $NR_3R_4$,
$R_2$ is $C_3$-$C_6$-cycloalkyl, or is $C_1$-$C_6$-alkyl that is unsubstituted or is mono-substituted by $C_1$-$C_4$-alkoxy or mono- or poly-substituted by halogen,
$R_3$ is $C_1$-$C_6$-alkyl or $C_3$-$C_6$-cycloalkyl, or is phenyl or benzyl each of which is unsubstituted or is mono-, di- or tri-substituted by $R_5$,
$R_4$ is hydrogen, $C_1$-$C_6$-alkyl, $C_1$-$C_4$-alkoxy or $C_3$-$C_6$-cycloalkyl,
$R_3$ and $R_4$ are, in addition, together with the nitrogen atom to which they are bonded, a pyrrolidine, morpholine or piperidine radical,
B is one of the radicals

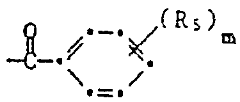 , 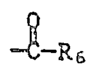 or 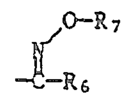 ,

m is 0, 1, 2 or 3,
$R_5$ is halogen, nitro, cyano, $C_1$-$C_4$-alkyl, $C_1$-$C_4$-alkoxy, $C_1$-$C_4$-alkylthio, $C_1$-$C_4$-haloalkyl, $C_1$-$C_4$-alkylsulphinyl or $C_1$-$C_4$-alkylsulphonyl,
$R_6$ is $C_1$-$C_6$-alkyl or $C_3$-$C_6$-cycloalkyl, and
$R_7$ is hydrogen, $C_1$-$C_6$-alkyl, $C_1$-$C_6$-haloalkyl, $C_3$-$C_6$-alkenyl, $C_3$-$C_6$-haloalkenyl or $C_3$-$C_6$-alkynyl,
and salts of the compounds of formula I with acids, bases or complexing agents.

2. A composition according to claim 1 containing a compound of formula I,
in which
R is hydrogen or $C_1$-$C_3$-alkyl,
A is $R_2$, $OR_3$ or $NR_3R_4$,
$R_2$ is $C_1$-$C_4$-alkyl, methoxymethyl or trifluoromethyl,
$R_3$ is $C_1$-$C_4$-alkyl, cyclopropyl, cyclohexyl or benzyl, or is phenyl that is unsubstituted or is substituted by chlorine, methoxy or by methyl,
$R_4$ is hydrogen, methyl, methoxy or ethyl,
$R_3$ and $R_4$ are, in addition, together with the nitrogen atom to which they are bonded, a pyrrolidine or piperidine radical,
B is one of the radicals

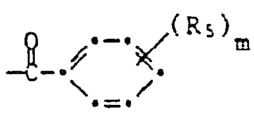 , 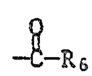 or 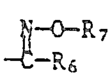 ,

m is 0, 1, 2 or 3,
$R_5$ is fluorine, chlorine, bromine, nitro, methyl, methoxy, trifluoromethyl, $C_1$-$C_4$-alkylthio, $C_1$-$C_4$-alkylsulphinyl or $C_1$-$C_4$-alkylsulphonyl,
$R_6$ is $C_1$-$C_6$-alkyl, cyclopropyl or cyclohexyl, and
$R_7$ is methyl, ethyl, allyl, propargyl or 3-chloro-prop-2-en-1-yl.

3. A composition according to claim 1 or 2 containing 2-butanoyl-5-ethoxycarbonylaminocyclohexane-1,3-dione,

2-propanoyl-5-(N',N'-dimethylureido)-cyclohexane-1,3-dione,
2-butanoyl-5-(N',N'-dimethylureido)-cyclohexane-1,3-dione,
5-acetamido-2-propanoyl-cyclohexane-1,3-dione,
2-(2,4-dichlorobenzoyl)-5-ethoxycarbonylamino-cyclohexane-1,3-dione,
2-(2,4-dichlorobenzoyl)-5-(N'-isopropylureido)-cyclohexane-1,3-dione,
2-(2,4-dichlorobenzoyl)-5-(N',N'-dimethylureido)-cyclohexane-1,3-dione,
5-(N'-tert-butylureido)-2-(1-allyloxyiminobutyl)-cyclohexane-1,3-dione,
5-(N',N'-dimethylureido)-2-(1-ethoxyiminopropyl)-cyclohexane-1,3-dione,
5-(N'-phenylureido)-2-(1-ethoxyiminobutyl)-cyclohexane-1,3-dione,
5-(N'-phenylureido)-2-[1-(3-chloroallyl)oxyiminobutyl]-cyclohexane-1,3-dione,
5-(N'-phenylureido)-2-(1-ethoxyiminoethyl)-cyclohexane-1,3-dione,
5-(N'-phenylureido)-2-(1-methoxyiminobutyl)-cyclohexane-1,3-dione,
5-[N'-(2-methylphenyl)-ureido]-2-(1-ethoxyiminobutyl)-cyclohexane-1,3-dione or
5-[N'-(3-chlorophenyl)-ureido]-2-(1-ethoxyiminobutyl)-cyclohexane-1,3-dione.

4. A process for the preparation of compounds of formula I

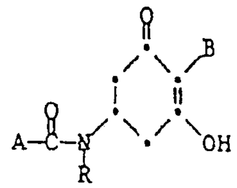

(I)

in which
R is hydrogen or $C_1$-$C_6$-alkyl,
A is $R_2$, $OR_3$ or $NR_3R_4$,
$R_2$ is $C_3$-$C_6$-cycloalkyl, or is $C_1$-$C_6$-alkyl that is unsubstituted or is mono-substituted by $C_1$-$C_4$-alkoxy or mono- or poly-substituted by halogen,
$R_3$ is $C_1$-$C_6$-alkyl or $C_3$-$C_6$-cycloalkyl, or is phenyl or benzyl each of which is unsubstituted or is mono-, di- or tri-substituted by $R_5$,
$R_4$ is hydrogen, $C_1$-$C_6$-alkyl, $C_1$-$C_4$-alkoxy or $C_3$-$C_6$-cycloalkyl,
$R_3$ and $R_4$ are, in addition, together with the nitrogen atom to which they are bonded, a pyrrolidine, morpholine or piperidine radical,
B is one of the radicals

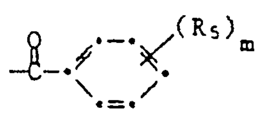 , 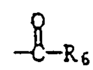 or 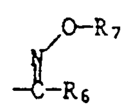 ,

m is 0, 1, 2 or 3,
$R_5$ is halogen, nitro, cyano, $C_1$-$C_4$-alkyl, $C_1$-$C_4$-alkoxy, $C_1$-$C_4$-alkylthio, $C_1$-$C_4$-haloalkyl, $C_1$-$C_4$-alkylsulphinyl or $C_1$-$C_4$-alkylsulphonyl,
$R_6$ is $C_1$-$C_6$-alkyl or $C_3$-$C_6$-cycloalkyl, and
$R_7$ is hydrogen, $C_1$-$C_6$-alkyl, $C_1$-$C_6$-haloalkyl, $C_3$-$C_6$-alkenyl, $C_3$-$C_6$-haloalkenyl or $C_3$-$C_6$-alkynyl,
and salts of the compounds of formula I with acids, bases or complexing agents, which comprises

a) reacting a cyclohexanedione of formula II with a compound of

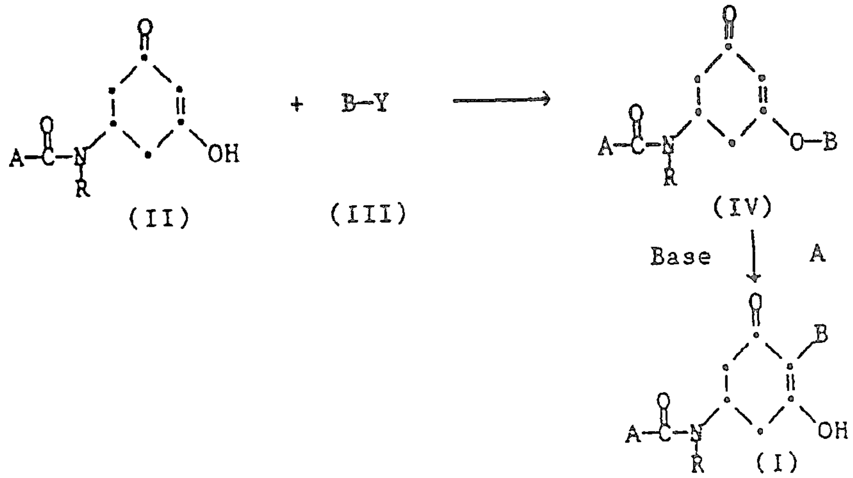

(II) (III) (IV) (I)

formula III to form a compound of formula IV which is then thermally rearranged in the presence of a base to form a compound of formula I, with A, B and R being as defined hereinbefore but with the limitation that B is not the oxime ether group

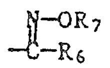

and Y being a nucleofugal group, such as chlorine, bromine or $C_1$-$C_4$-alkoxy, that can be removed under the reaction conditions,

or

b) reacting a cyclohexanedione of formula I with a compound

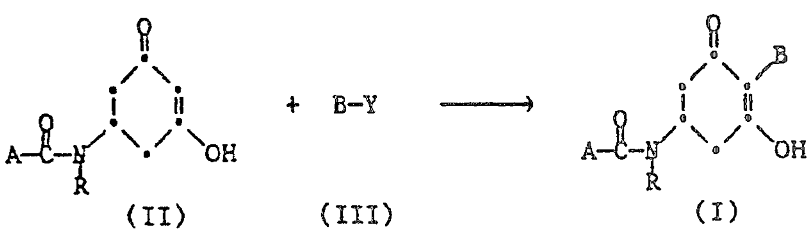
(II) (III) (I)

of formula III in which Y is a cyano group, in the presence of a base and $ZnCl_2$, to form a compound of formula I, the radicals A, R and B in formulae I, II and III being as defined hereinbefore, with the proviso that B is not the oxime ether group

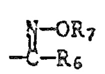

or

c) reacting a cyclohexyl ketone of formula Ia with a

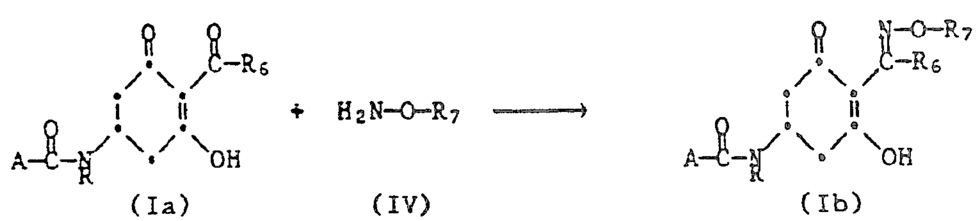
(Ia) (IV) (Ib)

hydroxylamine of formula IV to form an oxime of formula Ib, the radicals A, R, $R_6$ and $R_7$ being as defined hereinbefore.

5. A process for the preparation of compounds of formula II

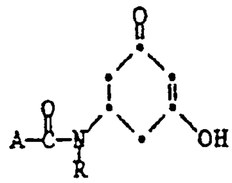

II

in which
R is hydrogen or $C_1$-$C_6$-alkyl,
A is $R_2$, $OR_3$ or $NR_3R_4$,
$R_2$ is $C_1$-$C_6$-alkyl or $C_3$-$C_6$-cycloalkyl,
$R_3$ is $C_1$-$C_6$-alkyl or $C_3$-$C_6$-cycloalkyl, or is phenyl or benzyl each of which is unsubstituted or is mono-, di- or tri-substituted by $R_5$,
$R_4$ is hydrogen, $C_1$-$C_6$-alkyl or $C_3$-$C_6$-cycloalkyl,
$R_3$ and $R_4$ are, in addition, together with the nitrogen atom to which they are bonded, a pyrrolidine or piperidine radical, which comprises

a) hydrogenating a resorcinol of formula V in accordance with methods known per se

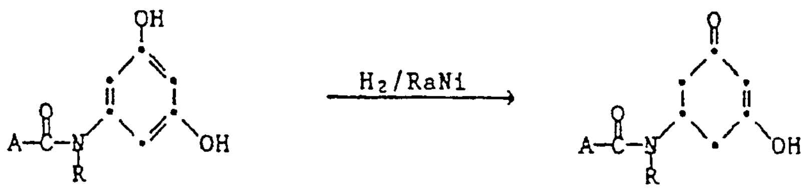


(V) (II)

the substituents A and R being as defined hereinbefore,

or

b) reacting an isocyanate of formula VI with an alcohol or an amine of formula VII or VIII, respectively,

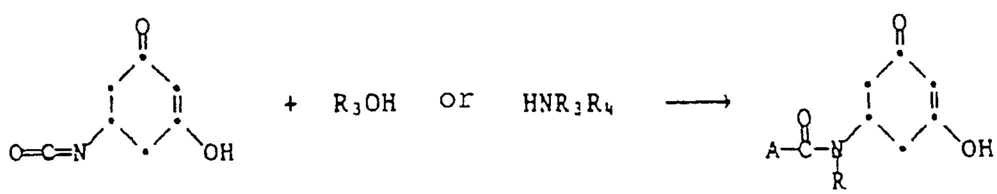


(VI) (VII) (VIII) (II)

to form a urethane or urea of formula II in which R is hydrogen and A is $R_3O$ or $NR_3R_4$ and $R_3$ and $R_4$ are as defined hereinbefore.

6. A process for the preparation of compounds of formula IV

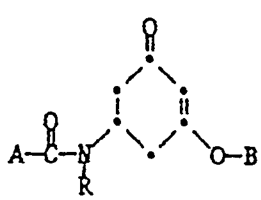

IV

(IV)

in which
R is hydrogen or $C_1$-$C_6$-alkyl,
A is $R_2$, $OR_3$ or $NR_3R_4$,
$R_2$ is $C_3$-$C_6$-cycloalkyl, or is $C_1$-$C_6$-alkyl that is unsubstituted or is mono-substituted by $C_1$-$C_4$-alkoxy or mono- or poly-substituted by halogen,
$R_3$ is $C_1$-$C_6$-alkyl or $C_3$-$C_6$-cycloalkyl, or is phenyl or benzyl each of which is unsubstituted or is mono-, di- or tri-substituted by $R_5$,
$R_4$ is hydrogen, $C_1$-$C_6$-alkyl, $C_1$-$C_4$-alkoxy or $C_3$-$C_6$-cycloalkyl,
$R_3$ and $R_4$ are, in addition, together with the nitrogen atom to which they are bonded, a pyrrolidine, morpholine or piperidine radical,
B is one of the radicals

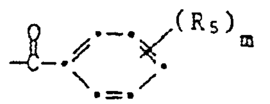 , 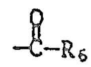 or 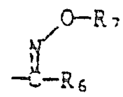 ,

m is 0, 1, 2 or 3,
$R_5$ is halogen, nitro, cyano, $C_1$-$C_4$-alkyl, $C_1$-$C_4$-alkoxy, $C_1$-$C_4$-alkylthio, $C_1$-$C_4$-haloalkyl, $C_1$-$C_4$-alkylsulphinyl or $C_1$-$C_4$-alkylsulphonyl,
$R_6$ is $C_1$-$C_6$-alkyl or $C_3$-$C_6$-cycloalkyl, and
$R_7$ is hydrogen, $C_1$-$C_6$-alkyl, $C_1$-$C_6$-haloalkyl, $C_3$-$C_6$-alkenyl, $C_3$-$C_6$-haloalkenyl or $C_3$-$C_6$-alkynyl, which comprises
a) reacting a cyclohexanedione of formula II with a compound of

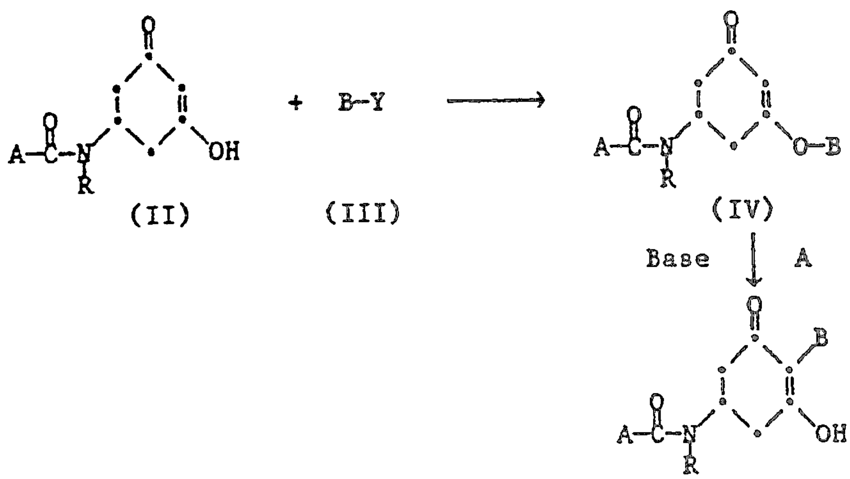

(II) (III) (IV)

formula III to form a compound of formula IV, with A, B and R being as defined hereinbefore, but with the limitation that B is not the oxime ester group

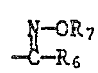

and Y being a nucleofugal group, such as chlorine, bromine or $C_1$-$C_4$-alkoxy, that can be removed under the reaction conditions.

7. A process for the preparation of a composition according to any one of claims 1 to 3, which comprises intimately mixing a compound of formula I with adjuvants and/or carriers.

8. The use of a composition according to any one of claims 1 to 3 for controlling undesired plant growth.

9. The use of a composition according to any one of claims 1 to 3 for regulating plant growth.

10. A method of controlling undesired plant growth or of regulating plant growth, which comprises treating the plant or the locus thereof with a herbicidally effective amount of a composition according to any one of claims 1 to 3, or with a composition according to any one of claims 1 to 3 in an amount capable of regulating growth.

11. A process according to claim 4 for the preparation of compounds of formula I in which
R is hydrogen or $C_1$-$C_3$-alkyl,
A is $R_2$, $OR_3$ or $NR_3R_4$,
$R_2$ is $C_1$-$C_4$-alkyl, methoxymethyl or trifluoromethyl,
$R_3$ is $C_1$-$C_4$-alkyl, cyclopropyl, cyclohexyl or benzyl, or is phenyl that is unsubstituted or is substituted by chlorine, methoxy or by methyl,
$R_4$ is hydrogen, methyl, methoxy or ethyl,
$R_3$ and $R_4$ are, in addition, together with the nitrogen atom to which they are bonded, a pyrrolidine or piperidine radical,
B is one of the radicals

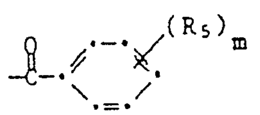 , 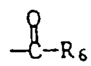 or 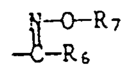 ,

m is 0, 1, 2 or 3,
$R_5$ is fluorine, chlorine, bromine, nitro, methyl, methoxy, trifluoromethyl, $C_1$-$C_4$-alkylthio, $C_1$-$C_4$-alkylsulphinyl or $C_1$-$C_4$-alkylsulphonyl,
$R_6$ is $C_1$-$C_6$-alkyl, cyclopropyl or cyclohexyl, and
$R_7$ is methyl, ethyl, allyl, propargyl or 3-chloro-prop-2-en-1-yl.

12. A process according to claim 4 or II for the preparation of
2-butanoyl-5-ethoxycarbonylaminocyclohexane-1,3-dione,
2-propanoyl-5-(N′,N′-dimethylureido)-cyclohexane-1,3-dione,
2-butanoyl-5-(N′,N′-dimethylureido)-cyclohexane-1,3-dione,
5-acetamido-2-propanoyl-cyclohexane-1,3-dione,
2-(2,4-dichlorobenzoyl)-5-ethoxycarbonylamino-cyclohexane-1,3-dione,
2-(2,4-dichlorobenzoyl)-5-(N′-isopropylureido)-cyclohexane-1,3-dione,
2-(2,4-dichlorobenzoyl)-5-(N′,N′-dimethylureido)-cyclohexane-1,3-dione,
5-(N′-tert-butylureido)-2-(1-allyloxyiminobutyl)-cyclohexane-1,3-dione,
5-(N′,N′-dimethylureido)-2-(1-ethoxyiminopropyl)-cyclohexane-1,3-dione,
5-(N′-phenylureido)-2-(1-ethoxyiminobutyl)-cyclohexane-1,3-dione,
5-(N′-phenylureido)-2-[1-(3-chloroallyl)oxyiminobutyl]-cyclohexane-1,3-dione,
5-(N′-phenylureido)-2-(1-ethoxyiminoethyl)-cyclohexane1,3-dione,
5-(N′-phenylureido)-2-(1-methoxyiminobutyl)-cyclohexane-1,3-dione,
5-[N′-(2-methylphenyl)-ureido]-2-(1-ethoxyiminobutyl)-cyclohexane-1,3-dione or
5-[N′-(3-chlorophenyl)-ureido]-2-(1-ethoxyiminobutyl)-cyclohexane-1,3-dione.

## Revendications

### Revendications pour les Etats Contractants : AT, BE, CH/LI, DE, FR, GB, GR, IT, LU, NL, SE

1. Composés de formule I

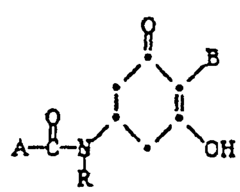 (I)

dans laquelle
R représente l'hydrogène ou un groupe alkyle en C 1-C 6,
A représente $R_2$, $OR_3$ ou $NR_3R_4$,
$R_2$ représente un groupe cycloalkyle en C 3-C 6 ou un groupe alkyle en C 1-C 6 portant éventuellement un

substituant alcoxy en C 1-C 4 ou un ou plusieurs substituants halogéno,
$R_3$ représente un groupe alkyle en C 1-C 6, cycloalkyle en C 3-C 6 ou un groupe phényle ou benzyle portant éventuellement jusqu'à trois substituants $R_5$,
$R_4$ représente l'hydrogène, un groupe alkyle en C 1-C 6, alcoxy en C 1-C 4 ou cycloalkyle en C 3-C 6,
$R_3$ et $R_4$ peuvent en outre former ensemble et avec l'atome d'azote auquel ils sont reliés un radical de pyrrolidine, de morpholine ou de pipéridine,
B représente l'un des groupes

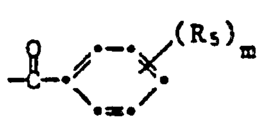 , 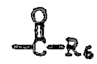 ou 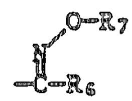 ,

m est égal à 0, 1, 2 ou 3,
$R_5$ représente un halogène, un groupe nitro, cyano, alkyle en C 1-C 4, alcoxy en C 1-C 4, alkylthio en C 1-C 4, halogénoalkyle en C 1-C 4, alkylsulfinyle en C 1-C 4 ou alkylsulfonyle en C 1-C 4,
$R_6$ représente un groupe alkyle en C 1-C 6 ou cycloalkyle en C 3-C 6,
$R_7$ représente l'hydrogène, un groupe alkyle en C 1-C 6, halogénoalkyle en C 1-C 6, alcényle en C 3-C 6, halogénoalcényle en C 3-C 6 ou alcynyle en C 3-C 6,
et les sels des composés de formule I et d'acides, de bases ou d'agents complexants.

2. Composés de formule I selon la revendication 1, dans laquelle
R représente l'hydrogène ou un groupe alkyle en C 1-C 3,
A représente $R_2$, $OR_3$ ou $NR_3R_4$,
$R_2$ représente un groupe alkyle en C 1-C 4, méthoxyméthyle ou trifluorométhyle,
$R_3$ représente un groupe alkyle en C 1-C 4, cyclopropyle, cyclohexyle, benzyle ou un groupe phényle éventuellement substitué par le chlore, des groupes méthoxy ou méthyle,
$R_4$ représente l'hydrogène, un groupe méthyle, méthoxy ou éthyle,
$R_3$ et $R_4$ peuvent en outre former ensemble et avec l'atome d'azote auquel ils sont reliés un radical de pyrrolidine ou de pipéridine,
B représente l'un des groupes

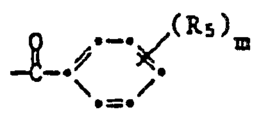 , 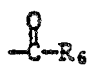 ou 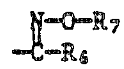 ,

m est égal à 0, 1, 2 ou 3,
$R_5$ représente le fluor, le chlore, le brome, un groupe nitro, méthyle, méthoxy, trifluorométhyle, alkylthio en C 1-C 4, alkylsulfinyle en C 1-C 4 ou alkylsulfonyle en C 1-C 4,
$R_5$ représente un groupe alkyle en C 1-C 6, cyclopropyle ou cyclohexyle,
$R_7$ représente un groupe méthyle, éthyle, allyle, propargyle ou 3-chloro-propa-2-ène-1-yle.

3. La 2-nutanoyl-5-éthoxycarbonylamino-cyclohexane-1,3-dione,
la 2-propanoyl-5-(N',N'-diméthyluréido)-cyclohexane-1,3-dione,
la 2-butanoyl-5-(N',N'-diméthyluréido)-cyclohexane-1,3-dione,
la 5-acétamido-2-propanoyl-cyclohexane-1,3-dione,
la 2-(2,4-dichlorobenzoyl)-5-éthoxycarbonylamino-cyclohexane-1,3-dione,
la 2-(2,4-dichlorobenzoyl)-5-(N'-isopropyluréido)-cyclohexane-1,3-dione,
la 2-(2,4-dichlorobenzoyl)-5-(N',N'-diméthyluréido)-cyclohexane-1,3-dione,
la 5-(N'-tert-butyluréido)-2-(1-allyloxyiminobutyl)-cyclohexane-1,3-dione,
la 5-(N',N'-diméthyluréido)-2-(1-éthoxyiminopropyl)-cyclohexane-1,3-dione,
la 5-(N'-phényluréido)-2-(1-éthoxyiminobutyl)-cyclohexane-1,3-dione,
la 5-(N'-phényluréido)-2-[1-(3-chlorallyl)-oxyiminobutyl]-cyclohexane-1,3-dione,
la 5-(N'-phényluréido)-2-(1-éthoxyiminoéthyl)-cyclohexane-1,3-dione,
la 5-(N'-phényluréido)-2-(1-méthoxyiminobutyl)-cyclohexane-1,3-dione,
la 5-[N'-(2-méthylphényl)-uréido]-2-(1-éthoxyiminobutyl)-cyclohexane-1,3-dione, ou
la 5-[N'-(3-chlorophényl)-uréido]-2-(1-éthoxyiminobutyl)-cyclohexane-1,3-dione,
en tant que composé de formule I selon la revendication 1 ou 2.

4. Procédé de préparation des composés de formule I selon l'une des revendications 1 à 3, caractérisé en ce que :

a) on fait réagir une cyclohexane-dione de formule II avec un composé de formule III, la réaction donnant un composé de formule IV qu'on transpose ensuite en présence d'une base, à la chaleur, en un composé de formule I

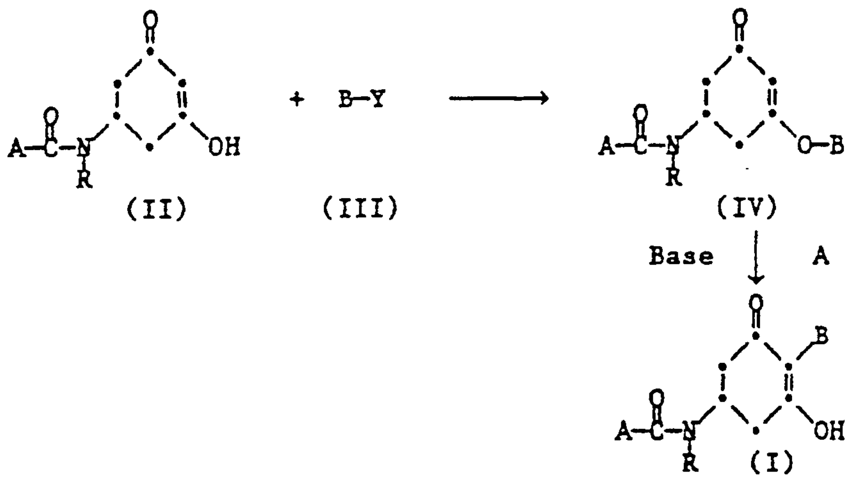


(II) (III) (IV) (I)

A, B et R ayant les significations indiquées ci-dessus, mais sous réserve que B ne peut représenter le groupe éther d'oxime

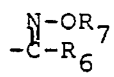

et Y représente un substituant nucléofuge éliminable dans les conditions de la réaction, tel que le chlore, le brome ou un groupe alcoxy en C 1-C 4, ou bien

b) on fait réagir une cyclohexane-dione de formule I avec un composé de formule III dans laquelle Y représente un groupe cyano, en présence d'une base et de $ZnCl_2$, la réaction donnant un composé de formule I

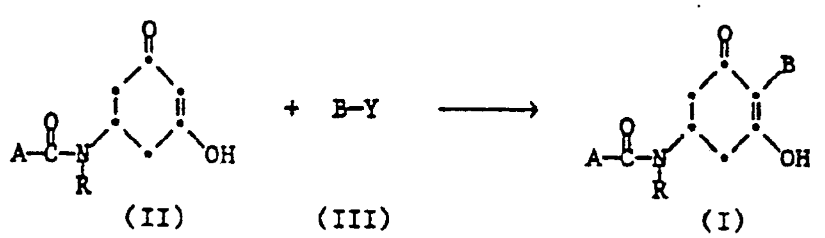

(II) (III) (I)

les symboles A, R et B ayant les significations indiquées ci-dessus en référence aux formules I, II et III, sous réserve que B ne peut représenter le groupe éther d'oxime

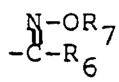

ou bien

c) on fait réagir une cyclohexylcétone de formule Ia avec une hydroxylamine de formule IV, la réaction donnant une oxime de formule Ib

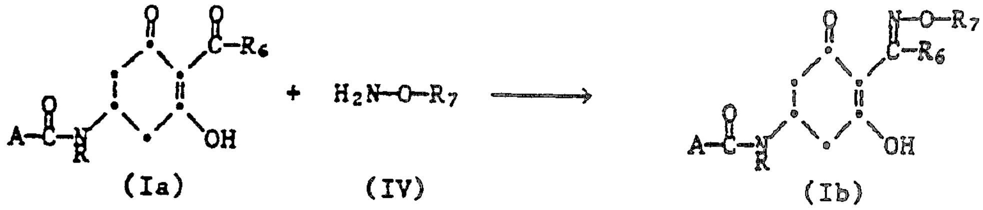


(Ia) (IV) (Ib)

A, R, $R_6$ et $R_7$ ayant les significations indiquées précédemment.

5. Composés de formule II

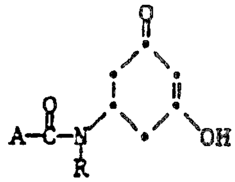

II

dans laquelle

R représente l'hydrogène ou un groupe alkyle en C 1-C 6,

A représente $R_2$, $OR_3$ ou $NR_3$ $R_4$,

$R_2$ représente un groupe alkyle en C 1-C 6 ou cycloalkyle en C 3-C 6,

$R_3$ représente un groupe alkyle en C 1-C 6, cycloalkyle en C 3-C 6 ou un groupe phényle ou benzyle portant éventuellement jusqu'à trois substituants $R_5$,

$R_4$ représente l'hydrogène, un groupe alkyle en C 1-C 6 ou cycloalkyle en C 3-C 6,

$R_3$ et $R_4$ peuvent en outre former ensemble et avec l'atome d'azote auquel ils sont reliés un radical de pyrrolidine ou de pipéridine.

6. Procédé de préparation des composés de formule II selon la revendication 5, caractérisé en ce que :

a) on hydrogène un résorcinol de formule V selon des procédés connus en soi

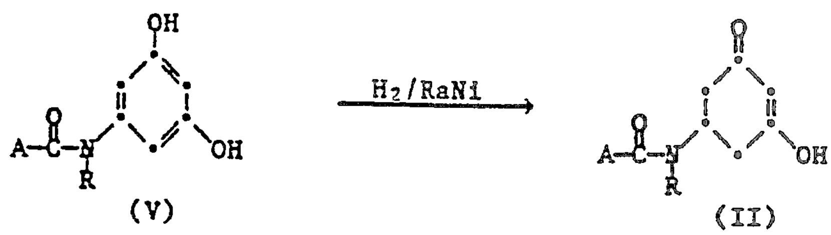


(V) (II)

A et R ayant les significations indiquées ci-dessus, ou bien

b) on fait réagir un isocyanate de formule VI avec un alcool de formule VII ou une amine de formule VIII

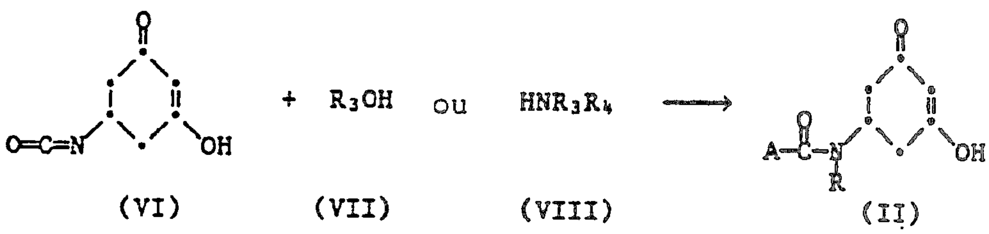


(VI) (VII) (VIII) (II)

la réaction donnant un uréthanne ou une urée de formule II dans laquelle R représente l'hydrogène et A représente $R_3O$ ou $NR_3R_4$, $R_3$ et $R_4$ ayant les significations indiquées ci-dessus.

7. Composés de formule IV

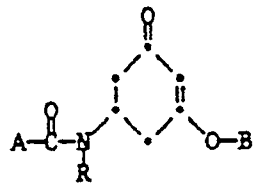

IV

(IV)

dans laquelle
R représente l'hydrogène ou un groupe alkyle en C 1-C 6,
A représente $R_2$, $OR_3$ ou $NR_3$ $R_4$,
$R_2$ représente un groupe cycloalkyle en C 3-C 6 ou un groupe alkyle en C 1-C 6 portant éventuellement un substituant alcoxy en C 1-C 4 ou un ou plusieurs substituants halogéno,
$R_3$ représente un groupe alkyle en C 1-C 6, cycloalkyle en C 3-C 6 ou un groupe phényle ou benzyle portant éventuellement jusqu'à trois substituants $R_5$,
$R_4$ représente l'hydrogène, un groupe alkyle en C 1-C 6, alcoxy en C 1-C 4 ou cycloalkyle en C 3-C 6,
$R_3$ et $R_4$ peuvent en outre former ensemble et avec l'atome d'azote auquel ils sont reliés un radical de pyrrolidine, de morpholine ou de pipéridine,
B représente l'un des groupes

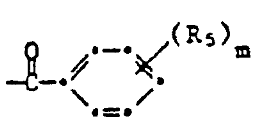 , 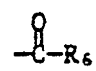 ou 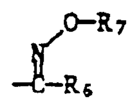 ,

m est égal à 0, 1, 2 ou 3,
$R_5$ représente un halogène, un groupe nitro, cyano, alkyle en C 1-C 4, alcoxy en C 1-C 4, alkylthio en C 1-C 4, halogénoalkyle en C 1-C 4, alkylsulfinyle en C 1-C 4 ou alkylsulfonyle en C 1-C 4,
$R_6$ représente un groupe alkyle en C 1-C 6 ou cycloalkyle en C 3-C 6,
$R_7$ représente l'hydrogène, un groupe alkyle en C 1-C 6, halogénoalkyle en C 1-C 6, alcényle en C 3-C 6, halogénoalcényle en C 3-C 6 ou alcynyle en C 3-C 6.

8. Procédé de préparation des composés de formule IV selon la revendication 7, caractérisé en ce que :
a) on fait réagir une cyclohexane-dione de formule II avec un composé de formule III, la réaction donnant un composé de formule IV

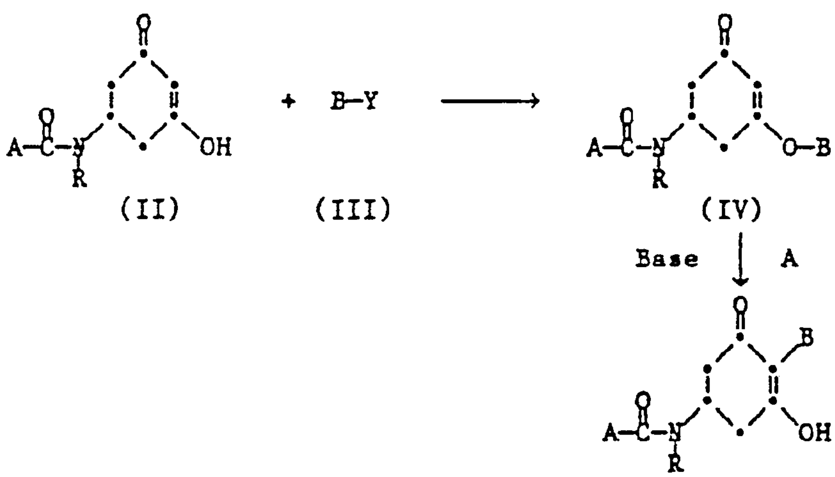


(II) (III) (IV)

A, B et R ayant les significations indiquées ci-dessus, sous réserve cependant que B ne peut représenter le groupe ester d'oxime

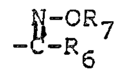

et Y représente un substituant nucléofuge éliminable dans les conditions de la réaction tel que le chlore, le brome ou un groupe alcoxy en C 1-C 4.

9. Un produit herbicide ou régulateur de la croissance des végétaux, caractérisé en ce qu'il contient, avec des véhicules et/ou d'autres produits auxiliaires, au moins une substance active consistant en un composé de formule I selon l'une des revendications 1 à 3.

10. L'utilisation d'un composé de formule I selon l'une des revendications 1 à 3 ou d'un produit selon la revendication 9, pour combattre les croissances de végétaux indésirables.

11. L'utilisation d'un composé de formule I selon l'une des revendications 1 à 3 ou d'un produit selon la revendication 9, pour la régulation de la croissance des végétaux.

12. Un procédé pour combattre les croissances de végétaux indésirables ou pour la régulation de la croissance des végétaux, caractérisé en ce que l'on traite les végétaux ou leur biotope par une quantité herbicide efficace d'une substance active selon l'une des revendications 1 à 3 ou d'un produit selon la revendication 9, ou par une quantité efficace en tant que régulatrice de croissance d'un composé selon l'une des revendications 1 à 3 ou d'un produit selon la revendication 9.

**Revendications pour l'Etat Contractant : ES**

1. Produit herbicide ou régulateur de la croissance des végétaux, contenant un composé de formule I

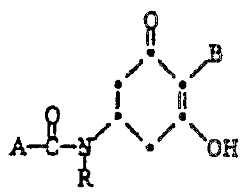

(I)

dans laquelle
R représente l'hydrogène ou un groupe alkyle en C 1-C 6, A représente $R_2$, $OR_3$ ou $NR_3$ $R_4$,
$R_2$ représente un groupe cycloalkyle en C 3-C 6 ou un groupe alkylz en C 1-C 6 portant éventuellement un substituant alcoxy en C 1-C 4 ou un ou plusieurs substituants halogéno,
$R_3$ représente un groupe alkyle en C 1-C 6, cycloalkyle en C 3-C 6 ou un groupe phényle ou benzyle portant éventuellement jusqu'à trois substituants $R_5$,
$R_4$ représente l'hydrogène, un groupe alkyle en C 1-C 6, alcoxy en C 1-C 4 ou cycloalkyle en C 3-C 6,
$R_3$ et $R_4$ peuvent en outre former ensemble et avec l'atome d'azote auquel ils sont reliés un radical de pyrrolidine, de morpholine ou de pipéridine,
B représente l'un des groupes

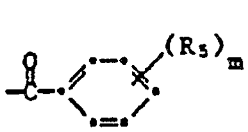 , 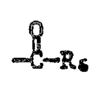 ou 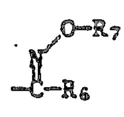 ,

m est égal à 0, 1, 2 ou 3,
$R_5$ représente un halogène, un groupe nitro, cyano, alkyle en C 1-C 4, alcoxy en C 1-C 4, alkylthio en C 1-C 4, halogénoalkyle en C 1-C 4, alkylsulfinyle en C 1-C 4 ou alkylsulfonyle en C 1-C 4,
$R_6$ représente un groupe alkyle en C 1-C 6 ou cycloalkyle en C 3-C 6,
$R_7$ représente l'hydrogène, un groupe alkyle en C 1-C 6, halogénoalkyle en C 1-C 6, alcényle en C 3-C 6, halogénoalcényle en C 3-C 6 ou alcynyle en C 3-C 6,
ou des sels des composés de formule I et d'acides, de bases ou d'agents complexants.

2. Produit selon la revendication 1, contenant un composé de formule I dans laquelle
R représente l'hydrogène ou un groupe alkyle en C 1-C 3,
A représente $R_2$, $OR_3$ ou $NR_3$ $R_4$,

$R_2$ représente un groupe alkyle en C 1-C 4, méthoxyméthyle ou trifluorométhyle,
$R_3$ représente un groupe alkyle en C 1-C 4, cyclopropyle, cyclohexyle, benzyle ou un groupe phényle éventuellement substitué par le chlore, des groupes méthoxy ou méthyle,
$R_4$ représente l'hydrogène, un groupe méthyle, méthoxy ou éthyle,
$R_3$ et $R_4$ peuvent en outre former ensemble et avec l'atome d'azote auquel ils sont reliés un radical de pyrrolidine ou de pipéridine,
B représente l'un des groupes

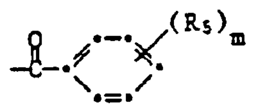 , 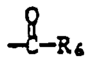 ou 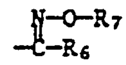 ,

m est égal à 0, 1, 2 ou 3,
$R_5$ représente le fluor, le chlore, le brome, un groupe nitro, méthyle, méthoxy, trifluorométhyle, alkylthio en C 1-C 4, alkylsulfinyle en C 1-C 4 ou alkylsulfonyle en C 1-C 4,
$R_6$ représente un groupe alkyle en C 1-C 6, cyclopropyle ou cyclohexyle,
$R_7$ représente un groupe méthyle, éthyle, allyle, propargyle ou 3-chloro-propa-2-ène-1-yle.

3. Produit selon la revendication 1 ou 2, contenant
la 2-butanoyl-5-éthoxycarbonylamino-cyclohexane-1,3-dione,
la 2-propanoyl-5-(N',N'-diméthyluréido)-cyclohexane-1,3-dione,
la 2-butanoyl-5-(N',N'-diméthyluréido)-cyclohexane-1,3-dione,
la 5-acétamido-2-propanoyl-cyclohexane-1,3-dione,
la 2-(2,4-dichlorobenzoyl)-5-éthoxycarbonylamino-cyclohexane-1,3-dione,
la 2-(2,4-dichlorobenzoyl)-5-(N'-isopropyluréido)-cyclohexane-1,3-dione,
la 2-(2,4-dichlorobenzoyl)-5-(N',N'-diméthyluréido)-cyclohexane-1,3-dione,
la 5-(N'-tert-butyluréido)-2-(1-allyloxyiminobutyl)-cyclohexane-1,3-dione,
la 5-(N',N'-diméthyluréido)-2-(1-éthoxyiminopropyl)-cyclohexane-1,3-dione,
la 5-(N'-phényluréido)-2-(1-éthoxyiminobutyl) cyclohexane-1,3-dione,
la 5-(N'-phényluréido)-2-[1-(3-chlorallyl)-oxyiminobutyl]-cyclohexane-1,3-dione,
la 5-(N'-phényluréido)-2-(1-éthoxyiminoéthyl)-cyclohexane-1,3-dione,
la 5-(N'-phényluréido)-2-(1-méthoxyiminobutyl)-cyclohexane-1,3-dione,
la 5-[N'-(2-méthylphényl)-uréido]-2-(1-éthoxyiminobutyl)-cyclohexane-1,3-dione, ou
la 5-[N'-(3-chlorophényl)-uréido]-2-(1-éthoxyiminobutyl)-cyclohexane-1,3-dione.

4. Procédé de préparation des composés de formule I

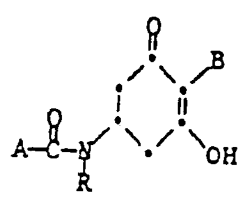 (I)

dans laquelle

R représente l'hydrogène ou un groupe alkyle en C 1-C 6, A représente $R_2$, $OR_3$ ou $NR_3$ $R_4$,
$R_2$ représente un groupe cycloalkyle en C 3-C 6 ou un groupe alkyle en C 1-C 6 portant éventuellement un substituant alcoxy en C 1-C 4 ou un ou plusieurs substituants halogéno,
$R_3$ représente un groupe alkyle en C 1-C 6, cycloalkyle en C 3-C 6 ou un groupe phényle ou benzyle portant éventuellement jusqu'à trois substituants $R_5$,
$R_4$ représente l'hydrogène, un groupe alkyle en C 1-C 6, alcoxy en C 1-C 4 ou cycloalkyle en C 3-C 6,
$R_3$ et $R_4$ peuvent en outre former ensemble et avec l'atome d'azote auquel ils sont reliés un radical de pyrrolidine, de morpholine ou de pipéridine,
B représente l'un des groupes

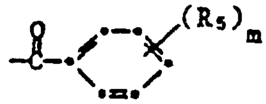 , 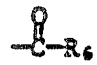 ou 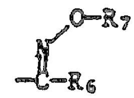 ,

m est égal à 0, 1, 2 ou 3,

$R_5$ représente un halogène, un groupe nitro, cyano, alkyle en C 1-C 4, alcoxy en C 1-C 4, alkylthio en C 1-C 4, halogénoalkyle en C 1-C 4, alkylsulfinyle en C 1-C 4 ou alkylsulfonyle en C 1-C 4,

$R_6$ représente un groupe alkyle en C 1-C 6 ou cycloalkyle en C 3-C 6,

$R_7$ représente l'hydrogène, un groupe alkyle en C 1-C 6, halogénoalkyle en C 1-C 6, alcényle en C 3-C 6, halogénoalcényle en C 3-C 6 ou alcynyle en C 3-C 6, et des sels des composés de formule I et d'acides, de bases ou d'agents complexants, caractérisé en ce que

a) on fait réagir une cyclohexane-dione de formule II avec un composé de formule III, la réaction donnant un composé de formule IV qu'on transpose ensuite en présence d'une base, à la chaleur, en un composé de formule I

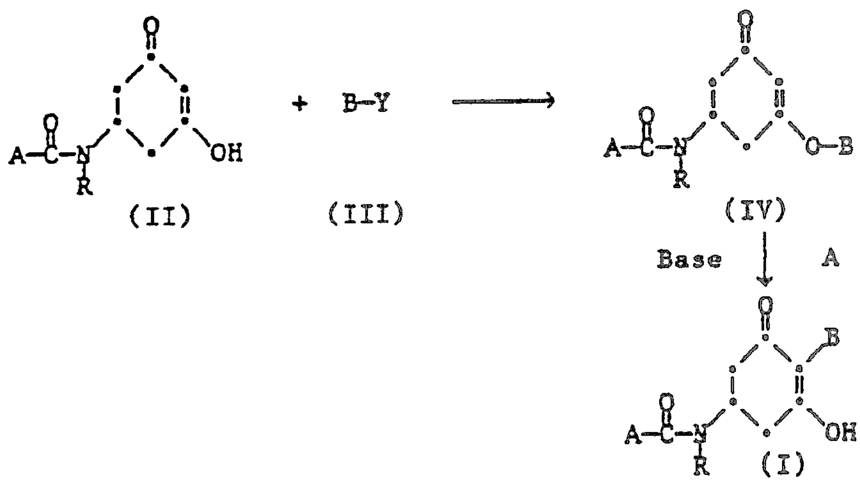


A, B et R ayant les significations indiquées ci-dessus, mais sous réserve que B ne peut représenter le groupe éther d'oxime

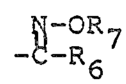

et Y représente un substituant nucléofuge éliminable dans les conditions de la réaction, tel que le chlore, le brome ou un groupe alcoxy en C 1-C 4, ou bien

b) on fait réagir une cyclohexane-dione de formule I avec un composé de formule III dans laquelle Y représente un groupe cyano, en présence d'une base et de $ZnCl_2$, la réaction donnant un composé de formule I

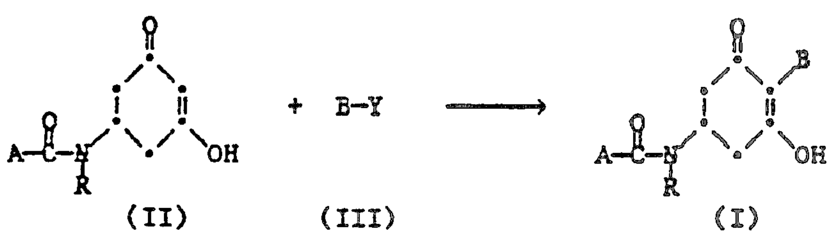


les symboles A, R et B ayant les significations indiquées ci-dessus en référence aux formules I, II et III, sous réserve que B ne peut représenter le groupe éther d'oxime

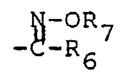

ou bien

c) on fait réagir une cyclohexylcétone de formule Ia avec une hydroxylamine de formule IV, la réaction donnant une oxime de formule Ib

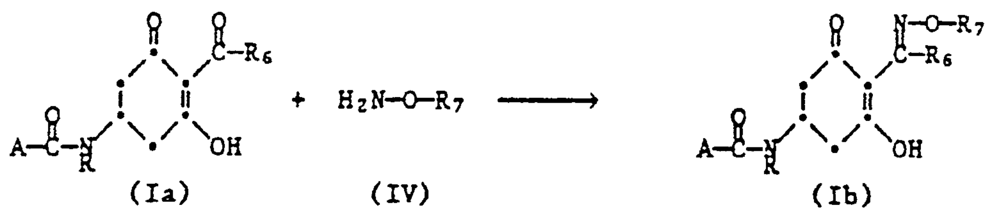

(Ia) (IV) (Ib)

A, R, $R_6$ et $R_7$ ayant les significations indiquées précédemment.

5. Procédé de préparation des composés de formule II

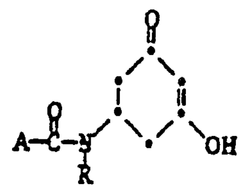

(II)

dans laquelle
R représente l'hydrogène ou un groupe alkyle en C 1-C 6, A représente $R_2$, $OR_3$ ou $NR_3$ $R_4$,
$R_2$ représente un groupe alkyle en C 1-C 6 ou cycloalkyle en C 3-C 6,
$R_3$ représente un groupe alkyle en C 1-C 6, cycloalkyle en C 3-C 6 ou un groupe phényle ou benzyle portant éventuellement jusqu'à trois substituants $R_5$,
$R_4$ représente l'hydrogène, un groupe alkyle en C 1-C 6 ou cycloalkyle en C 3-C 6,
$R_3$ et $R_4$ peuvent en outre former ensemble et avec l'atome d'azote auquel ils sont reliés un radical de pyrrolidine ou de pipéridine,
caractérisé en ce que

a) on hydrogène un résorcinol de formule V selon des procédés connus en soi

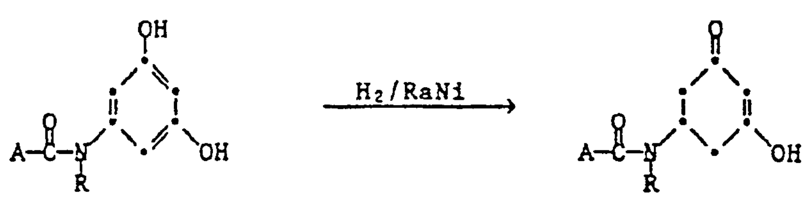


(V) (II)

A et R ayant les significations indiquées ci-dessus, ou bien

b) on fait réagir un isocyanate de formule VI avec un alcool de formule VII ou une amine de formule VIII

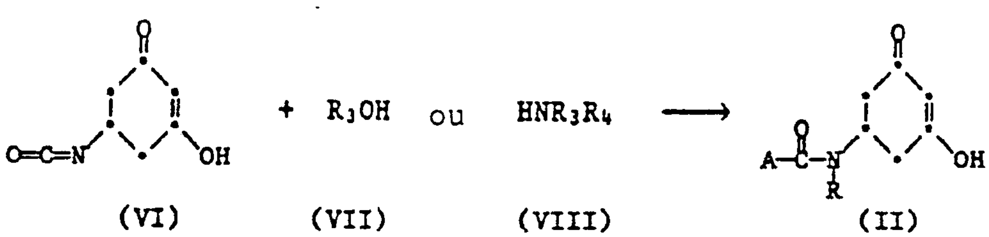
ou

(VI) (VII) (VIII) (II)

la réaction donnant un uréthanne ou une urée de formule II dans laquelle R représente l'hydrogène et A représente $R_3O$ ou $NR_3R_4$, $R_3$ et $R_4$ ayant les significations indiquées ci-dessus.

6. Procédé de préparation des composés de formule IV

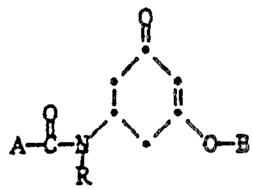

(IV)

(IV)

dans laquelle
R représente l'hydrogène ou un groupe alkyle en C1-C6,
A représente $R_2$, $OR_3$ ou $NR_3R_4$,
$R_2$ représente un groupe cycloalkyle en C3-C6 ou un groupe alkyle en C1-C6 portant éventuellement un substituant alcoxy en C1-C4 ou un ou plusieurs substituants halogéno,
$R_3$ représente un groupe alkyle en C1-C6, cycloalkyle en C3-C6 ou un groupe phényle ou benzyle portant éventuellement jusqu'à trois substituants $R_5$,
$R_4$ représente l'hydrogène, un groupe alkyle en C1-C6, alcoxy en C1-C4 ou cycloalkyle en C3-C6,
$R_3$ et $R_4$ peuvent en outre former ensemble et avec l'atome d'azote auquel ils sont reliés un radical de pyrrolidine, de morpholine ou de pipéridine,
B représente l'un des groupes

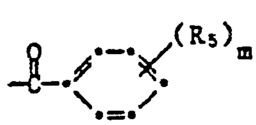 , 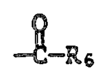 ou 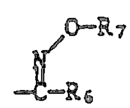 ,

m est égal à 0, 1, 2 ou 3,
$R_5$ représente un halogène, un groupe nitro, cyano, alkyle en C1-C4, alcoxy en C1-C4, alkylthio en C1-C4, halogénoalkyle en C1-C4, alkylsulfinyle en C1-C4 ou alkylsulfonyle en C1-C4,
$R_6$ représente un groupe alkyle en C1-C6 ou cycloalkyle en C3-C6,
$R_7$ représente l'hydrogène, un groupe alkyle en C1-C6, halogénoalkyle en C1-C6, alcényle en C3-C6, halogénoalcényle en C3-C6 ou alcynyle en C3-C6
caractérisé en ce que
a) on fait réagir une cyclohexane-dione de formule II avec un composé de formule III, la réaction donnant un composé de formule IV

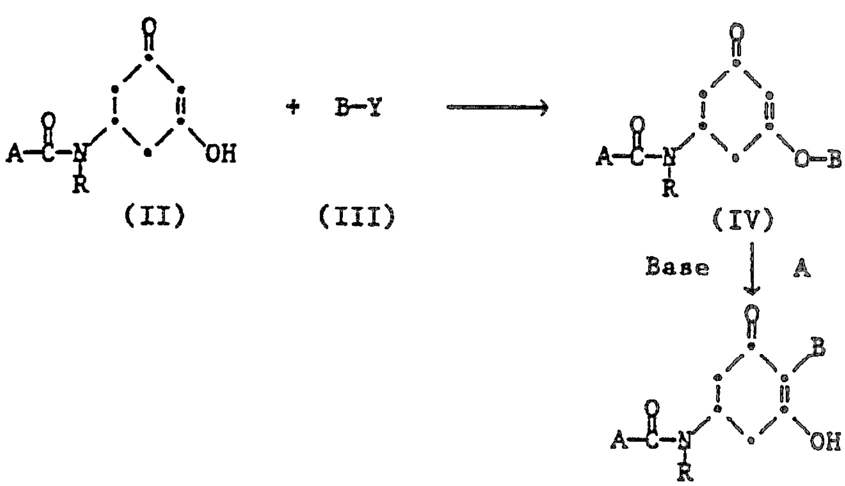


(II) (III) (IV)

A, B et R ayant les significations indiquées ci-dessus, sous réserve cependant que B ne peut représenter le groupe ester d'oxime

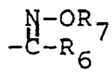

et Y représente un substituant nucléofuge éliminable dans les conditions de la réaction tel que le chlore, le brome ou un groupe alcoxy en C1-C4.

7. Procédé de préparation d'un produit selon l'une des revendications 1 à 3, caractérisé en ce que l'on mélange intimement un composé de formule I avec des produits auxiliaires et/ou véhicules.

8. L'utilisation d'un produit selon l'une des revendications 1 à 3, pour combattre les croissances de végétaux indésirables.

9. L'utilisation d'un produit selon l'une des revendications 1 à 3, pour la régulation de la croissance des végétaux.

10. Un procédé pour combattre les croissances de végétaux indésirables ou pour la régulation de la croissance des végétaux, caractérisé en ce que l'on traite la plante ou son biotope par une quantité herbicide efficace d'un produit selon l'une des revendications 1 à 3 ou par une quantité régulatrice de croissance efficace d'un produit selon l'une des revendications 1 à 3.

11. Procédé selon la revendication 4, pour la préparation de composés de formule I dans laquelle
R représente l'hydrogène ou un groupe alkyle en C 1-C 3, A représente $R_2$, $OR_3$ ou $NR_3$ $R_4$,
$R_2$ représente un groupe alkyle en C 1-C 4, méthoxyméthyle ou trifluorométhyle,
$R_3$ représente un groupe alkyle en C 1-C 4, cyclopropyle, cyclohexyle, benzyle ou un groupe phényle éventuellement substitué par le chlore, des groupes méthoxy ou méthyle,
$R_4$ représente l'hydrogène, un groupe méthyle, méthoxy ou éthyle,
$R_3$ et $R_4$ peuvent en outre former ensemble et avec l'atome d'azote auquel ils sont reliés un radical de pyrrolidine ou de pipéridine,
B représente l'un des groupes

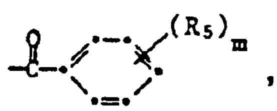 , 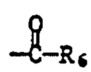 ou 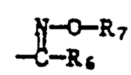 ,

m est égal à 0, 1, 2 ou 3,
$R_5$ représente le fluor, le chlore, le brome, un groupe nitro, méthyle, méthoxy, trifluorométhyle, alkylthio en C 1-C 4, alkylsulfinyle en C 1-C 4 ou alkylsulfonyle en C 1-C 4,
$R_6$ représente un groupe alkyle en C 1-C 6, cyclopropyle ou cyclohexyle,
$R_7$ représente un groupe méthyle, éthyle, allyle, propargyle ou 3-chloro-propa-2-ène-1-yle.

12. Procédé selon la revendication 4 ou 11, pour la préparation de :
la 2-butanoyl-5-éthoxycarbonylamino-cyclohexane-1,3-dione,
la 2-propanoyl-5-(N',N'-diméthyluréido)-cyclohexane-1,3-dione,
la 2-butanoyl-5-(N',N'-diméthyluréido)-cyclohexane-1,3-dione,
la 5-acétamido-2-propanoyl-cyclohexane-1,3-dione,
la 2-(2,4-dichlorobenzoyl)-5-éthoxycarbonylamino-cyclohexane-1,3-dione,
la 2-(2,4-dichlorobenzoyl)-5-(N'-isopropyluréido)-cyclohexane-1,3-dione,
la 2-(2,4-dichlorobenzoyl)-5-(N',N'-diméthyluréido)-cyclohexane-1,3-dione,
la 5-(N'-tert-butyluréido)-2-(1-allyloxyiminobutyl)-cyclohexane-1,3-dione,
la 5-(N',N'-diméthyluréido)-2-(1-éthoxyiminopropyl)-cyclohexane-1,3-dione,
la 5-(N'-phényluréido)-2-(1-éthoxyiminobutyl)-cyclohexane-1,3-dione,
la 5-(N'-phényluréido)-2-[1-(3-chlorallyl)-oxyiminobutyl]-cyclohexane-1,3-dione,
la 5-(N'-phényluréido)-2-(1-éthoxyiminoéthyl)-cyclohexane-1,3-dione,
la 5-(N'-phényluréido)-2-(1-méthoxyiminobutyl)-cyclohexane-1,3-dione,
la 5-[N'-(2-méthylphényl)-uréido]-2-(1-éthoxyiminobutyl)-cyclohexane-1,3-dione, ou
la 5-[N'-(3-chlorophényl)-uréido]-2-(1-éthoxyiminobutyl)-cyclohexane-1,3-dione.